# EUROPEAN PATENT APPLICATION

(11) **EP 1 621 529 A1**
(43) Date of publication of application: **01.02.2006**
(21) Application number: 04721361.6
(22) Date of filing: 17.03.2004
(51) Int. Cl.: C07C 229/34, C07C 227/18, C07B 61/00

(54) **3-AMINO-3-ARYLPROPIONIC ACID n-ALKYL ESTERS, PROCESS FOR PRODUCTION THEREOF, AND PROCESS FOR PRODUCTION OF OPTICALLY ACTIVE 3-AMINO-3-ARYLPROPIONIC ACIDS AND ESTERS OF THE ANTIPODES THERETO**

(30) Priority: 17.03.2003 JP 2003071458; 17.03.2003 JP 2003071459
(71) Applicant: Ube Industries, Ltd., Ube-shi, Yamaguchi-ken 755-8633 (JP)
(72) Inventor: YAMAMOTO, Yasuhito c/o Ube Research Laboratory, Yamaguchi 755-8633 (JP); MIYATA, Hiroyuki c/o Ube Research Laboratory, Yamaguchi 755-8633 (JP); KONEGAWA, Tadayoshi c/o Ube Research Laboratory, Yamaguchi 755-8633 (JP); SAKATA, Kazuma c/o Ube Research Laboratory, Yamaguchi 755-8633 (JP)
(74) Representative: HOFFMANN EITLE
(86) International application number: PCT/JP2004/003543
(87) International publication number: WO 2004/083163

(57) **Abstract**

The present invention is to provide an n-alkyl 3-amino-3-arylpropionate represented by the formula (I): 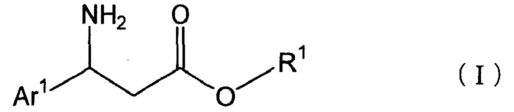
wherein Ar¹ represents an aryl group which may have a substituent(s), provided that a phenyl group and 4-methoxyphenyl group are excluded, R¹ represents an n-propyl group or an n-butyl group,
and a process for preparing the same, and its optically active compound and an optically active (S or R)-3-amino-3-arylpropionic acid represented by the formula (III-a): 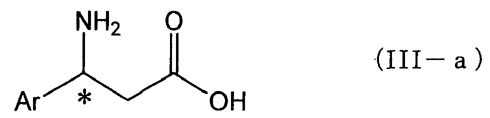
wherein Ar represents an aryl group which may have a substituent(s), and * represents an asymmetric carbon,
and a process for preparing an optically active n-alkyl (R or S)-3-amino-3-arylpropionate represented by the formula (IV-a): 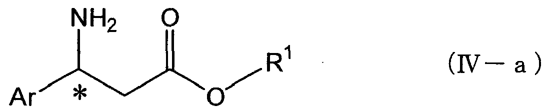
wherein Ar and R¹ have the same meanings as defined above, * represents an asymmetric carbon, provided that it has a reverse absolute configuration to the compound of the formula (III-a).

## Description

### Technical field

The present invention relates to a novel n-alkyl 3-amino-3-arylpropionate and a process for preparing the same. The n-alkyl 3-amino-3-arylpropionate can be easily led to an optically active 3-amino-3-arylpropionic acid which is useful as a synthetic intermediate of a physiologically active peptide or a lactam series antibiotic according to the conventionally known selective hydrolysis method.

The present invention also relates to a method of obtaining optically active (S or R)-3-amino-3-arylpropionic acid and optically active (R or S)-3-amino-3-arylpropionic acid ester simultaneously from n-alkyl 3-amino-3-arylpropionate (racemic mixtures). These 3-amino-3-arylpropionic acid and its ester are useful as a starting material or a synthetic intermediate of a physiologically active peptide or a lactam series antibiotic.

### Background art

In the prior art, the n-alkyl 3-amino-3-arylpropionate (provided that the case where the aryl group is a phenyl group or a 4-methoxyphenyl group is excluded.) of the present invention is a nove compound, and the presence thereof and a preparation process have never been known.

Also, as a conventional process for simultaneously obtaining an optically active (S or R)-3-amino-3-arylpropionic acid and an optically active (R or S)-3-amino-3-arylpropionic acid ester from a 3-amino-3-arylpropionic acid ester (racemic mixtures) using hydrolase, a process in which an optically active (S)-3-amino-3-arylpropionic acid and an optically active (R)-ethyl 3-amino-3-arylpropionate are obtained by selectively hydrolyzing one of enantiomers of ethyl 3-amino-3-arylpropionate (racemic mixtures) in water in the presence of lipase (tradename: Amano PS) orininated from *Burkholderia cepacia* (*Pseudomonas cepacia*) has been disclosed (for example, see Tetrahedron Lett., 41, 2679 (2000).). However, according to this process, there is a problem that an E value which is an index of selectivity between enantiomers by an enzyme is low. Incidentally, the E value has widely been utilized as an index of selectivity of kinetic resolution (for example, see J. Am. Chem. Soc., 104, 7294 (1982).).

An object of the present invention is to solve the above-mentioned problems, and to provide a novel n-alkyl 3-amino-3-arylpropionate by a simple and easy process with high yield, and a process for preparing the same.

Other objects of the present invention is to solve the above-mentioned problems, and to provide a process for preparing optically active 3-amino-3-arylpropionic acid and optically active 3-amino-3-arylpropionic acid ester from 3-amino-3-arylpropionic acid ester (racemic mixtures), which is industrially suitable and obtain optically active (S or R)-3-amino-3-arylpropionic acid and optically active (R or S)-3-amino-3-arylpropionic acid ester simultaneously with high E values by a simple and easy method.

### Disclosure of the invention

The present invention relates to an n-alkyl 3-amino-3-arylpropionate represented by the formula (I): 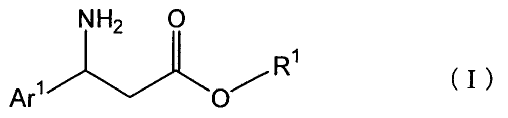
wherein Ar¹ represents an aryl group which may have a substituent(s), provided that a phenyl group and a 4-methoxyphenyl group are excluded, R¹ represents an n-propyl group or an n-butyl group.

The present invention also relates to an (R or S)-n-alkyl 3-amino-3-arylpropionate represented by the formula (I-a) : 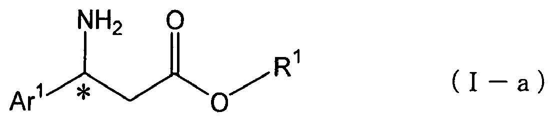
wherein Ar¹ and R¹ have the same meanings as defined above, and * represents an asymmetric carbon.

The present invention further relates to a process for preparing an n-alkyl 3-amino-3-arylpropionate represented by the formula (IV): 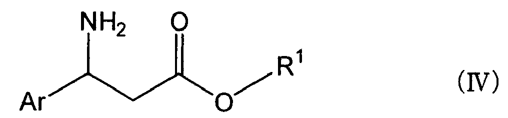
wherein Ar represents an aryl group which may have a substituent(s), provided that a phenyl gruop is excluded, and R¹ has the same meanings as defined above,
which comprises (A) the first step of reacting an arylaldehyde represented by the formula (II): 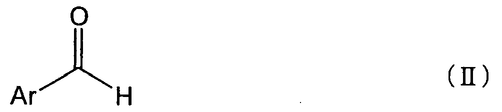
wherein Ar has the same meaning as defined above, malonic acid and ammonium acetate in an organic solvent to prepare a 3-amino-3-arylpropionic acid represented by the formula (III): 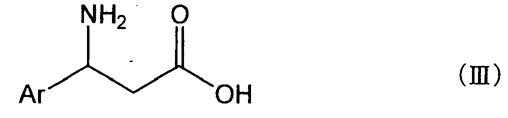
wherein Ar has the same meaning as defined above, (B) the second step of then reacting the 3-amino-3-arylpropionic acid with n-propyl alcohol or n-butyl alcohol in the presence of an acid catalyst.

The present inventors have further studied earnestly to attracted attention to the ester portion of the 3-amino-3-arylpropionic acid ester (racemic mixtures) to be optically resolved, and as a result, they have found that a substrate in which the ester group is an n-propyl group or n-butyl group alone shows a specifically high E value in the present hydrolysis reaction.

The present invention further relates to a process for preparing an optically active (S or R)-3-amino-3-arylpropionic acid represented by the formula (III-a): 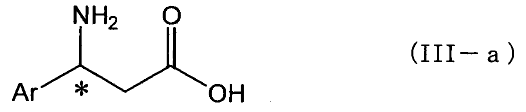 wherein Ar has the same meaning as defined above,
and * represents an asymmetric carbon, and an optically active (R or S)-n-alkyl 3-amino-3-arylpropionate represented by the formula (IV-a): 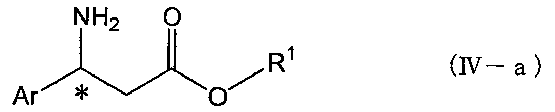
wherein Ar and R¹ has the same meaning as defined above, and * represents an asymmetric carbon, provided that it has a reverse absolute configuretion to that of the compound of the formula (III-a),
which comprises subjecting either one of the enantiomers of the n-alkyl 3-amino-3-arylpropionates represented by the above-mentioned formula (IV) which are racemic mixtures to selective hydrolysis reaction in the presence of a hydrolase in a mixed solvent of an organic solvent and a buffer.

The present invention further relates to a process for preparing an optically active (S or R)-3-amino-3-arylpropionic acid and the ester of the other enantiomer, which comprises isolating each of the compound from a mixture containing the optically active (S or R)-3-amino-3-arylpropionic acid represented by the formula (III-a): 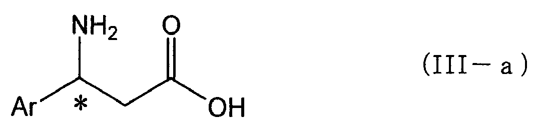
wherein Ar has the same meaning as defined above,
and * represents an asymmetric carbon, and the optically active (R or S)-n-alkyl 3-amino-3-arylpropionate represented by the formula (IV-a): 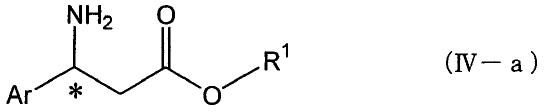
wherein Ar and R¹ have the same meanings as defined above, and * represents an asymmetric carbon, incidentally, it has an opposite configuration to the compound of the formula (III-a),
formed by the above hydrolysis reaction.

### Best mode for carrying out the invention

The n-propyl 3-amino-3-arylpropionate in the present invention is represented by the above-mentioned formula (I). In the formula (I), Ar¹ represents an aryl group which may have a substituent(s) (provided that phenyl group and 4-methoxyphenyl group are excluded).

The aryl group in the above-mentioned "an aryl group which may have a substituent(s)" means a substituted phenyl group or naphthyl gruop. Also, the substituent(s) of the aryl group which may have a substituent(s) may be mentioned an alkyl group having 1 to 4 carbon atoms (Incidentally, these groups contain various kinds of isomers.) such as a methyl group, an ethyl group, a propyl group, a butyl group, etc.; hydroxyl group; a halogen atom such as a chlorine atom, a bromine atom, an iodine atom, a fluorine atom, etc.; an alkoxy group having 2 to 4 carbon atoms such as an ethoxy group, etc. (Incidentally, these groups contain various kinds of isomers.); an alkylenedioxy group having 1 to 4 carbon atoms such as a methylenedioxy group, etc.; a nitro group and the like.

Such "an aryl group which may have a substituent(s)" may be specifically mentioned, for example, a 2-tolyl group, 3-tolyl group, 4-tolyl group, 2,3-xylyl group, 2,6-xylyl group, 2,4-xylyl group, 3,4-xylyl group, mesityl group, 2-hydroxyphenyl group, 4-hydroxyphenyl group, 3,4-dihydroxyphenyl group, 2-chlorophenyl group, 3-chlorophenyl group, 4-chlorophenyl group, 3,4-dichlorophenyl group, 4-bromophenyl group, 4-iodophenyl group, 2-fluorophenyl group, 3-fluorophenyl group, 4-fluorophenyl group, 3-bromo-5-chloro-2-hydroxyphenyl group, 2-methoxyphenyl group, 3-methoxyphenyl group, 3,4-dimethoxyphenyl group, 3,4-methylenedioxyphenyl group, 4-ethoxyphenyl group, 4-butoxyphenyl group, 4-isopropoxyphenyl group, 4-nitrophenyl group, 2-nitrophenyl group, 1-naphthyl gruop, 2-naphthyl group, etc., preferably a 2-tolyl group, 4-tolyl group, 2,3-xylyl group, 3,4-xylyl group, 4-hydroxyphenyl group, 3,4-dihydroxyphenyl group, 2-chlorophenyl group, 4-chlorophenyl group, 3,4-dichlorophenyl group, 2-fluorophenyl, 3-fluorophenyl group, 4-fluorophenyl group, 3-bromo-5-chloro-2-hydroxyphenyl group, 2-methoxyphenyl group, 3,4-dimethoxyphenyl group, 3,4-methylenedioxyphenyl group, 4-ethoxyphenyl group, 4-nitrophenyl group, 2-nitrophenyl group, 1-naphthyl gruop, 2-naphthyl gruop, more preferably 4-tolyl group, 4-hydroxyphenyl group, 3,4-dihydroxyphenyl group, 4-chlorophenyl group, 3-fluorophenyl group, 4-fluorophenyl group, 3,4-dimethoxyphenyl group, 3,4-methylenedioxyphenyl group, 4-nitrophenyl group, 1-naphthyl gruop, 2-naphthyl gruop.

Also, R¹ represents an n-propyl group or n-butyl group. In the present invention, when R¹ is an n-propyl group or n-butyl group, an optically active (S or R)-3-amino-3-arylpropionic acid and an optically active (R or S)-3-amino-3-arylpropionic acid ester can be simultaneously obtained with higher E values.

Specific examples of the n-propyl 3-amino-3-arylpropionate having the above-mentoined Ar¹, there may be mentioned, for example,
n-propyl 3-amino-3-(2-tolyl)propionate,
n-propyl 3-amino-3-(3-tolyl)propionate,
n-propyl 3-amino-3-(4-tolyl)propionate,
n-propyl 3-amino-3-(2,3-xylyl)propionate,
n-propyl 3-amino-3-(4-ethylphenyl)propionate,
n-propyl 3-amino-3-(2-chlorophenyl)propionate,
n-propyl 3-amino-3-(3-chlorophenyl)propionate,
n-propyl 3-amino-3-(4-chlorophenyl)propionate,
n-propyl 3-amino-3-(2,3-dichlorophenyl)propionate,
n-propyl 3-amino-3-(2,4-dichlorophenyl)propionate,
n-propyl 3-amino-3-(3,4-dichlorophenyl)propionate,
n-propyl 3-amino-3-(2-bromophenyl)propionate,
n-propyl 3-amino-3-(3-bromophenyl)propionate,
n-propyl 3-amino-3-(4-bromophenyl)propionate,
n-propyl 3-amino-3-(2-fluorophenyl)propionate,
n-propyl 3-amino-3-(3-fluorophenyl)propionate,
n-propyl 3-amino-3-(4-fluorophenyl)propionate,
n-propyl 3-amino-3-(2-iodophenyl)propionate,
n-propyl 3-amino-3-(3-iodophenyl)propionate,
n-propyl 3-amino-3-(4-iodophenyl)propionate,
n-propyl 3-amino-3-(2-methoxyphenyl)propionate,
n-propyl 3-amino-3-(3-methoxyphenyl)propionate,
n-propyl 3-amino-3-(4-ethoxyphenyl)propionate,
n-propyl 3-amino-3-(2-hydroxyphenyl)propionate,
n-propyl 3-amino-3-(3-hydroxyphenyl)propionate,
n-propyl 3-amino-3-(4-hydroxyphenyl)propionate,
n-propyl 3-amino-3-(4-nitrophenyl)propionate,
n-propyl 3-amino-3-(4-cyanophenyl)propionate,
n-propyl 3-amino-3-(3,4-dimethoxyphenyl)propionate,
n-propyl 3-amino-3-(3,4-methylenedioxyphenyl)propionate,
n-propyl 3-amino-3-(1-phenoxyphenyl)propionate,
n-propyl 3-amino-3-(4-benzyloxyphenyl)propionate,
n-propyl 3-amino-3-(1-naphthyl)propionate,
n-propyl 3-amino-3-(2-naphthyl)propionate,
n-propyl 3-amino-3-(2-pyridyl)propionate,
n-propyl 3-amino-3-(3-pyridyl)propionate,
n-propyl 3-amino-3-(4-pyridyl)propionate,
n-propyl 3-amino-3-(2-thienyl)propionate,
n-propyl 3-amino-3-(2-furyl)propionate,
n-propyl 3-amino-3-(2-quinolyl)propionate and
n-propyl 3-amino-3-(3-bromo-5-chloro-2-hydroxyphenyl)propionate
and the like, preferably
n-propyl 3-amino-3-(2-tolyl)propionate,
n-propyl 3-amino-3-(3-tolyl)propionate,
n-propyl 3-amino-3-(4-tolyl)propionate,
n-propyl 3-amino-3-(2,3-xylyl)propionate,
n-propyl 3-amino-3-(2-chlorophenyl)propionate,
n-propyl 3-amino-3-(3-chlorophenyl)propionate,
n-propyl 3-amino-3-(4-chlorophenyl)propionate,
n-propyl 3-amino-3-(3,4-dichlorophenyl)propionate,
n-propyl 3-amino-3-(2-bromophenyl)propionate,
n-propyl 3-amino-3-(3-bromophenyl)propionate,
n-propyl 3-amino-3-(4-bromophenyl)propionate,
n-propyl 3-amino-3-(2-fluorophenyl)propionate,
n-propyl 3-amino-3-(3-fluorophenyl)propionate,
n-propyl 3-amino-3-(4-fluorophenyl)propionate,
n-propyl 3-amino-3-(2-iodophenyl)propionate,
n-propyl 3-amino-3-(3-iodophenyl)propionate,
n-propyl 3-amino-3-(4-iodophenyl)propionate,
n-propyl 3-amino-3-(2-methoxyphenyl)propionate,
n-propyl 3-amino-3-(3-methoxyphenyl)propionate,
n-propyl 3-amino-3-(4-ethoxyphenyl)propionate,
n-propyl 3-amino-3-(4-hydroxyphenyl)propionate,
n-propyl 3-amino-3-(4-nitrophenyl)propionate,
n-propyl 3-amino-3-(4-cyanophenyl)propionate,
n-propyl 3-amino-3-(3,4-dimethoxyphenyl)propionate,
n-propyl 3-amino-3-(3,4-methylenedioxyphenyl)propionate,
n-propyl 3-amino-3-(1-naphthyl)propionate;
n-propyl 3-amino-3-(2-pyridyl)propionate,
n-propyl 3-amino-3-(3-pyridyl)propionate and
n-propyl 3-amino-3-(4-pyridyl)propionate
and the like, more preferably
n-propyl 3-amino-3-(2-tolyl)propionate,
n-propyl 3-amino-3-(3-tolyl)propionate,
n-propyl 3-amino-3-(4-tolyl)propionate,
n-propyl 3-amino-3-(2-chlorophenyl)propionate,
n-propyl 3-amino-3-(3-chlorophenyl)propionate,
n-propyl 3-amino-3-(4-chlorophenyl)propionate,
n-propyl 3-amino-3-(3,4-dichlorophenyl)propionate,
n-propyl 3-amino-3-(2-bromophenyl)propionate,
n-propyl 3-amino-3-(3-bromophenyl)propionate,
n-propyl 3-amino-3-(4-bromophenyl)propionate,
n-propyl 3-amino-3-(2-fluorophenyl)propionate,
n-propyl 3-amino-3-(3-fluorophenyl)propionate,
n-propyl 3-amino-3-(4-fluorophenyl)propionate,
n-propyl 3-amino-3-(4-iodophenyl)propionate,
n-propyl 3-amino-3-(2-methoxyphenyl)propionate,
n-propyl 3-amino-3-(3-methoxyphenyl)propionate,
n-propyl 3-amino-3-(4-hydroxyphenyl)propionate,
n-propyl 3-amino-3-(3,4-methylenedioxyphenyl)propionate,
n-propyl 3-amino-3-(3-pyridyl)propionate and
n-propyl 3-amino-3-(4-pyridyl)propionate
and the like, particularly preferably
n-propyl 3-amino-3-(2-tolyl)propionate,
n-propyl 3-amino-3-(3-tolyl)propionate,
n-propyl 3-amino-3-(4-tolyl)propionate,
n-propyl 3-amino-3-(2-chlorophenyl)propionate,
n-propyl 3-amino-3-(3-chlorophenyl)propionate,
n-propyl 3-amino-3-(4-chlorophenyl)propionate,
n-propyl 3-amino-3-(2-bromophenyl)propionate,
n-propyl 3-amino-3-(3-bromophenyl)propionate,
n-propyl 3-amino-3-(4-bromophenyl)propionate,
n-propyl 3-amino-3-(2-fluorophenyl)propionate,
n-propyl 3-amino-3-(3-fluorophenyl)propionate,
n-propyl 3-amino-3-(4-fluorophenyl)propionate,
n-propyl 3-amino-3-(2-methoxyphenyl)propionate,
n-propyl 3-amino-3-(3-methoxyphenyl)propionate, and
n-propyl 3-amino-3-(3,4-methylenedioxyphenyl)propionate.

Also, specific examples of the n-butyl 3-amino-3-arylpropionate having the above-mentoined Ar¹, there may be mentioned, for example,
n-butyl 3-amino-3-(2-tolyl)propionate,
n-butyl 3-amino-3-(3-tolyl)propionate,
n-butyl 3-amino-3-(4-tolyl)propionate,
n-butyl 3-amino-3-(2,3-xylyl)propionate,
n-butyl 3-amino-3-(2-chlorophenyl)propionate,
n-butyl 3-amino-3-(3-chlorophenyl)propionate,
n-butyl 3-amino-3-(4-chlorophenyl)propionate,
n-butyl 3-amino-3-(2,3-dichlorophenyl)propionate,
n-butyl 3-amino-3-(2,4-dichlorophenyl)propionate,
n-butyl 3-amino-3-(3,4-dichlorophenyl)propionate,
n-butyl 3-amino-3-(3,5-dichlorophenyl)propionate,
n-butyl 3-amino-3-(2-bromophenyl)propionate,
n-butyl 3-amino-3-(3-bromophenyl)propionate,
n-butyl 3-amino-3-(4-bromophenyl)propionate,
n-butyl 3-amino-3-(2-fluorophenyl)propionate,
n-butyl 3-amino-3-(3-fluorophenyl)propionate,
n-butyl 3-amino-3-(4-fluorophenyl)propionate,
n-butyl 3-amino-3-(3,4-difluorophenyl)propionate,
n-butyl 3-amino-3-(2-iodophenyl)propionate,
n-butyl 3-amino-3-(3-iodophenyl)propionate,
n-butyl 3-amino-3-(4-iodophenyl)propionate,
n-butyl 3-amino-3-(2-methoxyphenyl)propionate,
n-butyl 3-amino-3-(3-methoxyphenyl)propionate,
n-butyl 3-amino-3-(4-methoxyphenyl)propionate,
n-butyl 3-amino-3-(2,3-dimethoxyphenyl)propionate,
n-butyl 3-amino-3-(3,4-dimethoxyphenyl)propionate,
n-butyl 3-amino-3-(3,5-dimethoxyphenyl)propionate and
n-butyl 3-amino-3-(3,4-methylenedioxyphenyl)propionate
and the like, preferably
n-butyl 3-amino-3-(2-tolyl)propionate,
n-butyl 3-amino-3-(3-tolyl)propionate,
n-butyl 3-amino-3-(4-tolyl)propionate,
n-butyl 3-amino-3-(2,3-xylyl)propionate,
n-butyl 3-amino-3-(2-chlorophenyl)propionate,
n-butyl 3-amino-3-(3-chlorophenyl)propionate,
n-butyl 3-amino-3-(4-chlorophenyl)propionate,
n-butyl 3-amino-3-(3,4-dichlorophenyl)propionate,
n-butyl 3-amino-3-(2-bromophenyl)propionate,
n-butyl 3-amino-3-(3-bromophenyl)propionate,
n-butyl 3-amino-3-(4-bromophenyl)propionate,
n-butyl 3-amino-3-(2-fluorophenyl)propionate,
n-butyl 3-amino-3-(3-fluorophenyl)propionate,
n-butyl 3-amino-3-(4-fluorophenyl)propionate,
n-butyl 3-amino-3-(2-iodophenyl)propionate,
n-butyl 3-amino-3-(3-iodophenyl)propionate,
n-butyl 3-amino-3-(4-iodophenyl)propionate,
n-butyl 3-amino-3-(2-methoxyphenyl)propionate,
n-butyl 3-amino-3-(3-methoxyphenyl)propionate,
n-butyl 3-amino-3-(4-methoxyphenyl)propionate,
n-butyl 3-amino-3-(2,3-dimethoxyphenyl)propionate and n-butyl 3-amino-3-(3,4-methylenedioxyphenyl)propionate
and the like, more preferably
n-butyl 3-amino-3-(2-tolyl)propionate,
n-butyl 3-amino-3-(3-tolyl)propionate,
n-butyl 3-amino-3-(4-tolyl)propionate,
n-butyl 3-amino-3-(2-chlorophenyl)propionate,
n-butyl 3-amino-3-(3-chlorophenyl)propionate,
n-butyl 3-amino-3-(4-chlorophenyl)propionate,
n-butyl 3-amino-3-(3,4-dichlorophenyl)propionate,
n-butyl 3-amino-3-(2-bromophenyl)propionate,
n-butyl 3-amino-3-(3-bromophenyl)propionate,
n-butyl 3-amino-3-(4-bromophenyl)propionate,
n-butyl 3-amino-3-(2-fluorophenyl)propionate,
n-butyl 3-amino-3-(3-fluorophenyl)propionate,
n-butyl 3-amino-3-(4-fluorophenyl)propionate,
n-butyl 3-amino-3-(4-iodophenyl)propionate,
n-butyl 3-amino-3-(2-methoxyphenyl)propionate,
n-butyl 3-amino-3-(3-methoxyphenyl)propionate,
n-butyl 3-amino-3-(4-methoxyphenyl)propionate and
n-butyl 3-amino-3-(3,4-methylenedioxyphenyl)propionate
and the like, particularly preferably
n-butyl 3-amino-3-(2-tolyl)propionate,
n-butyl 3-amino-3-(3-tolyl)propionate,
n-butyl 3-amino-3-(4-tolyl)propionate,
n-butyl 3-amino-3-(2-chlorophenyl)propionate,
n-butyl 3-amino-3-(3-chlorophenyl)propionate,
n-butyl 3-amino-3-(4-chlorophenyl)propionate,
n-butyl 3-amino-3-(2-bromophenyl)propionate,
n-butyl 3-amino-3-(3-bromophenyl)propionate,
n-butyl 3-amino-3-(4-bromophenyl)propionate,
n-butyl 3-amino-3-(2-fluorophenyl)propionate,
n-butyl 3-amino-3-(3-fluorophenyl)propionate,
n-butyl 3-amino-3-(4-fluorophenyl)propionate,
n-butyl 3-amino-3-(2-methoxyphenyl)propionate,
n-butyl 3-amino-3-(3-methoxyphenyl)propionate,
n-butyl 3-amino-3-(4-methoxyphenyl)propionate and
n-butyl 3-amino-3-(3,4-methylenedioxyphenyl)propionate.

Next, the process for preparing the n-alkyl 3-amino-3-arylpropionate represented by the formula (IV-a) according to the present invention will be explained.

### (A) First step

The arylaldehyde to be used in the first step of the present invention is represented by the above-mentioned formula (II). In the formula (II), Ar represents an aryl group which may have a substituent(s).

The aryl group in the above-mentioned "an aryl group which may have a substituent(s)" is a substituted phenyl group, naphthyl gruop, pyridyl group, quinolyl group, thienyl group, or furyl group. Also, as the substituent(s) in the aryl group which may have a substituent(s), there may be mentioned an alkyl group having 1 to 4 carbon atoms such as methyl group, ethyl group, propyl group, butyl group, etc. (incidentally, these groups contain various kinds of isomers.); hydroxyl group; a halogen atom such as a chlorine atom, a bromine atom, an iodine atom, a fluorine atom, etc.; an alkoxy group having 2 to 4 carbon atoms such as an ethoxy group, etc. (incidentally, these groups contain various kinds of isomers.); an alkylenedioxy group having 1 to 4 carbon atoms such as a methylenedioxy group, etc.; nitro group, cyano group, trifluoromethyl group, etc.

Such "an aryl group which may have a substituent(s)" may be specifically mentioned, for example, 2-tolyl group, 3-tolyl group, 4-tolyl group, 2,3-xylyl group, 2,6-xylyl group, 2,4-xylyl group, 3,4-xylyl group, mesityl group, 2-hydroxyphenyl group, 3-hydroxyphenyl group, 4-hydroxyphenyl group, 2,3-dihydroxyphenyl group, 2,4-dihydroxyphenyl group, 3,4-dihydroxyphenyl group, 2-chlorophenyl group, 3-chlorophenyl group, 4-chlorophenyl group, 2,3-dichlorophenyl group, 2,4-dichlorophenyl group, 3,4-dichlorophenyl group, 3,5-dichlorophenyl group, 2-bromophenyl group, 3-bromophenyl group, 4-bromophenyl group, 2-iodophenyl group, 3-iodophenyl group, 4-iodophenyl group, 2-fluorophenyl group, 3-fluorophenyl group, 4-fluorophenyl group, 2,3-difluorophenyl group, 2,4-difluorophenyl group, 3,4-difluorophenyl group, 3-bromo-5-chloro-2-hydroxyphenyl group, 2-methoxyphenyl group, 3-methoxyphenyl group, 4-methoxyphenyl group, 2,3-dimethoxyphenyl group, 2,4-dimethoxyphenyl group, 3,4-dimethoxyphenyl group, 3,5-dimethoxyphenyl group, 3,4-methylenedioxyphenyl group, 4-ethoxyphenyl group, 4-butoxyphenyl group, 4-isopropoxyphenyl group, 1-phenoxyphenyl group, 4-benzyloxyphenyl group, 4-trifluoromethylphenyl group, 2-nitrophenyl group, 3-nitrophenyl group, 4-nitrophenyl group, 4-cyanophenyl group, 4-methoxycarbonylphenyl group, 1-naphthyl gruop, 2-naphthyl gruop, 2-pyridyl group, 3-pyridyl group, 4-pyridyl group, 3-thienyl group, 3-furyl group, 3-quinolyl group, etc., preferably phenyl group, 2-tolyl group, 3-tolyl group, 4-tolyl group, 2,3-xylyl group, 2-hydroxyphenyl group, 3-hydroxyphenyl group, 4-hydroxyphenyl group, 2,3-dihydroxyphenyl group, 2,4-dihydroxyphenyl group, 3,4-dihydroxyphenyl group, 2-chlorophenyl group, 3-chlorophenyl group, 4-chlorophenyl group, 2,3-dichlorophenyl group, 2,4-dichlorophenyl group, 3,4-dichlorophenyl group, 3,5-dichlorophenyl group, 2-bromophenyl group, 3-bromophenyl group, 4-bromophenyl group, 2-iodophenyl group, 3-iodophenyl group, 4-iodophenyl group, 2-fluorophenyl group, 3-fluorophenyl group, 4-fluorophenyl group, 3,4-difluorophenyl group, 3-bromo-5-chloro-2-hydroxyphenyl group, 2-methoxyphenyl group, 3-methoxyphenyl group, 4-methoxyphenyl group, 2,3-dimethoxyphenyl group, 2,4-dimethoxyphenyl group, 3,4-dimethoxyphenyl group, 3,5-dimethoxyphenyl group, 3,4-methylenedioxyphenyl group, 4-ethoxyphenyl group, 4-trifluoromethylphenyl group, 4-nitrophenyl group, 4-cyanophenyl group, 1-naphthyl gruop, 2-naphthyl gruop, 2-pyridyl group, 3-pyridyl group, 4-pyridyl group, more preferably phenyl group, 2-tolyl group, 3-tolyl group, 4-tolyl group, 2,3-xylyl group, 4-hydroxyphenyl group, 3,4-dihydroxyphenyl group, 2-chlorophenyl group, 3-chlorophenyl group, 4-chlorophenyl group, 2,3-dichlorophenyl group, 2,4-dichlorophenyl group, 3,4-dichlorophenyl group, 3,5-dichlorophenyl group, 2-bromophenyl group, 3-bromophenyl group, 4-bromophenyl group, 4-iodophenyl group, 2-fluorophenyl group, 3-fluorophenyl group, 4-fluorophenyl group, 3,4-difluorophenyl group, 2-iodophenyl group, 3-iodophenyl group, 4-iodophenyl group, 2-methoxyphenyl group, 3-methoxyphenyl group, 4-methoxyphenyl group, 2,3-dimethoxyphenyl group, 2,4-dimethoxyphenyl group, 3,4-dimethoxyphenyl group, 3,5-dimethoxyphenyl group, 3,4-methylenedioxyphenyl group, 4-trifluoromethylphenyl group, 4-nitrophenyl group, 1-naphthyl gruop, 2-naphthyl gruop, 3-pyridyl group, particularly preferably phenyl group, 2-tolyl group, 3-tolyl group, 4-tolyl group, 2,3-xylyl group, 2-chlorophenyl group, 3-chlorophenyl group, 4-chlorophenyl group, 2,3-dichlorophenyl group, 2,4-dichlorophenyl group, 3,4-dichlorophenyl group, 3,5-dichlorophenyl group, 2-bromophenyl group, 3-bromophenyl group, 4-bromophenyl group, 2-fluorophenyl group, 3-fluorophenyl group, 4-fluorophenyl group, 3,4-difluorophenyl group, 2-iodophenyl group, 3-iodophenyl group, 4-iodophenyl group, 2-methoxyphenyl group, 3-methoxyphenyl group, 4-methoxyphenyl group, 2,3-dimethoxyphenyl group, 3,4-dimethoxyphenyl group, 3,5-dimethoxyphenyl group or 3,4-methylenedioxyphenyl group.

An amount of the malonic acid to be used in the first step of the present invention is preferably 0.5 to 5.0 mol, more preferably 1.0 to 1.5 mol based on 1 mol of the arylaldehyde.

An amount of the ammonium acetate to be used in the first step of the present invention is preferably 1.0 to 5.0 mol, more preferably 1.0 to 3.0 mol based on 1 mol of the arylaldehyde.

As the organic solvent to be used in the first step of the present invention, an alcohol solvent and a nitrile solvent are preferred. The alcohol solvent may be mentioned, for example, methanol, ethanol, n-propyl alcohol, isopropyl alcohol, n-butyl alcohol, isobutyl alcohol, s-butyl alcohol, t-butyl alcohol, etc., preferably methanol, ethanol, isopropyl alcohol, more preferably ethanol, isopropyl alcohol are used. Also, as the nitrile solvent, there may be mentioned, for example, acetonitrile, propionitrile, etc. Incidentally, these the organic solvents may be used alone or in combination of two or more kinds.

An amount of the above-mentioned organic solvent is optionally adjusted depending on the uniformity or stirrability of the reaction mixture, and preferably 1 to 50 mL, more preferably 2 to 10 mL based on 1 g of the arylaldehyde.

The first step of the present invention can be carried out, for example, under nitrogen atmosphere, by the method of mixing an arylaldehyde, malonic acid, ammonium acetate and an alcoholic solvent, and reacting the same while stirring, and the like. The reaction temperature at the time is preferably 40 to 150°C, more preferably 50 to 100°C, and the reaction pressure is not specifically limited.

The 3-amino-3-arylpropionic acid obtained by the first step of the present invention is isolated and purified by, after completion of the reaction, a general method such as filtration, extraction, concentration, recrystallization, crystallization, column chromatography, etc., and then, used in the next second step.

### (B) Second step

As the acid catalyst to be used in the second step of the present invention, there may be mentioned, for example, hydrochloric acid, sulfuric acid, phosphoric acid, nitric acid, p-toluenesulfonic acid, etc., preferably hydrochloric acid, sulfuric acid, p-toluenesulfonic acid, more preferably hydrochloric acid, sulfuric acid are used. Incidentally, these acids may be used alone or in combination of two or more kinds in admixture.

An amount of the above-mentioned acid catalyst to be used is preferably 0.1 to 20 mol, more preferably 1.0 to 5.0 mol based on 1 mol of 3-amino-3-arylpropionic acid.

An amount of the above-mentioned n-propyl alcohol or n-butyl alcohol to be used is preferably 1 to 100 mol, more preferably 5 to 40 mol based on 1 mol of 3-amino-3-arylpropionic acid.

The second step of the present invention can be carried out, for example, by a method in which 3-amino-3-arylpropionic acid and n-propyl alcohol or n-butyl alcohol are mixed under nitrogen atmosphere, and these materials were reacted under stirring, and the like. The reaction temperature at the time is preferably 40 to 100°C, more preferably 50 to 70°C, and the reaction pressure is not specifically limited.

The 3-amino-3-arylpropionic acid n-propyl ester or n-butyl ester obtained by the second step of the present invention can be isolated and purified by a conventional method, for example, such as filtration, extraction, concentration, recrystallization, crystallization, column chromatography, etc. after completion of the reaction.

Next, a hydrolysis reaction of n-alkyl 3-amino-3-arylpropionate represented by the formula (IV-a) of the present invention is explained. In this hydrolysis reaction, for example, as shown by the following formula (V) : 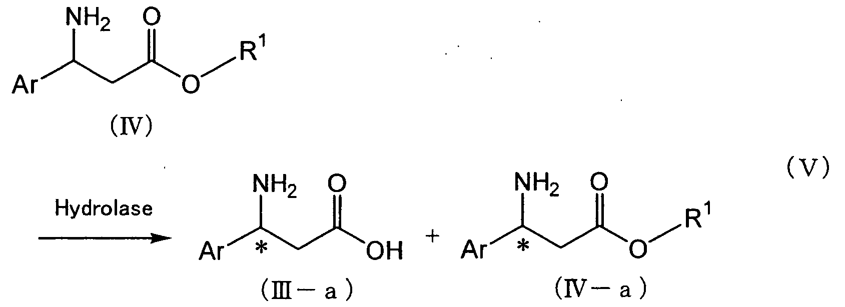 wherein Ar, R¹ and * have the same meanings as defined above,
only one enantiomer of the racemic mixtures of n-alkyl 3-amino-3-arylpropionate (hereinafter sometimes referred to as Compound (IV-a).) represented by the above-mentioned formula (IV-a) is selectively hydrolyzed in the presence of a hydrolase, to form an optically active (S or R)-3-amino-3-arylpropionic acid (hereinafter sometimes referred to as Compound (III-a).) represented by the formula (III-a), and simultaneously to obtain an unreacted optically active (R or S)-n-alkyl 3-amino-3-arylpropionate (hereinafter sometimes referred to as Compound (IV-a).) represented by the formula (IV-a). Incidentally, Compound (III-a) and Compound (IV-a) have the reverse absolute configuration to each other.

Ar of Compound (IV) is as mentioned above.

Compound (IV) to be used in the hydrolysis reaction of the present invention can be prepared by, for example, reacting an aldehyde compound, malonic acid and ammonium acetate, and then, further reacting n-propyl alcohol or n-butyl alcohol in the presence of an acid catalyst.

Specific examples of the n-propyl ester of Compound (IV) having the above-mentioned Ar may be mentioned, for example,
n-propyl 3-amino-3-phenylpropionate,
n-propyl 3-amino-3-(2-tolyl)propionate,
n-propyl 3-amino-3-(3-tolyl)propionate,
n-propyl 3-amino-3-(4-tolyl)propionate,
n-propyl 3-amino-3-(2,3-xylyl)propionate,
n-propyl 3-amino-3-(4-ethylphenyl)propionate,
n-propyl 3-amino-3-(2-chlorophenyl)propionate,
n-propyl 3-amino-3-(3-chlorophenyl)propionate,
n-propyl 3-amino-3-(4-chlorophenyl)propionate,
n-propyl 3-amino-3-(2,3-dichlorophenyl)propionate,
n-propyl 3-amino-3-(2,4-dichlorophenyl)propionate,
n-propyl 3-amino-3-(3,4-dichlorophenyl)propionate,
n-propyl 3-amino-3-(2-bromophenyl)propionate,
n-propyl 3-amino-3-(3-bromophenyl)propionate,
n-propyl 3-amino-3-(4-bromophenyl)propionate,
n-propyl 3-amino-3-(2-fluorophenyl)propionate,
n-propyl 3-amino-3-(3-fluorophenyl)propionate,
n-propyl 3-amino-3-(4-fluorophenyl)propionate,
n-propyl 3-amino-3-(2-iodophenyl)propionate,
n-propyl 3-amino-3-(3-iodophenyl)propionate,
n-propyl 3-amino-3-(4-iodophenyl)propionate,
n-propyl 3-amino-3-(2-methoxyphenyl)propionate,
n-propyl 3-amino-3-(3-methoxyphenyl)propionate,
n-propyl 3-amino-3-(4-methoxyphenyl)propionate,
n-propyl 3-amino-3-(4-ethoxyphenyl)propionate,
n-propyl 3-amino-3-(2-hydroxyphenyl)propionate,
n-propyl 3-amino-3-(3-hydroxyphenyl)propionate,
n-propyl 3-amino-3-(4-hydroxyphenyl)propionate,
n-propyl 3-amino-3-(4-nitrophenyl)propionate,
n-propyl 3-amino-3-(4-cyanophenyl)propionate,
n-propyl 3-amino-3-(3,4-dimethoxyphenyl)propionate,
n-propyl 3-amino-3-(3,4-methylenedioxyphenyl)propionate,
n-propyl 3-amino-3-(1-phenoxyphenyl)propionate,
n-propyl 3-amino-3-(4-benzyloxyphenyl)propionate,
n-propyl 3-amino-3-(1-naphthyl)propionate,
n-propyl 3-amino-3-(2-naphthyl)propionate,
n-propyl 3-amino-3-(2-pyridyl)propionate,
n-propyl 3-amino-3-(3-pyridyl)propionate,
n-propyl 3-amino-3-(4-pyridyl)propionate,
n-propyl 3-amino-3-(2-thienyl)propionate,
n-propyl 3-amino-3-(2-furyl)propionate,
n-propyl 3-amino-3-(2-quinolyl)propionate and
n-propyl 3-amino-3-(3-bromo-5-chloro-2-hydroxyphenyl)propionate,
and the like, preferably
n-propyl 3-amino-3-phenylpropionate,
n-propyl 3-amino-3-(2-tolyl)propionate,
n-propyl 3-amino-3-(3-tolyl)propionate,
n-propyl 3-amino-3-(4-tolyl)propionate,
n-propyl 3-amino-3-(2,3-xylyl)propionate,
n-propyl 3-amino-3-(2-chlorophenyl)propionate,
n-propyl 3-amino-3-(3-chlorophenyl)propionate,
n-propyl 3-amino-3-(4-chlorophenyl)propionate,
n-propyl 3-amino-3-(3,4-dichlorophenyl)propionate,
n-propyl 3-amino-3-(2-bromophenyl)propionate,
n-propyl 3-amino-3-(3-bromophenyl)propionate,
n-propyl 3-amino-3-(4-bromophenyl)propionate,
n-propyl 3-amino-3-(2-fluorophenyl)propionate,
n-propyl 3-amino-3-(3-fluorophenyl)propionate,
n-propyl 3-amino-3-(4-fluorophenyl)propionate,
n-propyl 3-amino-3-(2-iodophenyl)propionate,
n-propyl 3-amino-3-(3-iodophenyl)propionate,
n-propyl 3-amino-3-(4-iodophenyl)propionate,
n-propyl 3-amino-3-(2-methoxyphenyl)propionate,
n-propyl 3-amino-3-(3-methoxyphenyl)propionate,
n-propyl 3-amino-3-(4-methoxyphenyl)propionate,
n-propyl 3-amino-3-(4-ethoxyphenyl)propionate,
n-propyl 3-amino-3-(4-hydroxyphenyl)propionate,
n-propyl 3-amino-3-(4-nitrophenyl)propionate,
n-propyl 3-amino-3-(4-cyanophenyl)propionate,
n-propyl 3-amino-3-(3,4-dimethoxyphenyl)propionate,
n-propyl 3-amino-3-(3,4-methylenedioxyphenyl)propionate,
n-propyl 3-amino-3-(1-naphthyl)propionate,
n-propyl 3-amino-3-(2-pyridyl)propionate,
n-propyl 3-amino-3-(3-pyridyl)propionate and
n-propyl 3-amino-3-(4-pyridyl)propionate,
and the like, more preferably
n-propyl 3-amino-3-phenylpropionate,
n-propyl 3-amino-3-(2-tolyl)propionate,
n-propyl 3-amino-3-(3-tolyl)propionate,
n-propyl 3-amino-3-(4-tolyl)propionate,
n-propyl 3-amino-3-(2-chlorophenyl)propionate,
n-propyl 3-amino-3-(3-chlorophenyl)propionate,
n-propyl 3-amino-3-(4-chlorophenyl)propionate,
n-propyl 3-amino-3-(3,4-dichlorophenyl)propionate,
n-propyl 3-amino-3-(2-bromophenyl)propionate,
n-propyl 3-amino-3-(3-bromophenyl)propionate,
n-propyl 3-amino-3-(4-bromophenyl)propionate,
n-propyl 3-amino-3-(2-fluorophenyl)propionate,
n-propyl 3-amino-3-(3-fluorophenyl)propionate,
n-propyl 3-amino-3-(4-fluorophenyl)propionate,
n-propyl 3-amino-3-(4-iodophenyl)propionate,
n-propyl 3-amino-3-(2-methoxyphenyl)propionate,
n-propyl 3-amino-3-(3-methoxyphenyl)propionate,
n-propyl 3-amino-3-(4-methoxyphenyl)propionate,
n-propyl 3-amino-3-(4-hydroxyphenyl)propionate,
n-propyl 3-amino-3-(3,4-methylenedioxyphenyl)propionate,
n-propyl 3-amino-3-(3-pyridyl)propionate and
n-propyl 3-amino-3-(4-pyridyl)propionate,
and the like, particularly preferably
n-propyl 3-amino-3-phenylpropionate,
n-propyl 3-amino-3-(2-tolyl)propionate,
n-propyl 3-amino-3-(3-tolyl)propionate,
n-propyl 3-amino-3-(4-tolyl)propionate,
n-propyl 3-amino-3-(2-chlorophenyl)propionate,
n-propyl 3-amino-3-(3-chlorophenyl)propionate,
n-propyl 3-amino-3-(4-chlorophenyl)propionate,
n-propyl 3-amino-3-(2-bromophenyl)propionate,
n-propyl 3-amino-3-(3-bromophenyl)propionate,
n-propyl 3-amino-3-(4-bromophenyl)propionate,
n-propyl 3-amino-3-(2-fluorophenyl)propionate,
n-propyl 3-amino-3-(3-fluorophenyl)propionate,
n-propyl 3-amino-3-(4-fluorophenyl)propionate,
n-propyl 3-amino-3-(2-methoxyphenyl)propionate,
n-propyl 3-amino-3-(3-methoxyphenyl)propionate,
n-propyl 3-amino-3-(4-methoxyphenyl)propionate and
n-propyl 3-amino-3-(3,4-methylenedioxyphenyl)propionate.

Also, specific examples of an n-butyl ester of Compound (IV) having the above-mentioned Ar may be mentioned, for example,
n-butyl 3-amino-3-phenylpropionate,
n-butyl 3-amino-3-(2-tolyl)propionate,
n-butyl 3-amino-3-(3-tolyl)propionate,
n-butyl 3-amino-3-(4-tolyl)propionate,
n-butyl 3-amino-3-(2,3-xylyl)propionate,
n-butyl 3-amino-3-(2-chlorophenyl)propionate,
n-butyl 3-amino-3-(3-chlorophenyl)propionate,
n-butyl 3-amino-3-(4-chlorophenyl)propionate,
n-butyl 3-amino-3-(2,3-dichlorophenyl)propionate,
n-butyl 3-amino-3-(2,4-dichlorophenyl)propionate,
n-butyl 3-amino-3-(3,4-dichlorophenyl)propionate,
n-butyl 3-amino-3-(3,5-dichlorophenyl)propionate,
n-butyl 3-amino-3-(2-bromophenyl)propionate,
n-butyl 3-amino-3-(3-bromophenyl)propionate,
n-butyl 3-amino-3-(4-bromophenyl)propionate,
n-butyl 3-amino-3-(2-fluorophenyl)propionate,
n-butyl 3-amino-3-(3-fluorophenyl)propionate,
n-butyl 3-amino-3-(4-fluorophenyl)propionate,
n-butyl 3-amino-3-(3,4-difluorophenyl)propionate,
n-butyl 3-amino-3-(2-iodophenyl)propionate,
n-butyl 3-amino-3-(3-iodophenyl)propionate,
n-butyl 3-amino-3-(4-iodophenyl)propionate,
n-butyl 3-amino-3-(2-methoxyphenyl)propionate,
n-butyl 3-amino-3-(3-methoxyphenyl)propionate,
n-butyl 3-amino-3-(4-methoxyphenyl)propionate,
n-butyl 3-amino-3-(2,3-dimethoxyphenyl)propionate,
n-butyl 3-amino-3-(3,4-dimethoxyphenyl)propionate,
n-butyl 3-amino-3-(3,5-dimethoxyphenyl)propionate and
n-butyl 3-amino-3-(3,4-methylenedioxyphenyl)propionate,
and the like, preferably
n-butyl 3-amino-3-phenylpropionate,
n-butyl 3-amino-3-(2-tolyl)propionate,
n-butyl 3-amino-3-(3-tolyl)propionate,
n-butyl 3-amino-3-(4-tolyl)propionate,
n-butyl 3-amino-3-(2,3-xylyl)propionate,
n-butyl 3-amino-3-(2-chlorophenyl)propionate,
n-butyl 3-amino-3-(3-chlorophenyl)propionate,
n-butyl 3-amino-3-(4-chlorophenyl)propionate,
n-butyl 3-amino-3-(3,4-dichlorophenyl)propionate,
n-butyl 3-amino-3-(2-bromophenyl)propionate,
n-butyl 3-amino-3-(3-bromophenyl)propionate,
n-butyl 3-amino-3-(4-bromophenyl)propionate,
n-butyl 3-amino-3-(2-fluorophenyl)propionate,
n-butyl 3-amino-3-(3-fluorophenyl)propionate,
n-butyl 3-amino-3-(4-fluorophenyl)propionate,
n-butyl 3-amino-3-(2-iodophenyl)propionate,
n-butyl 3-amino-3-(3-iodophenyl)propionate,
n-butyl 3-amino-3-(4-iodophenyl)propionate,
n-butyl 3-amino-3-(2-methoxyphenyl)propionate,
n-butyl 3-amino-3-(3-methoxyphenyl)propionate,
n-butyl 3-amino-3-(4-methoxyphenyl)propionate,
n-butyl 3-amino-3-(2,3-dimethoxyphenyl)propionate and
n-butyl 3-amino-3-(3,4-methylenedioxyphenyl)propionate,
and the like, more preferably
n-butyl 3-amino-3-phenylpropionate,
n-butyl 3-amino-3-(2-tolyl)propionate,
n-butyl 3-amino-3-(3-tolyl)propionate,
n-butyl 3-amino-3-(4-tolyl)propionate,
n-butyl 3-amino-3-(2-chlorophenyl)propionate,
n-butyl 3-amino-3-(3-chlorophenyl)propionate,
n-butyl 3-amino-3-(4-chlorophenyl)propionate,
n-butyl 3-amino-3-(3,4-dichlorophenyl)propionate,
n-butyl 3-amino-3-(2-bromophenyl)propionate,
n-butyl 3-amino-3-(3-bromophenyl)propionate,
n-butyl 3-amino-3-(4-bromophenyl)propionate,
n-butyl 3-amino-3-(2-fluorophenyl)propionate,
n-butyl 3-amino-3-(3-fluorophenyl)propionate,
n-butyl 3-amino-3-(4-fluorophenyl)propionate,
n-butyl 3-amino-3-(4-iodophenyl)propionate,
n-butyl 3-amino-3-(2-methoxyphenyl)propionate,
n-butyl 3-amino-3-(3-methoxyphenyl)propionate,
n-butyl 3-amino-3-(4-methoxyphenyl)propionate and
n-butyl 3-amino-3-(3,4-methylenedioxyphenyl)propionate,
and the like, particularly preferably
n-butyl 3-amino-3-phenylpropionate,
n-butyl 3-amino-3-(2-tolyl)propionate,
n-butyl 3-amino-3-(3-tolyl)propionate,
n-butyl 3-amino-3-(4-tolyl)propionate,
n-butyl 3-amino-3-(2-chlorophenyl)propionate,
n-butyl 3-amino-3-(3-chlorophenyl)propionate,
n-butyl 3-amino-3-(4-chlorophenyl)propionate,
n-butyl 3-amino-3-(2-bromophenyl)propionate,
n-butyl 3-amino-3-(3-bromophenyl)propionate,
n-butyl 3-amino-3-(4-bromophenyl)propionate,
n-butyl 3-amino-3-(2-fluorophenyl)propionate,
n-butyl 3-amino-3-(3-fluorophenyl)propionate,
n-butyl 3-amino-3-(4-fluorophenyl)propionate,
n-butyl 3-amino-3-(2-methoxyphenyl)propionate,
n-butyl 3-amino-3-(3-methoxyphenyl)propionate,
n-butyl 3-amino-3-(4-methoxyphenyl)propionate and
n-butyl 3-amino-3-(3,4-methylenedioxyphenyl)propionate.

As the hydrolase to be used in the hydrolysis of the present invention, there may be mentioned, for example, protease, esterase, lipase, etc., preferably lipase of microorganism which can be isolated from yeast or bacteria, more preferably lipase (for example, Amano PS (available from Amano Enzyme Co.), etc.) originated from *Burkholderia cepacia* (*Pseudomonas cepacia*) may be used. Incidentally, as these hydrolases, a commercially available product can be used in a natural form or a fixed enzyme as such, and they may be used singly or in admixture of two or more kinds. Also, an enzyme-fixing agent to be contained in a commercially available product may be previously removed and then used.

An amount of the above-mentioned hydrolase is preferably 0.1 to 1000 mg, more preferably 1 to 200 mg based on 1 g of Compound (IV-a).

Hydrolysis reaction of the present invention can be carried out in a mixed solvent of an organic solvent and a buffer.

In the hydrolysis reaction of the present invention, water may be added. As the water, purified water such as deionized water, distilled water, etc. is preferably used. Incidentally, when water is used as the solvent, weak base such as potassium hydrogen carbonate, sodium hydrogen carbonate, etc. may be present in the reaction system to neutralize the formed Compound (III-a). An amount of the above-mentioned weak base is preferably 0.5 to 1.0 mol based on 1 mol of Compound (III-a).

As the above-mentioned buffer, there may be mentioned, for example, an aqueous solution of an inorganic acid salt such as an aqueous sodium phosphate solution, an aqueous potassium phosphate solution, etc.; an aqueous solution of an organic acid salt such as an aqueous sodium acetate solution, an aqueous ammonium acetate solution, an aqueous sodium citrate solution, etc., preferably an aqueous solution of an inorganic acid salt, more preferably an aqueous sodium phosphate solution, an aqueous potassium phosphate solution, an aqueous ammonium acetate solution is/are used. These buffers may be used used singly or in admixture of two or more kinds.

A concentration of the buffer is preferably 0.01 to 2 mol/L, more preferably 0.05 to 0.5 mol/L, and a pH of the buffer is preferably 4 to 9, more preferably 7 to 8.5.

As the above-mentioned organic solvent, there may be mentioned, for example, aliphatic hydrocarbons such as n-pentane, n-hexane, n-heptane, n-octane, cyclopentane, cyclohexane, cyclopentane, etc.; aromatic hydrocarbons such as benzene, toluene, xylene, etc.; ethers such as diethyl ether, t-butyl methyl ether, diisopropyl ether, tetrahydrofuran, 1,4-dioxane, etc., preferably n-hexane, n-heptane, cyclopentane, cyclohexane, toluene, diisopropyl ether, t-butyl methyl ether, tetrahydrofuran, more preferably n-hexane, cyclohexane, toluene, diisopropyl ether, t-butyl methyl ether, particularly preferably cyclohexane, toluene, t-butyl methyl ether is/are used.

An amount of the solvent (a mixed solvent of the organic solvent and the buffer) to be used in the hydrolysis reaction of the present invention is preferably 2 to 200 mL, more preferably 5 to 80 mL based on 1 g of Compound (IV-a).

In the hydrolysis reaction of the present invention, an amount of the organic solvent to be used when the mixed solvent of the organic solvent and the buffer is used as a solvent is preferably 0.1 to 10 mL, more preferably 0.5 to 5 mL based on 1 mL of the buffer.

The hydrolysis reaction of the present invention can be carried out, for example, by a method in which Compound (IV-a), the hydrolase and the solvent (a mixed solvent of the organic solvent and the buffer) are mixed and reacted while stirring, and the like. A reaction temperature at that time is preferably 0 to 80°C, more preferably 10 to 50°C, particularly preferably 30 to 45°C, and a reaction pressure is not specifically limited.

From Compound (III-a) and Compound (IV-a) obtained by the hydrolysis reaction of the present invention, for example, after completion of the reaction, Compound (III-a) can be obtained by adding a suitable organic solvent (for example, acetonitrile, acetone, etc.) to the reaction mixture and then filtering the same, and Compound (IV-a) can be also obtained by controlling a pH of the filtrate, and then concentrating the organic layer. Also, filtration or addition of sodium chloride before obtaining the desired compound may be carried out to improve a recovery rate of Compound (III-a). Incidentally, these compounds may be further purified by a conventional manner such as crystallization, recrystallization, distillation, column chromatography, etc.

Also, Compound (III-a) can be converted into a corresponding optically active 3-amino-3-arylpropionic acid n-propyl ester or n-butyl ester by reacting with n-propyl alcohol or n-butyl alcohol in the presence of an acid catalyst according to the above-mentioned method.

Specific examples of Compound (III-a) obtained by the hydrolysis reaction of the present invention may be mentioned, for example,
optically active (S or R)-3-amino-3-phenylpropionic acid,
optically active (S or R)-3-amino-3-(2-tolyl)propionic acid,
optically active (S or R)-3-amino-3-(3-tolyl)propionic acid,
optically active (S or R)-3-amino-3-(4-tolyl)propionic acid,
optically active (S or R)-3-amino-3-(2,3-xylyl)propionic acid,
optically active (S or R)-3-amino-3-(4-ethylphenyl)propionic acid,
optically active (S or R)-3-amino-3-(2-chlorophenyl)propionic acid,
optically active (S or R)-3-amino-3-(3-chlorophenyl)propionic acid,
optically active (S or R)-3-amino-3-(4-chlorophenyl)propionic acid,
optically active (S or R)-3-amino-3-(2,3-dichlorophenyl)propionic acid,
optically active (S or R)-3-amino-3-(2,4-dichlorophenyl)propionic acid,
optically active (S or R)-3-amino-3-(3,4-dichlorophenyl)propionic acid,
optically active (S or R)-3-amino-3-(2-bromophenyl)propionic acid,
optically active (S or R)-3-amino-3-(3-bromophenyl)propionic acid,
optically active (S or R)-3-amino-3-(4-bromophenyl)propionic acid,
optically active (S or R)-3-amino-3-(2-fluorophenyl)propionic acid,
optically active (S or R)-3-amino-3-(3-fluorophenyl)propionic acid,
optically active (S or R)-3-amino-3-(4-fluorophenyl)propionic acid,
optically active (S or R)-3-amino-3-(2-iodophenyl)propionic acid,
optically active (S or R)-3-amino-3-(3-iodophenyl)propionic acid,
optically active (S or R)-3-amino-3-(4-iodophenyl)propionic acid,
optically active (S or R)-3-amino-3-(2-methoxyphenyl)propionic acid,
optically active (S or R)-3-amino-3-(3-methoxyphenyl)propionic acid,
optically active (S or R)-3-amino-3-(4-methoxyphenyl)propionic acid,
optically active (S or R)-3-amino-3-(4-ethoxyphenyl)propionic acid,
optically active (S or R)-3-amino-3-(2-hydroxyphenyl)propionic acid,
optically active (S or R)-3-amino-3-(3-hydroxyphenyl)propionic acid,
optically active (S or R)-3-amino-3-(4-hydroxyphenyl)propionic acid,
optically active (S or R)-3-amino-3-(4-nitrophenyl)propionic acid,
optically active (S or R)-3-amino-3-(4-cyanophenyl)propionic acid,
optically active (S or R)-3-amino-3-(3,4-dimethoxyphenyl)propionic acid,
optically active (S or R)-3-amino-3-(3,4-methylenedioxyphenyl)propionic acid,
optically active (S or R)-3-amino-3-(1-phenoxyphenyl)propionic acid,
optically active (S or R)-3-amino-3-(4-benzyloxyphenyl)propionic acid,
optically active (S or R)-3-amino-3-(1-naphthyl)propionic acid,
optically active (S or R)-3-amino-3-(2-naphthyl)propionic acid,
optically active (S or R)-3-amino-3-(2-pyridyl)propionic acid,
optically active (S or R)-3-amino-3-(3-pyridyl)propionic acid,
optically active (S or R)-3-amino-3-(4-pyridyl)propionic acid,
optically active (S or R)-3-amino-3-(2-thienyl)propionic acid,
optically active (S or R)-3-amino-3-(2-furyl)propionic acid,
optically active (S or R)-3-amino-3-(2-quinolyl)propionic acid and
optically active (S or R)-3-amino-3-(3-bromo-5-chloro-2-hydroxyphenyl)propionic acid,
and the like, preferably
optically active (S or R)-3-amino-3-phenylpropionic acid,
optically active (S or R)-3-amino-3-(2-tolyl)propionic acid,
optically active (S or R)-3-amino-3-(3-tolyl)propionic acid,
optically active (S or R)-3-amino-3-(4-tolyl)propionic acid,
optically active (S or R)-3-amino-3-(2,3-xylyl)propionic acid,
optically active (S or R)-3-amino-3-(4-ethylphenyl)propionic acid,
optically active (S or R)-3-amino-3-(2-chlorophenyl)propionic acid,
optically active (S or R)-3-amino-3-(3-chlorophenyl)propionic acid,
optically active (S or R)-3-amino-3-(4-chlorophenyl)propionic acid,
optically active (S or R)-3-amino-3-(3,4-dichlorophenyl)propionic acid,
optically active (S or R)-3-amino-3-(2-bromophenyl)propionic acid,
optically active (S or R)-3-amino-3-(3-bromophenyl)propionic acid,
optically active (S or R)-3-amino-3-(4-bromophenyl)propionic acid,
optically active (S or R)-3-amino-3-(2-fluorophenyl)propionic acid,
optically active (S or R)-3-amino-3-(3-fluorophenyl)propionic acid,
optically active (S or R)-3-amino-3-(4-fluorophenyl)propionic acid,
optically active (S or R)-3-amino-3-(2-iodophenyl)propionic acid,
optically active (S or R)-3-amino-3-(3-iodophenyl)propionic acid,
optically active (S or R)-3-amino-3-(4-iodophenyl)propionic acid,
optically active (S or R)-3-amino-3-(2-methoxyphenyl)propionic acid,
optically active (S or R)-3-amino-3-(3-methoxyphenyl)propionic acid,
optically active (S or R)-3-amino-3-(4-methoxyphenyl)propionic acid,
optically active (S or R)-3-amino-3-(4-ethoxyphenyl)propionic acid,
optically active (S or R)-3-amino-3-(4-hydroxyphenyl)propionic acid,
optically active (S or R)-3-amino-3-(4-nitrophenyl)propionic acid,
optically active (S or R)-3-amino-3-(4-cyanophenyl)propionic acid,
optically active (S or R)-3-amino-3-(3,4-dimethoxyphenyl)propionic acid,
optically active (S or R)-3-amino-3-(3,4-methylenedioxyphenyl)propionic acid,
optically active (S or R)-3-amino-3-(1-naphthyl)propionic acid,
optically active (S or R)-3-amino-3-(2-pyridyl)propionic acid,
optically active (S or R)-3-amino-3-(3-pyridyl)propionic acid and
optically active (S or R)-3-amino-3-(4-pyridyl)propionic acid,
and the like, more preferably
optically active (S or R)-3-amino-3-phenylpropionic acid,
optically active (S or R)-3-amino-3-(2-tolyl)propionic acid,
optically active (S or R)-3-amino-3-(3-tolyl)propionic acid,
optically active (S or R)-3-amino-3-(4-tolyl)propionic acid,
optically active (S or R)-3-amino-3-(2-chlorophenyl)propionic acid,
optically active (S or R)-3-amino-3-(3-chlorophenyl)propionic acid,
optically active (S or R)-3-amino-3-(4-chlorophenyl)propionic acid,
optically active (S or R)-3-amino-3-(3,4-dichlorophenyl)propionic acid,
optically active (S or R)-3-amino-3-(2-bromophenyl)propionic acid,
optically active (S or R)-3-amino-3-(3-bromophenyl)propionic acid,
optically active (S or R)-3-amino-3-(4-bromophenyl)propionic acid,
optically active (S or R)-3-amino-3-(2-fluorophenyl)propionic acid,
optically active (S or R)-3-amino-3-(3-fluorophenyl)propionic acid,
optically active (S or R)-3-amino-3-(4-fluorophenyl)propionic acid,
optically active (S or R)-3-amino-3-(4-iodophenyl)propionic acid,
optically active (S or R)-3-amino-3-(2-methoxyphenyl)propionic acid,
optically active (S or R)-3-amino-3-(3-methoxyphenyl)propionic acid,
optically active (S or R)-3-amino-3-(4-methoxyphenyl)propionic acid,
optically active (S or R)-3-amino-3-(4-hydroxyphenyl)propionic acid,
optically active (S or R)-3-amino-3-(3,4-methylenedioxyphenyl)propionic acid,
optically active (S or R)-3-amino-3-(3-pyridyl)propionic acid and
optically active (S or R)-3-amino-3-(4-pyridyl)propionic acid,
and the like, particularly preferably
optically active (S or R)-3-amino-3-phenylpropionic acid,
optically active (S or R)-3-amino-3-(2-tolyl)propionic acid,
optically active (S or R)-3-amino-3-(3-tolyl)propionic acid,
optically active (S or R)-3-amino-3-(4-tolyl)propionic acid,
optically active (S or R)-3-amino-3-(2-chlorophenyl)propionic acid,
optically active (S or R)-3-amino-3-(3-chlorophenyl)propionic acid,
optically active (S or R)-3-amino-3-(4-chlorophenyl)propionic acid,
optically active (S or R)-3-amino-3-(2-bromophenyl)propionic acid,
optically active (S or R)-3-amino-3-(3-bromophenyl)propionic acid,
optically active (S or R)-3-amino-3-(4-bromophenyl)propionic acid,
optically active (S or R)-3-amino-3-(2-fluorophenyl)propionic acid,
optically active (S or R)-3-amino-3-(3-fluorophenyl)propionic acid,
optically active (S or R)-3-amino-3-(4-fluorophenyl)propionic acid,
optically active (S or R)-3-amino-3-(2-methoxyphenyl)propionic acid,
optically active (S or R)-3-amino-3-(3-methoxyphenyl)propionic acid,
optically active (S or R)-3-amino-3-(4-methoxyphenyl)propionic acid and
optically active (S or R)-3-amino-3-(3,4-methylenedioxyphenyl)propionic acid.

Specific examples of the n-propyl ester of the unreacted Compound (IV-a) (which has a reverse absolute configuration to that of Compound (III-a).) which did not reacted in the hydrolysis reaction of the present invention may be mentioned, for example,
optically active (R or S)-n-propyl 3-amino-3-phenylpropionate,
optically active (R or S)-n-propyl 3-amino-3-(2-tolyl)propionate,
optically active (R or S)-n-propyl 3-amino-3-(3-tolyl)propionate,
optically active (R or S)-n-propyl 3-amino-3-(4-tolyl)propionate,
optically active (R or S)-n-propyl 3-amino-3-(2,3-xylyl)propionate,
optically active (R or S)-n-propyl 3-amino-3-(4-ethylphenyl)propionate,
optically active (R or S)-n-propyl 3-amino-3-(2-chlorophenyl)propionate,
optically active (R or S)-n-propyl 3-amino-3-(3-chlorophenyl)propionate,
optically active (R or S)-n-propyl 3-amino-3-(4-chlorophenyl)propionate,
optically active (R or S)-n-propyl 3-amino-3-(2,3-dichlorophenyl)propionate,
optically active (R or S)-n-propyl 3-amino-3-(2,4-dichlorophenyl)propionate,
optically active (R or S)-n-propyl 3-amino-3-(3,4-dichlorophenyl)propionate,
optically active (R or S)-n-propyl 3-amino-3-(2-bromophenyl)propionate,
optically active (R or S)-n-propyl 3-amino-3-(3-bromophenyl)propionate,
optically active (R or S)-n-propyl 3-amino-3-(4-bromophenyl)propionate,
optically active (R or S)-n-propyl 3-amino-3-(2-fluorophenyl)propionate,
optically active (R or S)-n-propyl 3-amino-3-(3-fluorophenyl)propionate,
optically active (R or S)-n-propyl 3-amino-3-(4-fluorophenyl)propionate,
optically active (R or S)-n-propyl 3-amino-3-(2-iodophenyl)propionate,
optically active (R or S)-n-propyl 3-amino-3-(3-iodophenyl)propionate,
optically active (R or S)-n-propyl 3-amino-3-(4-iodophenyl)propionate,
optically active (R or S)-n-propyl 3-amino-3-(2-methoxyphenyl)propionate,
optically active (R or S)-n-propyl 3-amino-3-(3-methoxyphenyl)propionate,
optically active (R or S)-n-propyl 3-amino-3-(4-methoxyphenyl)propionate,
optically active (R or S)-n-propyl 3-amino-3-(4-ethoxyphenyl)propionate,
optically active (R or S)-n-propyl 3-amino-3-(2-hydroxyphenyl)propionate,
optically active (R or S)-n-propyl 3-amino-3-(3-hydroxyphenyl)propionate,
optically active (R or S)-n-propyl 3-amino-3-(4-hydroxyphenyl)propionate,
optically active (R or S)-n-propyl 3-amino-3-(4-nitrophenyl)propionate,
optically active (R or S)-n-propyl 3-amino-3-(4-cyanophenyl)propionate,
optically active (R or S)-n-propyl 3-amino-3-(3,4-dimethoxyphenyl)propionate,
optically active (R or S)-n-propyl 3-amino-3-(3,4-methylenedioxyphenyl)propionate,
optically active (R or S)-n-propyl 3-amino-3-(1-phenoxyphenyl)propionate,
optically active (R or S)-n-propyl 3-amino-3-(4-benzyloxyphenyl)propionate,
optically active (R or S)-n-propyl 3-amino-3-(1-naphthyl)propionate,
optically active (R or S)-n-propyl 3-amino-3-(2-pyridyl)propionate,
optically active (R or S)-n-propyl 3-amino-3-(3-pyridyl)propionate,
optically active (R or S)-n-propyl 3-amino-3-(4-pyridyl)propionate,
optically active (R or S)-n-propyl 3-amino-3-(2-thienyl)propionate,
optically active (R or S)-n-propyl 3-amino-3-(2-furyl)propionate,
optically active (R or S)-n-propyl 3-amino-3-(2-quinolyl)propionate and
optically active (R or S)-n-propyl 3-amino-3-(3-bromo-5-chloro-2-hydroxyphenyl)propionate
and the like, preferably
optically active (R or S)-n-propyl 3-amino-3-phenylpropionate,
optically active (R or S)-n-propyl 3-amino-3-(2-tolyl)propionate,
optically active (R or S)-n-propyl 3-amino-3-(3-tolyl)propionate,
optically active (R or S)-n-propyl 3-amino-3-(4-tolyl)propionate,
optically active (R or S)-n-propyl 3-amino-3-(2,3-xylyl)propionate,
optically active (R or S)-n-propyl 3-amino-3-(2-chlorophenyl)propionate,
optically active (R or S)-n-propyl 3-amino-3-(3-chlorophenyl)propionate,
optically active (R or S)-n-propyl 3-amino-3-(4-chlorophenyl)propionate,
optically active (R or S)-n-propyl 3-amino-3-(3,4-dichlorophenyl)propionate,
optically active (R or S)-n-propyl 3-amino-3-(2-bromophenyl)propionate,
optically active (R or S)-n-propyl 3-amino-3-(3-bromophenyl)propionate,
optically active (R or S)-n-propyl 3-amino-3-(4-bromophenyl)propionate,
optically active (R or S)-n-propyl 3-amino-3-(2-fluorophenyl)propionate,
optically active (R or S)-n-propyl 3-amino-3-(3-fluorophenyl)propionate,
optically active (R or S)-n-propyl 3-amino-3-(4-fluorophenyl)propionate,
optically active (R or S)-n-propyl 3-amino-3-(2-iodophenyl)propionate,
optically active (R or S)-n-propyl 3-amino-3-(3-iodophenyl)propionate,
optically active (R or S)-n-propyl 3-amino-3-(4-iodophenyl)propionate,
optically active (R or S)-n-propyl 3-amino-3-(2-methoxyphenyl)propionate,
optically active (R or S)-n-propyl 3-amino-3-(3-methoxyphenyl)propionate,
optically active (R or S)-n-propyl 3-amino-3-(4-methoxyphenyl)propionate,
optically active (R or S)-n-propyl 3-amino-3-(4-ethoxyphenyl)propionate,
optically active (R or S)-n-propyl 3-amino-3-(4-hydroxyphenyl)propionate,
optically active (R or S)-n-propyl 3-amino-3-(4-nitrophenyl)propionate,
optically active (R or S)-n-propyl 3-amino-3-(4-cyanophenyl)propionate,
optically active '(R or S)-n-propyl 3-amino-3-(3,4-dimethoxyphenyl)propionate,
optically active (R or S)-n-propyl 3-amino-3-(3,4-methylenedioxyphenyl)propionate,
optically active (R or S)-n-propyl 3-amino-3-(1-naphthyl)propionate,
optically active (R or S)-n-propyl 3-amino-3-(2-pyridyl)propionate,
optically active (R or S)-n-propyl 3-amino-3-(3-pyridyl)propionate and
optically active (R or S)-n-propyl 3-amino-3-(4-pyridyl)propionate,
and the like, more preferably
optically active (R or S)-n-propyl 3-amino-3-phenylpropionate,
optically active (R or S)-n-propyl 3-amino-3-(2-tolyl)propionate,
optically active (R or S)-n-propyl 3-amino-3-(3-tolyl)propionate,
optically active (R or S)-n-propyl 3-amino-3-(4-tolyl)propionate,
optically active (R or S)-n-propyl 3-amino-3-(2-chlorophenyl)propionate,
optically active (R or S)-n-propyl 3-amino-3-(3-chlorophenyl)propionate,
optically active (R or S)-n-propyl 3-amino-3-(4-chlorophenyl)propionate,
optically active (R or S)-n-propyl 3-amino-3-(3,4-dichlorophenyl)propionate,
optically active (R or S)-n-propyl 3-amino-3-(2-bromophenyl)propionate,
optically active (R or S)-n-propyl 3-amino-3-(3-bromophenyl)propionate,
optically active (R or S)-n-propyl 3-amino-3-(4-bromophenyl)propionate,
optically active (R or S)-n-propyl 3-amino-3-(2-fluorophenyl)propionate,
optically active (R or S)-n-propyl 3-amino-3-(3-fluorophenyl)propionate,
optically active (R or S)-n-propyl 3-amino-3-(4-fluorophenyl)propionate,
optically active (R or S)-n-propyl 3-amino-3-(4-iodophenyl)propionate,
optically active (R or S)-n-propyl 3-amino-3-(2-methoxyphenyl)propionate,
optically active (R or S)-n-propyl 3-amino-3-(3-methoxyphenyl)propionate,
optically active (R or S)-n-propyl 3-amino-3-(4-methoxyphenyl)propionate,
optically active (R or S)-n-propyl 3-amino-3-(4-ethoxyphenyl)propionate,
optically active (R or S)-n-propyl 3-amino-3-(4-hydroxyphenyl)propionate,
optically active (R or S)-n-propyl 3-amino-3-(3,4-methylenedioxyphenyl)propionate,
optically active (R or S)-n-propyl 3-amino-3-(3-pyridyl)propionate and
optically active (R or S)-n-propyl 3-amino-3-(4-pyridyl)propionate,
and the like, particularly preferably
optically active (R or S)-n-propyl 3-amino-3-phenylpropionate,
optically active (R or S)-n-propyl 3-amino-3-(2-tolyl)propionate,
optically active (R or S)-n-propyl 3-amino-3-(3-tolyl)propionate,
optically active (R or S)-n-propyl 3-amino-3-(4-tolyl)propionate
optically active (R or S)-n-propyl 3-amino-3-(2-chlorophenyl)propionate,
optically active (R or S)-n-propyl 3-amino-3-(3-chlorophenyl)propionate,
optically active (R or S)-n-propyl 3-amino-3-(4-chlorophenyl)propionate,
optically active (R or S)-n-propyl 3-amino-3-(2-bromophenyl)propionate,
optically active (R or S)-n-propyl 3-amino-3-(3-bromophenyl)propionate,
optically active (R or S)-n-propyl 3-amino-3-(4-bromophenyl)propionate,
optically active (R or S)-n-propyl 3-amino-3-(2-fluorophenyl)propionate,
optically active (R or S)-n-propyl 3-amino-3-(3-fluorophenyl)propionate,
optically active (R or S)-n-propyl 3-amino-3-(4-fluorophenyl)propionate,
optically active (R or S)-n-propyl 3-amino-3-(2-methoxyphenyl)propionate,
optically active (R or S)-n-propyl 3-amino-3-(3-methoxyphenyl)propionate,
optically active (R or S)-n-propyl 3-amino-3-(4-methoxyphenyl)propionate and
optically active (R or S)-n-propyl 3-amino-3-(3,4-methylenedioxyphenyl)propionate.

Also, specific examples of the n-butyl ester of Compound (IV-a) obtained by the hydrolysis reaction of the present invention may be mentioned, for example,
optically active (R or S)-n-butyl 3-amino-3-(2-tolyl)propionate,
optically active (R or S)-n-butyl 3-amino-3-(3-tolyl)propionate,
optically active (R or S)-n-butyl 3-amino-3-(4-tolyl)propionate,
optically active (R or S)-n-butyl 3-amino-3-(2,3-xylyl)propionate,
optically active (R or S)-n-butyl 3-amino-3-(2-chlorophenyl)propionate,
optically active (R or S)-n-butyl 3-amino-3-(3-chlorophenyl)propionate,
optically active (R or S)-n-butyl 3-amino-3-(4-chlorophenyl)propionate,
optically active (R or S)-n-butyl 3-amino-3-(2,3-dichlorophenyl)propionate,
optically active (R or S)-n-butyl 3-amino-3-(2,4-dichlorophenyl)propionate,
optically active (R or S)-n-butyl 3-amino-3-(3,4-dichlorophenyl)propionate,
optically active (R or S)-n-butyl 3-amino-3-(3,5-dichlorophenyl)propionate,
optically active (R or S)-n-butyl 3-amino-3-(2-bromophenyl)propionate,
optically active (R or S)-n-butyl 3-amino-3-(3-bromophenyl)propionate,
optically active (R or S)-n-butyl 3-amino-3-(4-bromophenyl)propionate,
optically active (R or S)-n-butyl 3-amino-3-(2-fluorophenyl)propionate,
optically active (R or S)-n-butyl 3-amino-3-(3-fluorophenyl)propionate,
optically active (R or S)-n-butyl 3-amino-3-(4-fluorophenyl)propionate,
optically active (R or S)-n-butyl 3-amino-3-(3,4-difluorophenyl)propionate,
optically active (R or S)-n-butyl 3-amino-3-(2-iodophenyl)propionate,
optically active (R or S)-n-butyl 3-amino-3-(3-iodophenyl)propionate,
optically active (R or S)-n-butyl 3-amino-3-(4-iodophenyl)propionate,
optically active (R or S)-n-butyl 3-amino-3-(2-methoxyphenyl)propionate,
optically active (R or S)-n-butyl 3-amino-3-(3-methoxyphenyl)propionate,
optically active (R or S)-n-butyl 3-amino-3-(4-methoxyphenyl)propionate,
optically active (R or S)-n-butyl 3-amino-3-(2,3-dimethoxyphenyl)propionate,
optically active (R or S)-n-butyl 3-amino-3-(3,4-dimethoxyphenyl)propionate,
optically active (R or S)-n-butyl 3-amino-3-(3,5-dimethoxyphenyl)propionate and
optically active (R or S)-n-butyl 3-amino-3-(3,4-methylenedioxyphenyl)propionate,
and the like, preferably
optically active (R or S)-n-butyl 3-amino-3-(2-tolyl)propionate,
optically active (R or S)-n-butyl 3-amino-3-(3-tolyl)propionate,
optically active (R or S)-n-butyl 3-amino-3-(4-tolyl)propionate,
optically active (R or S)-n-butyl 3-amino-3-(2,3-xylyl)propionate,
optically active (R or S)-n-butyl 3-amino-3-(2-chlorophenyl)propionate,
optically active (R or S)-n-butyl 3-amino-3-(3-chlorophenyl)propionate,
optically active (R or S)-n-butyl 3-amino-3-(4-chlorophenyl)propionate,
optically active (R or S)-n-butyl 3-amino-3-(3,4-dichlorophenyl)propionate,
optically active (R or S)-n-butyl 3-amino-3-(2-bromophenyl)propionate,
optically active (R or S)-n-butyl 3-amino-3-(3-bromophenyl)propionate,
optically active (R or S)-n-butyl 3-amino-3-(4-bromophenyl)propionate,
optically active (R or S)-n-butyl 3-amino-3-(2-fluorophenyl)propionate,
optically active (R or S)-n-butyl 3-amino-3-(3-fluorophenyl)propionate,
optically active (R or S)-n-butyl 3-amino-3-(4-fluorophenyl)propionate,
optically active (R or S)-n-butyl 3-amino-3-(2-iodophenyl)propionate,
optically active (R or S)-n-butyl 3-amino-3-(3-iodophenyl)propionate,
optically active (R or S)-n-butyl 3-amino-3-(4-iodophenyl)propionate,
optically active (R or S)-n-butyl 3-amino-3-(2-methoxyphenyl)propionate,
optically active (R or S)-n-butyl 3-amino-3-(3-methoxyphenyl)propionate,
optically active (R or S)-n-butyl 3-amino-3-(4-methoxyphenyl)propionate,
optically active (R or S)-n-butyl 3-amino-3-(3,4-dimethoxyphenyl)propionate and
optically active (R or S)-n-butyl 3-amino-3-(3,4-methylenedioxyphenyl)propionate,
and the like, more preferably
optically active (R or S)-n-butyl 3-amino-3-(2-tolyl)propionate,
optically active (R or S)-n-butyl 3-amino-3-(3-tolyl)propionate,
optically active (R or S)-n-butyl 3-amino-3-(4-tolyl)propionate,
optically active (R or S)-n-butyl 3-amino-3-(2-chlorophenyl)propionate,
optically active (R or S)-n-butyl 3-amino-3-(3-chlorophenyl)propionate,
optically active (R or S)-n-butyl 3-amino-3-(4-chlorophenyl)propionate,
optically active (R or S)-n-butyl 3-amino-3-(3,4-dichlorophenyl)propionate,
optically active (R or S)-n-butyl 3-amino-3-(2-bromophenyl)propionate,
optically active (R or S)-n-butyl 3-amino-3-(3-bromophenyl)propionate,
optically active (R or S)-n-butyl 3-amino-3-(4-bromophenyl)propionate,
optically active (R or S)-n-butyl 3-amino-3-(2-fluorophenyl)propionate,
optically active (R or S)-n-butyl 3-amino-3-(3-fluorophenyl)propionate,
optically active (R or S)-n-butyl 3-amino-3-(4-fluorophenyl)propionate,
optically active (R or S)-n-butyl 3-amino-3-(4-iodophenyl)propionate,
optically active (R or S)-n-butyl 3-amino-3-(2-methoxyphenyl)propionate,
optically active (R or S)-n-butyl 3-amino-3-(3-methoxyphenyl)propionate,
optically active (R or S)-n-butyl 3-amino-3-(4-methoxyphenyl)propionate and
optically active (R or S)-n-butyl 3-amino-3-(3,4-methylenedioxyphenyl)propionate,
and the like, particularly preferably
optically active (R or S)-n-butyl 3-amino-3-(2-tolyl)propionate,
optically active (R or S)-n-butyl 3-amino-3-(3-tolyl)propionate,
optically active (R or S)-n-butyl 3-amino-3-(4-tolyl)propionate
optically active (R or S)-n-butyl 3-amino-3-(2-chlorophenyl)propionate,
optically active (R or S)-n-butyl 3-amino-3-(3-chlorophenyl)propionate,
optically active (R or S)-n-butyl 3-amino-3-(4-chlorophenyl)propionate,
optically active (R or S)-n-butyl 3-amino-3-(2-bromophenyl)propionate,
optically active (R or S)-n-butyl 3-amino-3-(3-bromophenyl)propionate,
optically active (R or S)-n-butyl 3-amino-3-(4-bromophenyl)propionate,
optically active (R or S)-n-butyl 3-amino-3-(2-fluorophenyl)propionate,
optically active (R or S)-n-butyl 3-amino-3-(3-fluorophenyl)propionate,
optically active (R or S)-n-butyl 3-amino-3-(4-fluorophenyl)propionate,
optically active (R or S)-n-butyl 3-amino-3-(2-methoxyphenyl)propionate,
optically active (R or S)-n-butyl 3-amino-3-(3-methoxyphenyl)propionate,
optically active (R or S)-n-butyl 3-amino-3-(4-methoxyphenyl)propionate and
optically active (R or S)-n-butyl 3-amino-3-(3,4-methylenedioxyphenyl)propionate.

### Example

Next, the present invention is explained more specifically by referring to Examples, but the scope of the present invention is not limited by these.

### Reference example 1 (Synthesis of 3-amino-3-phenylpropionic acid (racemic mixtures))

To 250 mL of isopropyl alcohol were added 17.7 g (0.17 mol) of benzaldehyde, 18.2 g (0.17 mol) of malonic acid and 25.6 g (0.33 mol) of ammonium acetate, and the mixture was reacted while stirring and under reflux (80 to 90°C) for 7 hours. After completion of the reaction, the obtained reaction mixture was stirred at 0 to 5°C for 1 hour and then filtered to give 19.2 g of 3-amino-3-phenylpropionic acid (racemic mixtures) (isolation yield based on benzaldehyde: 70.0%) as white powder.

Incidentally, physical properties of the 3-amino-3-phenylpropionic acid (racemic mixtures) were as follows.
¹H-NMR (δ (ppm), D₂O+DCl) : 3.06 (dd, 1H, J=17.1, 6.8Hz), 3.17 (dd, 1H, J=17.1, 7.3Hz), 4.76 (dd, 1H, J=7.3, 6.8Hz), 3.77 (s, 2H), 7.45 (m, 5H)
¹³C-NMR (δ (ppm), D₂O+DCl) : 40.5, 54.4, 130.0, 132.3, 132.6, 138.0, 176.3
MS (EI) m/z: 165 (M⁺)
MS (CI, i-C₄H₁₀) m/z: 166 (MH⁺)
Elemental analysis; Calcd: C, 65.44%; H, 6.71%; N, 8.48%
Found: C, 65.18%; H, 6.78%; N, 8.34%

### Reference example 2 (Synthesis of n-propyl 3-amino-3-phenylpropionate (racemic mixtures))

To 6.00 mL (120 mmol) of n-propyl alcohol were added 2.00 g (12.1 mmol) of 3-amino-3-phenylpropionic acid (racemic mixtures) synthesized in Reference example 1 and 1.78 g (18.2 mmol) of conc. sulfuric acid, and the mixture was reacted while stirring at 60°C for 4 hours. After completion of the reaction, the obtained reaction mixture was concentrated under reduced pressure, then, 6 mol/L aqueous sodium hydroxide solution was added thereto to adjust a pH of the reaction mixture to 8.5. Then, 10 mL of ethyl acetate and 4 mL of water were added to the mixture to carry out extraction, and the organic layer was dried over anhydrous magnesium sulfate. After filtration, the filtrate was concentrated under reduced pressure to give 2.16 g of n-propyl 3-amino-3-phenylpropionate (racemic mixtures) (isolation yield based on 3-amino-3-phenylpropionic acid (racemic mixtures): 86.1%) as colorless liquid.

Incidentally, physical properties of the n-propyl 3-amino-3-phenylpropionate (racemic mixtures) were as follows.
¹H-NMR (δ (ppm), CDCl₃) : 0.90 (d, 3H, J=7.3Hz), 1.55-1.65 (tq, 2H, J=7.3, 6.8Hz), 2.63 (d, 2H, J=6.8Hz), 4.01 (t, 2H, J=6.8Hz), 4.39 (d, 1H, J=6.8Hz), 7.20-7.35 (m, 5H)
¹³C-NMR (δ (ppm), CDCl₃) : 10. 4, 21. 9, 44.2, 52. 7, 66.1, 126.2, 127.3, 128.6, 144.7, 172.0
MS (EI) m/z: 207 (M⁺)
MS (CI, i-C₄H₁₀) m/z: 208 (MH⁺)
Elemental analysis; Calcd: C, 69.54%; H, 8.27%; N, 6.76%
Found: C, 68.86%; H, 8.22%; N, 6.60%

### Example 1 (Syntheses of (S)-3-amino-3-phenylpropionic acid and (R)-n-propyl 3-amino-3-phenylpropionate)

To a mixed solution comprising 4.75 mL of 50 mmol/L aqueous potassium phosphate solution with a pH of 8.2 and 0.25 mL of t-butyl methyl ether was added 1.00 g (4.82 mmol) of n-propyl 3-amino-3-phenylpropionate (racemic mixtures) synthesized in Reference example 2 and the mixture was maintained to 30°C. To the obtained mixture was added 50 mg of lipase (Amano Lipase PS (trade name); available from Aldrich Corporation) originated from *Burkholderia cepacia (Pseudomonas cepacia)* at the same temperature, and the mixture was reacted while stirring at 30°C. After 15 hours, 3 mL of acetone was added to the reaction mixture and the resulting mixture was filtered to give 359 mg of (S)-3-amino-3-phenylpropionic acid (isolation yield based on n-propyl 3-amino-3-phenylpropionate (racemic mixtures)=44.0%) and 37 mg of an enzyme fixing agent as a mixture.

The (S)-3-amino-3-phenylpropionic acid was led to an n-propyl ester according to the conventional manner, and when an optical purity thereof was measured by using high performance liquid chromatography which uses an optically active column, it was 99.9%ee.

When an optical purity of the (R)-n-propyl 3-amino-3-phenylpropionate was measured by using high performance liquid chromatography which uses an optically active column, it was 99.9%ee.

Incidentally, the E value in the present reaction was larger than 10000.
Analytical conditions of high performance liquid chromatography;
Optically active n-propyl 3-amino-3-phenylpropioriate Column: Chiral CD-Ph (0.46cmΦx25cm, available from Shiseido Co., Ltd.)
Solvent: acetonitrile/water (=1/9 (volume ratio))
Potassium dihydrogen phosphate 40mM
Adjusted to pH 3.5 with phosphoric acid
Flow rate: 1.0 mL /min
Temperatrure: 30°C
Wavelength: 220 nm

Also, physical properties of the (S)-3-amino-3-phenylpropionic acid were as follows.
¹H-NMR (δ (ppm), D₂O+DCl) : 3.06 (dd, 1H, J=17.1, 6.8Hz), 3.17 (dd, 1H, J=17.1, 7.3Hz), 4.76 (dd, 1H, J=7.3, 6.8Hz), 3.77 (s, 2H), 7.45 (m, 5H)
¹³C-NMR (δ (ppm), D₂O+DCl) : 40.5, 54.4, 130.0, 132.3, 132. 6, 138.0, 176.3
MS (EI) m/z: 165 (M⁺)
MS (CI, i-C₄H₁₀) m/z: 166 (MH⁺)
Elemental analysis; Calcd: C, 65.44%; H, 6.71%; N, 8.48%
Found: C, 65.18%; H, 6.78%; N, 8.34%.

Physical properties of (R)-n-propyl 3-amino-3-phenylpropionate were as follows.
¹H-NMR (δ (ppm), CDCl₃): 0.90 (d, 3H, J=7.3Hz), 1.55-1.65 (tq, 2H, J=7.3, 6.8Hz), 2.63 (d, 2H, J=6.8Hz), 4.01 (t, 2H, J=6.8Hz), 4.39 (d, 1H, J=6.8Hz), 7.20-7.35 (m, 5H)
¹³C-NMR (δ (ppm), CDCl₃): 10.4, 21.9, 44.2, 52.7, 66.1, 126.2, 127.3, 128.6, 144.7, 172.0
MS (EI) m/z: 207 (M⁺)
MS (CI, i-C₄H₁₀) m/z: 208 (MH⁺)
Elemental analysis; Calcd: C,69.54%; H,8.27%; N,6.76%
Found: C,68.86%; H,8.22%; N,6.60%

### Example 2 (Syntheses of (S)-3-amino-3-phenylpropionic acid and (R)-n-propyl 3-amino-3-phenylpropionate)

To a mixed solution comprising 4.75 mL of 50 mmol/L aqueous ammonium acetate solution with a pH of 8.3 and 0.25 mL of t-butyl methyl ether was added 1.00 g (4.82 mmol) of n-propyl 3-amino-3-phenylpropionate (racemic mixtures) synthesized in Reference example 2, and the mixture was maintained to 30°C. To the obtained mixture was added 50 mg of lipase (Amano Lipase PS (trade name); available from Aldrich Corporation) originated from *Burkholderia cepacia (Pseudomonas cepacia)* at the same temperature, and the mixture was reacted while stirring at 30°C. After 15 hours, 3 mL of acetone was added to the reaction mixture and the resulting mixture was filtered to give 359 mg of (S)-3-amino-3-phenylpropionic acid (isolation yield based on n-propyl 3-amino-3-phenylpropionate (racemic mixtures)= 44.0%) and 38 mg of an enzyme fixing agent as a mixture.

The (S)-3-amino-3-phenylpropionic acid was led to an n-propyl ester according to the conventional manner, and when an optical purity thereof was measured by using high performance liquid chromatography which uses an optically active column, it was 99.8%ee.

When an optical purity of the (R)-n-propyl 3-amino-3-phenylpropionate was measured by using high performance liquid chromatography which uses an optically active column, it was 79.4%ee.

Incidentally, the E value in the present reaction was 1945.
Analytical conditions of high performance liquid chromatography;
Optically active n-propyl 3-amino-3-phenylpropionate Column: Chiral CD-Ph (0.46cmΦ×25cm, available from Shiseido Co., Ltd.)
Solvent: acetonitrile/water (=1/9 (volume ratio))
Potassium dihydrogen phosphate 40mM
Adjusted to pH 3.5 with phosphoric acid
Flow rate: 1.0 mL/min
Temperatrure: 30°C
Wavelength: 220 nm

Also, physical properties of (S)-3-amino-3-phenylpropionic acid and (R)-n-propyl 3-amino-3-phenylpropionate were the same as those shown in Example 1.

### Example 3 (Syntheses of (S)-3-amino-3-phenylpropionic acid and (R)-n-propyl 3-amino-3-phenylpropionate)

To 4.75 mL of 50 mmol/L aqueous potassium phosphate solution with a pH of 8.2 was added 50 mg of lipase (Amano Lipase PS (trade name); available from Aldrich Corporation) originated from *Burkholderia cepacia (Pseudomonas cepacia)* and the mixture was stirred at room temperature for 30 minutes. The mixture was filtered, and to the resulting filtrate were added 0.25 mL of toluene and 1.00 g (4.82 mmol) of n-propyl 3-amino-3-phenylpropionate (racemic mixtures) synthesized in Reference example 2, and the mixture was reacted at 30°C under stirring. After 18 hours, 3 mL of acetone was added to the reaction mixture and the resulting mixture was filtered to give 359 mg of (S)-3-amino-3-phenylpropionic acid (isolation yield based on n-propyl 3-amino-3-phenylpropionate (racemic mixtures)= 44.0%) and 38 mg of an enzyme fixing agent as a mixture.

The (S)-3-amino-3-phenylpropionic acid was led to an n-propyl ester according to the conventional manner, and when an optical purity thereof was measured by using high performance liquid chromatography which uses an optically active column, it was 99.5%ee.

When an optical purity of the (R)-n-propyl 3-amino-3-phenylpropionate was measured by using high performance liquid chromatography which uses an optically active column, it was 99.9%ee.

Incidentally, the E value in the present reaction was 4038.
Analytical conditions of high performance liquid chromatography;
Optically active n-propyl 3-amino-3-phenylpropionate Column: Chiral CD-Ph (0.46cmΦx25cm, available from Shiseido Co., Ltd.)
Solvent: acetonitrile/water (=1/9 (volume ratio))
Potassium dihydrogen phosphate 40 mM
Adjusted to pH 3.5 with phosphoric acid
Flow rate: 1.0 mL/min
Temperatrure: 30°C
Wavelength: 220 nm

Also, physical properties of (S)-3-amino-3-phenylpropionic acid and (R)-n-propyl 3-amino-3-phenylpropionate were the same as those shown in Example 1.

### Example 4 (Syntheses of (S)-3-amino-3-phenylpropionic acid and (R)-n-propyl 3-amino-3-phenylpropionate)

To 1.25 mL of 50 mmol/L aqueous potassium phosphate solution with a pH of 8.2 was added 25 mg of lipase (Amano Lipase PS (trade name); available from Aldrich Corporation) originated from *Burkholderia cepacia (Pseudomonas cepacia)* and the mixture was stirred at room temperature for 30 minutes. The mixture was filtered, 1.25 mL of cyclohexane and 500 mg (2.41 mmol) of n-propyl 3-amino-3-phenylpropionate (racemic mixtures) synthesized in Reference example 2 were added to the resulting filtrate, and the mixture was reacted at 30°C under stirring. After 20 hours, 1.5 mL of acetone was added to the reaction mixture and the resulting mixture was stirred at 0°C for 1 hour. The reaction mixture was filtered to give 156 mg of (S)-3-amino-3-phenylpropionic acid (isolation yield based on n-propyl 3-amino-3-phenylpropionate (racemic mixtures)=43.0%). Then, after the filtrate was concentrated under reduced pressure, 2.5 mL of ethyl acetate was added to the residue to extract the organic layer, and the organic layer was dried over anhydrous magnesium sulfate. After filtration, the fitrate was concentrated under reduced pressure to give 230 mg of (R)-n-propyl 3-amino-3-phenylpropionate (isolation yield based on n-propyl 3-amino-3-phenylpropionate (racemic mixtures)=46.0%).

(S)-3-amino-3-phenylpropionic acid was led to an n-propyl ester according to the conventional manner, and when an optical purity thereof was measured by using high performance liquid chromatography which uses an optically active column, it was 99.7%ee.

When an optical purity of the (R)-n-propyl 3-amino-3-phenylpropionate was measured by using high performance liquid chromatography which uses an optically active column, it was 97.3%ee.

Incidentally, the E value in the present reaction was 3037.
Analytical conditions of high performance liquid chromatography;
Optically active n-propyl 3-amino-3-phenylpropionate Column: Chiral CD-Ph (0.46cmΦx25cm, available from Shiseido Co., Ltd.)
Solvent: acetonitrile/water (=1/9 (volume ratio))
Potassium dihydrogen phosphate 40mM
Adjusted to pH 3.5 with phosphoric acid
Flow rate: 1.0 mL/min
Temperatrure: 30°C
Wavelength: 220 nm

Also, physical properties of the (S)-3-amino-3-phenylpropionic acid and (R)-n-propyl 3-amino-3-phenylpropionate were the same as those shown in Example 1. Comparative example 1 (Syntheses of (S)-3-amino-3-phenylpropionic acid and (R)-n-propyl 3-amino-3-phenylpropionate)

To 1.5 mL of 50 mmol/L aqueous potassium phosphate solution with a pH of 8.2 was added 300 mg (1.45 mmol) of n-propyl 3-amino-3-phenylpropionate (racemic mixtures) synthesized in Reference example 2 and the mixture was maintained to 30°C. To the obtained mixture was added 15 mg of lipase (Amano Lipase PS (trade name); available from Aldrich Corporation) originated from *Burkholderia cepacia (Pseudomonas cepacia)* at the same temperature, and the mixture was reacted while stirring at 30°C. After 17 hours, 1.0 mL of acetone was added to the reaction mixture and the resulting mixture was filtered to give 108 mg of (S)-3-amino-3-phenylpropionic acid (isolation yield based on n-propyl 3-amino-3-phenylpropionate (racemic mixtures)= 45.0%) and 12 mg of an enzyme fixing agent as a mixture.

The (S)-3-amino-3-phenylpropionic acid was led to an n-propyl ester according to the conventional manner, and when an optical purity thereof was measured by using high performance liquid chromatography which uses an optically active column, it was 99.3%ee.

When an optical purity of the (R)-n-propyl 3-amino-3-phenylpropionate was measured by using high performance liquid chromatography which uses an optically active column, it was 99.9%ee.

Incidentally, the E value in the present reaction was 1687.
Analytical conditions of high performance liquid chromatography;
Optically active n-propyl 3-amino-3-phenylpropionate Column: Chiral CD-Ph (0.46cmΦx25cm, available from Shiseido Co., Ltd.)
Solvent: acetonitrile/water (=1/9 (volume ratio))
Potassium dihydrogen phosphate 40 mmol/L
Adjusted to pH 3.5 with phosphoric acid
Flow rate: 1.0 mL/min
Temperatrure: 30°C
Wavelength: 220 nm

Also, physical properties of the (S)-3-amino-3-phenylpropionic acid and (R)-n-propyl 3-amino-3-phenylpropionate were the same as those shown in Example 1.

Incidentally, determination of the absolute configuration of the optically active 3-amino-3-phenylpropionic acid was carried out as follows. A sign of specific rotation ([α]²⁵_{D} -7.96° (c 1.0, H₂O)) of the optically active 3-amino-3-phenylpropionic acid obtained in Comparative example 1 and a sigh of specific rotation of (S)-3-amino-3-phenylpropionic acid described in Tetrahedron Asymmetry, 6 (7), 1601 (1995) (literature value [α]²⁰_{D} -6.42°(c 1.0, H₂O)) are compared to each other to determine the absolute configuration.

### Comparative example 2 (Syntheses of (S)-3-amino-3-phenylpropionic acid and (R)-methyl 3-amino-3-phenylpropionate)

To 1.5 mL of 50 mmol/L aqueous potassium phosphate solution with a pH of 8.2 was added 300 mg (1.67 mmol) of methyl 3-amino-3-phenylpropionate (racemic mixtures) and the mixture was maintained to 30°C. To the obtained mixture was added 15 mg of lipase (Amano Lipase PS (trade name); available from Aldrich Corporation) originated from *Burkholderia cepacia (Pseudomonas cepacia)* at the same temperature, and the mixture was reacted while stirring at 30°C. After 15 hours, 0.5 mL of acetone was added to the reaction mixture and the resulting mixture was filtered to give 96.8 mg of (S)-3-amino-3-phenylpropionic acid (isolation yield based on methyl 3-amino-3-phenylpropionate (racemic mixtures)=35.0%) and 10 mg of an enzyme fixing agent as a mixture.

The (S)-3-amino-3-phenylpropionic acid was led to an n-propyl ester according to the conventional manner, and when an optical purity thereof was measured by using high performance liquid chromatography which uses an optically active column, it was 49.9%ee.

When an optical purity of the (R)-methyl 3-amino-3-phenylpropionate was measured by using high performance liquid chromatography which uses an optically active column, it was 46.8%ee.

Incidentally, the E value in the present reaction was 4.
Analytical conditions of high performance liquid chromatography;
Optically active methyl 3-amino-3-phenylpropionate
Column: Chiral CD-Ph (0.46cmΦx25cm, available from
Shiseido Co., Ltd.)
Solvent: acetonitrile/water (=1/9 (volume ratio))
Potassium dihydrogen phosphate 40 mmol/L
Adjusted to pH 3.5 with phosphoric acid
Flow rate: 0.5 mL/min
Temperatrure: 30°C
Wavelength: 220 nm

Also, physical properties of the (S)-3-amino-3-phenylpropionic acid were the same as those shown in Example 1.

### Comparative example 3 (Syntheses of (S)-3-amino-3-phenylpropionic acid and (R)-ethyl 3-amino-3-phenylpropionate)

To 1.5 mL of 50 mmol/L aqueous potassium phosphate solution with a pH of 8.2 was added 300 mg (1.55 mmol) of ethyl 3-amino-3-phenylpropionate (racemic mixtures) and the mixture was maintained to 30°C. To the obtained mixture was added 13 mg of lipase (Amano Lipase PS (trade name); available from Aldrich Corporation) originated from *Burkholderia cepacia (Pseudomonas cepacia)* at the same temperature, and the mixture was reacted while stirring at 30°C. After 13 hours, 0.5 mL of acetone was added to the reaction mixture and the resulting mixture was filtered to give 103 mg of (S)-3-amino-3-phenylpropionic acid (isolation yield based on ethyl 3-amino-3-phenylpropionate (racemic mixtures)=40.0%) and 10 mg of an enzyme fixing agent as a mixture.

The (S)-3-amino-3-phenylpropionic acid was led to an n-propyl ester according to the conventional manner, and when an optical purity thereof was measured by using high performance liquid chromatography which uses an optically active column, it was 98.8%ee.

When an optical purity of the (R)-ethyl 3-amino-3-phenylpropionate was measured by using high performance liquid chromatography which uses an optically active column, it was 95.3%ee.

Incidentally, the E value in the present reaction was 628.
Analytical conditions of high performance liquid chromatography;
Optically active ethyl 3-amino-3-phenylpropionate
Column: Chiral CD-Ph (0.46cmΦx25cm, available from Shiseido Co., Ltd.)
Solvent: acetonitrile/water (=1/9 (volume ratio))
Potassium dihydrogen phosphate 40 mmol/L
Adjusted to pH 3.5 with phosphoric acid
Flow rate: 0.5 mL/min
Temperatrure: 30°C
Wavelength: 220 nm

Also, physical properties of the (S)-3-amino-3-phenylpropionic acid were the same as those shown in Example 1.

Physical properties of the (R)-ethyl 3-amino-3-phenylpropionate were as follows.
¹H-NMR (δ (ppm), CDCl₃): 1.19 (t, 3H, J=7.3Hz), 3.15 (dd, 1H, J=7.3, 16.6Hz), 3.25 (dd, 1H, J=7.3, 16.6Hz), 4.15 (q, 2H, J=7.3Hz), 4.85 (dd, 1H, J=7.3, 7.3Hz), 7.50-7.55 (m, 5H)
¹³C-NMR (δ (ppm), CDCl₃) : 16.0, 40.9, 54.3, 65.2, 129.9, 132.2, 132.5, 137.8, 174.3

### Comparative example 4 (Syntheses of (S)-3-amino-3-phenylpropionic acid and (R)-isopropyl 3-amino-3-phenylpropionate)

To 1.5 mL of 50 mmol/L aqueous potassium phosphate solution with a pH of 8.2 was added 300 mg (1.45 mmol) of isopropyl 3-amino-3-phenylpropionate (racemic mixtures) and the mixture was maintained to 30°C. To the obtained mixture was added 15 mg of lipase (Amano Lipase PS (trade name); available from Aldrich Corporation) originated from *Burkholderia cepacia* (*Pseudomonas cepacia*) at the same temperature, and the mixture was reacted while stirring at 30°C for 3 hours. However, the reaction dis not substantially proceed, and the objective product could not be obtained.
Analytical conditions of high performance liquid chromatography;
Isopropyl 3-amino-3-phenylpropionate (racemic mixtures) and 3-amino-3-phenylpropionic acid (racemic mixtures)
Column: Cadenza CD-C18 (4.6cmΦx150mm, available from
DAICEL CHEMICAL INDUSTRIES, LTD.)
Solvent: acetonitrile/water (=2/3 (volume ratio))
Potassium dihydrogen phosphate: 10 mmol/L
pH 6.0
Wavelength: 254 nm
Flow rate: 0.6 mL/min
Temperatrure: 40°C

### Reference example 3 (Syntheses of t-butyl 3-amino-3-phenylpropionate (racemic mixtures))

To 25.0 mL (0.27 mol) of n-butyl alcohol were added 5.00 g (30.3 mmol) of 3-amino-3-phenylpropionic acid (racemic mixtures) synthesized in Reference example 1 and 6.00 g (61.2 mmol) of conc. sulfuric acid, and the mixture was reacted while stirring at 70 to 80°C for 4 hours. After completion of the reaction, 10 mL of water and 6 mol/L aqueous sodium hydroxide solution were added to the obtained reaction mixture to adjust a pH of the reaction mixture to 7 to 8. Then, 1 g of sodium chloride was added to the mixture, the liquids were separated, and then, the organic layer was dried over anhydrous magnesium sulfate. After filtration, the filtrate was concentrated under reduced pressure to give 6.90 g of t-butyl 3-amino-3-phenylpropionate (racemic mixtures) (isolation yield based on 3-amino-3-phenylpropionic acid (racemic mixtures): 88.1%) as colorless liquid.

Incidentally, physical properties of the n-butyl 3-amino-3-phenylpropionate (racemic mixtures) were as follows.
¹H-NMR (δ (ppm), CDCl₃) : 0.90 (t, 3H, J=7.3Hz), 1.32 (tq, 2H, J=7.6, 7.3Hz), 1.56 (dt, 2H, J=7.6, 6.8Hz), 1.91 (s, 2H)2.64 (d, 2H, J=6.8Hz), 4.06 (t, 2H, J=6.8Hz), 4.39 (t, 1H, J=6.8Hz), 7.20-7.35 (m, 5H)
¹³C-NMR (δ (ppm), CDCl₃): 13.7, 19.1, 30.644.1, 52.7, 64.4, 126.2, 127.4, 128.6, 144.6, 172.0
MS (EI) m/z: 221 (M⁺)
MS (CI, i-C₄H₁₀) m/z: 222 (MH⁺)

### Example 5 (Syntheses of (S)-3-amino-3-phenylpropionic acid and (R)-n-butyl 3-amino-3-phenylpropionate)

To a mixture comprising 500 mg (2.26 mmol) of n-butyl 3-amino-3-phenylpropionate (racemic mixtures) synthesized in Reference example 3 and 1.25 mL of cyclohexane was added 1.25 mL of 50 mmol/L aqueous potassium phosphate solution with a pH of 8.2 and the mixture was maintained to 30°C. To the obtained mixture was added 25 mg of lipase (Amano Lipase PS (trade name); available from Aldrich Corporation) originated from *Burkholderia cepacia (Pseudomonas cepacia)* at the same temperature, and the mixture was reacted while stirring at 30°C. After 59 hours, a reaction conversion reached 45.4%.

The n-Butyl (R)-3-amino-3-phenylpropionate was led to (R)-n-butyl 3-(2-furoylamino)-3-phenylpropionate, and when an optical purity thereof was measured by using high performance liquid chromatography which uses an optically active column, it was 82.2%ee.

Incidentally, the E value in the present reaction was 513.
Optically active n-butyl 3-(2-furoylamino)-3-phenylpropionate Column: Chiralcel OJ-H (0.46cmΦx25cm, available from
DAICEL CHEMICAL INDUSTRIES, LTD.)
Solvent: hexane/isopropyl alcohol (=1/9 (volume ratio)) Flow rate: 0.5 mL/min
Temperatrure: 30°C
Wavelength: 220 nm

Also, physical properties of the (S)-3-amino-3-phenylpropionic acid were the same as those shown in Example 1.

Physical properties of the (R)-n-butyl 3-amino-3-phenylpropionate were as follows.
¹H-NMR (δ (ppm), CDCl₃) : 0.90 (t, 3H, J=7.3Hz), 1.32 (tq, 2H, J=7.6, 7.3Hz), 1.56 (dt, 2H, J=7.6, 6.8Hz), 1.91 (s, 2H)2.64 (d, 2H, J=6.8Hz), 4.06 (t, 2H, J=6.8Hz), 4.39 (t, 1H, J=6.8Hz), 7.20-7.35 (m, 5H)
¹³C-NMR (δ (ppm), CDCl₃) : 13.7, 19.1, 30.644.1, 52.7, 64.4, 126.2, 127.4, 128.6, 144.6, 172.0
MS (EI) m/z: 221 (M⁺)
MS (CI, i-C₄H₁₀) m/z: 222 (MH⁺)

### Example 6 (Synthesis of 3-amino-3-(4-tolyl)propionic acid (racemic mixtures))

To 250 mL of ethanol were added 50.0 g (0.42 mol) of 4-tolylaldehyde, 47.6 g (0.46 mol) of malonic acid and 64.2 g (0.83 mol) of ammonium acetate, and the mixture was reacted while stirring under reflux (80 to 90°C) for 7.5 hours. The obtained reaction mixture was stirred at 0 to 5°C for 30 minutes and then filtered to give 51.4 g of 3-amino-3-(4-tolyl)propionic acid (racemic mixtures) (isolation yield based on 4-tolylaldehyde: 68.9%) as white powder.

Incidentally, physical properties of the 3-amino-3-(4-tolyl)propionic acid (racemic mixtures) were as follows. ¹H-NMR (δ (ppm), D₂O+DCl) : 2.30 (s, 3H), 3.04 (dd, 1H, J=17.1, 6.8Hz), 3.20 (dd, 1H, J=17.1, 7.3Hz), 4.74 (dd, 1H, J=7.3, 6.8Hz), 7.29 (d, 2H, 8.3Hz), 7.36 (d, 2H, 8.3Hz)
¹³C-NMR (δ (ppm), D₂O+DCl): 23.4, 40.7, 54.4, 130.0, 133.0, 135.0, 143.1, 176.3
MS (EI) m/z: 179 (M⁺)
MS (CI, i-C₄H₁₀) m/z: 180 (MH⁺)
Elemental analysis; Calcd: C,67.02%; H,7.31%; N,7.82%
Found: C,67.05%; H,7.40%; N,7.66%
Example 7 (Syntheses of n-propyl 3-amino-3-(4-tolyl)propionate (racemic mixtures))

To 50.0 mL (1.03 mol) of n-propyl alcohol were added 10.0 g (55.8 mmol) of 3-amino-3-(4-tolyl)propionic acid (racemic mixtures) synthesized in Example 6 and 8.20 g (83.6 mmol) of conc. sulfuric acid, and the mixture was reacted while stirring at 60°C for 4 hours. After completion of the reaction, the obtained reaction mixture was concentrated under reduced pressure, and then 28% aqueous ammonia was added thereto to adjust a pH of the reaction mixture to 8.5. Then, 50 mL of ethyl acetate and 20 mL of water were added to the mixture to carry out extraction, and the organic layer was dried over anhydrous magnesium sulfate. After filtration, the filtrate was concentrated under reduced pressure to give 10.9 g of n-propyl 3-amino-3-(4-tolyl)propionate (racemic mixtures) (isolation yield based on 3-amino-3-(4-tolyl)propionic acid (racemic mixtures): 88.0%) as colorless liquid.

Incidentally, physical properties of the n-propyl 3-amino-3-(4-tolyl)propionate (racemic mixtures) were as follows.
¹H-NMR (δ (ppm), CDCl₃) : 0.90 (t, 3H, J=7.3Hz), 1.62 (tq, 2H, J=7.3, 6.8Hz), 2.30 (s, 3H), 2.62 (d, 2H, J=6.8Hz), 4.02 (t, 2H, J=6.8Hz), 4.36 (d, 1H, J=6.8Hz), 7.11 (d, 2H, 8.3Hz), 7.23 (d, 2H, 8.3Hz)
¹³C-NMR (δ (ppm), CDCl₃): 10.4, 21.0, 22.0, 44.2, 52.4, 66.0, 126.1, 129.2, 136.8, 141.9, 172.1
MS (EI) m/z: 221 (M⁺)
MS (CI, i-C₄H₁₀) m/z: 222 (MH⁺)

### Comparative example 5 (Syntheses of (S or R)-3-amino-3-(4-tolyl)propionic acid and (R or S)-n-propyl 3-amino-3-(4-tolyl)propionate)

To 2.5 mL of 50 mmol/L aqueous potassium phosphate solution with a pH of 8.2 was added 500 mg (2.26 mmol) of 3-amino-3-(4-tolyl)propionic acid n-propyl ester (racemic mixtures) synthesized in Example 7 and the mixture was maintained to 30°C. To the obtained mixture was added 25 mg of lipase (Amano Lipase PS (trade name); available from Aldrich Corporation) originated from *Burkholderia cepacia (Pseudomonas cepacia)* at the same temperature, and the mixture was reacted while stirring at 30°C. After 16 hours, 1 mL of acetone was added to the reaction mixture and the resulting mixture was filtered to give 364 mg of (S or R)-3-amino-3-(4-tolyl)propionic acid (isolation yield based on n-propyl 3-amino-3-(4-tolyl)propionate (racemic mixtures)=45.0%) and 20 mg of an enzyme fixing agent as a mixture.

The (S or R)-3-amino-3-(4-tolyl)propionic acid was led to an n-propyl ester according to the conventional manner, and when an optical purity thereof was measured by using high performance liquid chromatography which uses an optically active column, it was 98.7%ee.

When an optical purity of the (R or S)-n-propyl 3-amino-3-(4-tolyl)propionate was measured by using high performance liquid chromatography which uses an optically active column, it was 98.6%ee.

Incidentally, the E value in the present reaction was 772.
Analytical conditions of high performance liquid chromatography;
Optically active n-propyl 3-amino-3-(4-tolyl)propionate Column: Chiral CD-Ph (0.46cmΦ×25cm, available from Shiseido Co., Ltd.)
Solvent: acetonitrile/water (=1/9 (volume ratio))
Potassium dihydrogen phosphate 40 mmol/L
Adjusted to pH 3.5 with phosphoric acid
Flow rate: 1.0 mL/min
Temperatrure: 30°C
Wavelength: 220 nm

Also, physical properties of the (S or R)-3-amino-3-(4-tolyl)propionic acid were as follows.
¹H-NMR (δ (ppm), D₂O+DCl): 2.30 (s, 3H), 3.04 (dd, 1H, J=17.1, 6.8Hz), 3.20 (dd, 1H, J=17.1, 7.3Hz), 4.74 (dd, 1H, J=7.3, 6.8Hz), 7.29 (d, 2H, 8.3Hz), 7.36 (d, 2H, 8.3Hz)
¹³C-NMR (δ (ppm), D₂O+DCl) : 23.4, 40.7, 54.4, 130.0, 133.0, 135.0, 143.1, 176.3
MS (EI) m/z: 179 (M⁺)
MS (CI, i-C₄H₁₀) m/z: 180 (MH⁺)
Elemental analysis; Calcd: C,67.02%; H,7.31%; N,7.82%
Found: C,67.05%; H,7.40%; N,7.66%
Specific rotation: [α]²⁵_{D} +2.93° (c 1.0, 2N HCl)

Physical properties of the (R or S)-n-propyl 3-amino-3-(4-tolyl)propionate were as follows.
¹H-NMR (δ (ppm), CDCl₃) : 0.90 (t, 3H, J=7.3Hz), 1.62 (tq, 2H, J=7.3, 6.8Hz), 2.30 (s, 3H), 2.62 (d, 2H, J=6.8Hz), 4.02 (t, 2H, J=6.8Hz), 4.36 (d, 1H, J=6.8Hz), 7.11 (d, 2H, 8.3Hz), 7.23 (d, 2H, 8.3Hz)
¹³C-NMR (δ (ppm), CDCl₃): 10.4, 21.0, 22.0, 44.2, 52.4, 66.0, 126.1, 129.2, 136.8, 141.9, 172.1
MS (EI) m/z: 221 (M⁺)
MS (CI, i-C₄H₁₀) m/z: 222 (MH⁺)

### Example 8 (Syntheses of (S or R)-3-amino-3-(4-tolyl)propionic acid and (R or S)-n-propyl 3-amino-3-(4-tolyl)propionate)

To 4.75 mL of 10 mmol/L aqueous potassium phosphate solution with a pH of 8.2 was added 50 mg of lipase (Amano Lipase PS (trade name); available from Aldrich Corporation) originated from *Burkholderia cepacia (Pseudomonas cepacia)* and the mixture was stirred at room temperature for 30 minutes. The mixture was filtered, and to the obtained filtrate were added 0.25 mL of t-butyl methyl ether and 1.00 g (4.52 mmol) of 3-amino-3-(4-tolyl)propionic acid n-propyl ester (racemic mixtures) synthesized in Example 7, and the mixture was reacted while stirring at 30°C. After 16 hours, 3 mL of acetone was added to the reaction mixture and the resulting mixture was filtered to give 348 mg of (S or R)-3-amino-3-(4-tolyl)propionic acid (yield based on n-propyl 3-amino-3-(4-tolyl)propionate (racemic mixtures)= 43.0%). Then, after the filtrate was concentrated under reduced pressure, 10 mL of ethyl acetate, 5 mL of water and 6 mol/L aqueous sodium hydroxide solution were added to the concentrate to adjust a pH to 8.0. The organic layer was collected by separation and dried over anhydrous magnesium sulfate. After filtration, the filtrate was concentrated under reduced pressure to give 450 mg of (R or S)-n-propyl 3-amino-3-phenylpropionate (isolation yield based on n-propyl 3-amino-3-phenylpropionate (racemic mixtures)= 45.0%).

The (S or R)-3-amino-3-(4-tolyl)propionic acid was led to an n-propyl ester according to the conventional manner, and when an optical purity thereof was measured by using high performance liquid chromatography which uses an optically active column, it was 97.1%ee.

When an optical purity of the (R or S)-n-propyl 3-amino-3-(4-tolyl)propionate was measured by using high performance liquid chromatography which uses an optically active column, it was 99.9%ee.

Incidentally, the E value in the present reaction was 663.
Analytical conditions of high performance liquid chromatography;
Optically active n-propyl 3-amino-3-(4-tolyl)propionate Column: Chiral CD-Ph (0.46cmΦ×25cm, available from Shiseido Co., Ltd.)
Solvent: acetonitrile/water (=1/9 (volume ratio))
Potassium dihydrogen phosphate 40 mmol/L
Adjusted to pH 3.5 with phosphoric acid
Flow rate: 1.0 mL/min
Temperatrure: 30°C
Wavelength: 220 nm

Also, physical properties of the (S or R)-3-amino-3-(4-tolyl)propionic acid and (R or S)-n-propyl 3-amino-3-(4-tolyl)propionate were the same as those shown in Comparative example 5.

### Example 9 (Syntheses of (S or R)-3-amino-3-(4-tolyl)propionic acid and (R or S)-n-propyl 3-amino-3-(4-tolyl)propionate)

To 10 mL of 10 mmol/L aqueous potassium phosphate solution with a pH of 8.2 was added 200 mg of lipase (Amano Lipase PS (trade name); available from Aldrich Corporation) originated from *Burkholderia cepacia* (*Pseudomonas cepacia*) and the mixture was stirred at room temperature for 30 minutes. The mixture was filtered, to the obtained filtrate were added 10 mL of t-butyl methyl ether and 4.00 g (18.1 mmol) of n-propyl 3-amino-3-(4-tolyl)propionate (racemic mixtures) synthesized in Example 7, and the mixture was reacted while stirring at 30°C. After 16 hours, 1 mL of ethanol was added to the reaction mixture and the resulting mixture was filtered to give 1.49 g of (S or R)-3-amino-3-(4-tolyl)propionic acid (yield based on n-propyl 3-amino-3-(4-tolyl)propionate (racemic mixtures)= 46.0%). Then, after the filtrate was concentrated under reduced pressure, 10 mL of ethyl acetate, 5 mL of water and 6 mol/L aqueous sodium hydroxide solution were added to the concentrate to adjust a pH to 8.5. The organic layer was collected by separation and dried over anhydrous magnesium sulfate. After filtration, the filtrate was concentrated to give 2 g of (R or S)-n-propyl 3-amino-3-phenylpropionate (isolation yield based on n-propyl 3-amino-3-phenylpropionate (racemic mixtures)=45.0%).

The (S or R)-3-amino-3-(4-tolyl)propionic acid was led to an n-propyl ester according to the conventional manner, and when an optical purity thereof was measured by using high performance liquid chromatography which uses an optically active column, it was 99.7%ee.

When an optical purity of the (R or S)-n-propyl 3-amino-3-(4-tolyl)propionate was measured by using high performance liquid chromatography which uses an optically active column, it was 96.8%ee.

Incidentally, the E value in the present reaction was 3321.
Analytical conditions of high performance liquid chromatography;
Optically active 3-amino-3-(4-tolyl)propionic acid n-propyl ester
Column: Chiral CD-Ph (0.46cmΦ×25cm, available from Shiseido Co., Ltd.)
Solvent: acetonitrile/water (=1/9 (volume ratio))
Potassium dihydrogen phosphate 40 mmol/L
Adjusted to pH 3.5 with phosphoric acid
Flow rate: 1.0 mL/min
Temperatrure: 30°C
Wavelength: 220 nm

Also, physical properties of the (S or R)-3-amino-3-(4-tolyl)propionic acid and (R or S)-n-propyl 3-amino-3-(4-tolyl)propionate were the same as those shown in Comparative example 5.

### Comparative example 6 (Syntheses of (S)-3-amino-3-(4-tolyl)propionic acid and (R)-ethyl 3-amino-3-(4-tolyl)-propionate)

To 2.5 mL of 50 mmol/L aqueous potassium phosphate solution with a pH of 8.2 was added 500 mg (2.41 mol) of ethyl 3-amino-3-(4-tolyl)propionate (racemic mixtures) and the mixture was maintained to 30°C, 25 mg of lipase (Amano Lipase PS (trade name); available from Aldrich Corporation) originated from *Burkholderia cepacia* (*Pseudomonas cepacia*) was added to the mixture at the same temperature, and the mixture was reacted while stirring at 30°C. After 16 hours, 1.0 mL of acetone was added to the reaction mixture and the resulting mixture was filtered to give 173 mg of (S)-3-amino-3-(4-tolyl)propionic acid (isolation yield based on ethyl 3-amino-3-(4-tolyl)propionate (racemic mixtures)=40.0%) and 20 mg of an enzyme fixing agent as a mixture.

The (S)-3-amino-3-(4-tolyl)propionic acid was led to an ethyl ester according to the conventional manner, and when an optical purity thereof was measured by using high performance liquid chromatography which uses an optically active column, it was 88.4%ee.

When an optical purity of the (R)-ethyl 3-amino-3-(4-tolyl)propionate was measured by using high performance liquid chromatography which uses an optically active column, it was 47.2%ee.

Incidentally, the E value in the present reaction was 26.
Analytical conditions of high performance liquid chromatography;
Optically active ethyl 3-amino-3-(4-tolyl)propionate Column: Chiral CD-Ph (0.46cmΦx25cm, available from Shiseido Co., Ltd.)
Solvent: acetonitrile/water (=1/9 (volume ratio))
Potassium dihydrogen phosphate 40 mmol/L
Adjusted to pH 3.5 with phosphoric acid
Flow rate: 0.5 mL/min
Temperatrure: 30°C
Wavelength: 220 nm

Also, physical properties of the (S)-3-amino-3-(4-tolyl)propionic acid were the same as those shown in Comparative example 5.

Physical properties of the (R)-ethyl 3-amino-3-(4-tolyl)propionate were as follows.
¹H-NMR (δ (ppm), CDCl₃) : 1.20 (t, 3H, J=7.3Hz), 2.36 (s, 3H), 3.11 (dd, 1H, J=7.8, 16.6Hz), 3.26 (dd, 1H, J=6.3, 16.6Hz), 4.11-4.18 (m, 2H), 4.82 (dd, 1H, J=6.3, 7.8Hz), 7.35 (d, 2H, J=7.8Hz), 7.42 (d, 2H, J=8.3Hz)
¹³C-NMR (δ (ppm), CDCl₃) : 16.1, 23.1, 41.1, 54.2, 65.2, 129.9, 132.8, 134.9, 142.9, 174.3

### Example 10 (Synthesis of n-butyl 3-amino-3-(4-tolyl)propionate (racemic mixtures))

To 60.0 mL (652 mmol) of n-butyl alcohol were added 6.00 g (25.0 mmol) of 3-amino-3-(4-tolyl)propionic acid (racemic mixtures) synthesized in Example 6 and 4.90 g (50.2 mmol) of conc. sulfuric acid, and the mixture was reacted while stirring at 55 to 60°C for 3 hours. After completion of the reaction, 12 mL of water was added to the reaction mixture, a pH of the mixture was adjusted to 7.0 with 6 mol/L sodium hydroxide and the mixture was concentrated under reduced pressure. To the obtained concentrated residue was added 30 mL of cyclohexane, and a pH of the mixture was adjusted to 9.5 with 6 mol/L aqueous sodium hydroxide solution. The obtained mixture was allowed to stand, liquids were separated and the extracted organic phase was dried over anhydrous magnesium sulfate. After filtration, the filtrate was concentrated under reduced pressure to give 6.70 g of t-butyl 3-amino-3-(4-tolyl)-propionate (racemic mixtures) (isolation yield based on 3-amino-3-(4-tolyl)propionic acid (racemic mixtures): 85%) as colorless liquid.

Incidentally, physical properties of the n-butyl 3-amino-3-(4-tolyl)propionate (racemic mixtures) were as follows.
¹H-NMR (δ (ppm), CDCl₃) : 0.91 (t, 3H, J=7.3Hz), 1.22-1.42 (m, 2H), 1.51-1.62 (m, 2H), 2.33 (s, 5H), 2.68 (d, 6.8Hz), 4.07 (t, 1H, 6.8Hz), 4.40 (t, 2H, 6.8Hz), 7.13-7.27 (m, 4H) ¹³C-NMR (δ (ppm), CDCl₃): 13.69, 19.12, 21.05, 30.64, 43.82, 52.36, 64.45, 126.20, 129.31, 137.13, 144.54, 172.01
MS (EI) m/z: 235 (M⁺)
MS (CI, i-C₄H₁₀) m/z: 236 (MH⁺)

### Example 11 (Syntheses of (S or R)-3-amino-3-(4-tolyl)propionic acid and (R or S)-n-butyl 3-amino-3-(4-tolyl)-propionate)

To a mixture comprising 500 mg (2.22 mmol) of 3-amino-3-(4-tolyl)propionic acid n-butyl ester (racemic mixtures) synthesized in Example 10 and 1.25 mL of cyclohexane was added 1.25 mL of 50 mmol/L aqueous potassium phosphate solution with a pH of 8.2 and the mixture was maintained to 30°C. To the obtained mixture was added 25 mg of lipase (Amano Lipase PS (trade name); available from Aldrich Corporation) originated from *Burkholderia cepacia* (*Pseudomonas cepacia*) at the same temperature, and the mixture was reacted while stirring at 30°C. After 40 hours, a reaction conversion reached 50.6%.

When an optical purity of the (S or R)-3-amino-3-(4-tolyl)propionic acid was measured by using high performance liquid chromatography which uses an optically active column, it was 97.3%ee.

The (R or S)-n-butyl 3-amino-3-(4-tolyl)propionate was led to (R or S)-n-butyl 3-(2-furoylamino)-3-(4-tolyl)-propionate, and when an optical purity thereof was measured by using high performance liquid chromatography which uses an optically active column, it was 99.4%ee.

Incidentally, the E value in the present reaction was 421.
Analytical conditions of high performance liquid chromatography;
Optically active 3-amino-3-(4-tolyl)propionic acid
Column: Chiral CD-Ph (0.46cmΦ×25cmx2 columns connected, available from Shiseido Co., Ltd.)
Solvent: acetonitrile/water (=5/95 (volume ratio))
Potassium dihydrogen phosphate 40 mmol/L
Adjusted to pH 3.5 with phosphoric acid
Flow rate: 0.5 mL/min
Temperatrure: 30°C
Wavelength: 220 nm
Optically active n-butyl 3-(2-furoylamino)-3-(4-tolyl)-propionate
Column: Chiralcel OJ-H (0.46cmΦ×25cm, available from
DAICEL CHEMICAL INDUSTRIES, LTD.)
Solvent: hexane/isopropyl alcohol (=1/9 (volume ratio)) Flow rate: 0.5 mL/min
Temperatrure: 30°C
Wavelength: 220 nm

Also, physical properties of the (S or R)-3-amino-3-(4-tolyl)propionic acid were the same as those shown in Comparative example 5.

Physical properties of the (R or S)-n-butyl 3-amino-3-(4-tolyl)propionate (racemic mixtures) were as follows. ¹H-NMR (δ (ppm), CDCl₃): 0.91 (t, 3H, J=7.3Hz), 1.22-1.42 (m, 2H), 1.51-1.62 (m, 2H), 2.33 (s, 5H), 2.68 (d, 6.8Hz), 4.07 (t, 1H, 6.8Hz), 4.40 (t, 2H, 6.8Hz), 7.13-7.27 (m, 4H) ¹³C-NMR (δ (ppm), CDCl₃) : 13. 69, 19.12, 21.05, 30. 64, 43.82, 52.36, 64.45, 126.20, 129.31, 137.13, 144.54, 172.01
MS (EI) m/z: 235 (M⁺)
MS (CI, i-C₄H₁₀) m/z: 236 (MH⁺)

### Example 12 (Synthesis of 3-amino-3-(4-chlorophenyl)propionic acid (racemic mixtures))

To 25 mL of ethanol were added 5.00 g (35.6 mmol) of 4-chlorobenzaldehyde, 3.70 g (35.6 mmol) of malonic acid and 4.10 g (53.2 mmol) of ammonium acetate, and the mixture was reacted while stirring under reflux (75 to 80°C) for 8 hours. After completion of the reaction, the obtained reaction mixture was stirred at room temperature for 1 hour and then filtered to give 5.9 g of 3-amino-3-(4-chlorophenyl)propionic acid (racemic mixtures) (isolation yield based on 4-chlorobenzaldehyde: 82.3%) as white powder.

Incidentally, physical properties of the 3-amino-3-(4-chlorophenyl)propionic acid (racemic mixtures) were as follows.
¹H-NMR (δ (ppm), D₂O) : 2.93 (dd, 1H, J=17.1, 6.8Hz), 3.04 (dd, 1H, J=17.1, 7.8Hz), 4.63 (dd, 1H, J=7.8, 6.8Hz), 7.22 (s, 1H), 7.24 (s, 1H), 7.47 (s, 1H), 7.49 (s, 1H)
¹³C-NMR (δ (ppm), D₂O) : 40.4, 53.9, 126.0, 131.9, 135.3, 137.1, 175.9
MS (EI) m/z: 199 (M⁺)
MS (CI, i-C₄H₁₀) m/z: 200 (MH⁺)

### Example 13 (Synthesis of n-propyl 3-amino-3-(4-chlorophenyl)propionate (racemic mixtures))

To 20.0 mL (258 mmol) of n-propyl alcohol were added 5.00 g (25.0 mmol) of 3-amino-3-(4-chlorophenyl)propionic acid (racemic mixtures) synthesized in Example 12 and 3.90 g (39.8 mmol) of conc. sulfuric acid, and the mixture was reacted while stirring at 55 to 60°C for 2.5 hours. At the same temperature, the solvent was removed by distillation from the reaction system with a predetermined amount, and the same amount of n-propyl alcohol to that of the removed solvent was added to the mixture and the reaction was continued. After completion of the reaction, 10 mL of water was added to the reaction mixture, a pH of the mixture was adjusted to 7.5 with 6 mol/L sodium hydroxide and the mixture was concentrated under reduced pressure. To the obtained concentrated residue was added 25 mL of t-butyl methyl ether, a pH of the mixture was adjusted to 10.1 by using 6 mol/L aqueous sodium hydroxide solution. The obtained mixture was allowed to stand, liquids were separated and the extracted organic phase was dried over anhydrous magnesium sulfate. After filtration, the filtrate was concentrated under reduced pressure to give 4.89 g of n-propyl 3-amino-3-(4-chlorophenyl)propionate (racemic mixtures) (isolation yield based on 3-amino-3-(4-chlorophenyl)propionic acid (racemic mixtures): 80.8%) as colorless liquid.

Incidentally, physical properties of the n-propyl 3-amino-3-(4-chlorophenyl)propionate (racemic mixtures) were as follows.
¹H-NMR (δ (ppm), CDCl₃) : 0.91 (t, 3H, J=7.8Hz), 1.62 (qt, 2H, 7.8, 6.8Hz), 1.72 (br, 2H), 2.63 (d, 2H, J=6.8Hz), 4.04 (t, 2H, 6.8Hz), 4.41 (t, 2H, 6.8Hz), 7.31 (s, 4H)
¹³C-NMR (δ (ppm), CDCl₃) : 10. 4, 21.9, 44.1, 52.1, 66. 3, 127.7, 128.7, 133.1, 143.2, 171.8
MS (EI) m/z: 241 (M⁺)
MS (CI, i-C₄H₁₀) m/z: 242 (MH⁺)

### Example 14 (Syntheses of (S or R)-3-amino-3-(4-chlorophenyl)propionic acid and (R or S)-n-propyl 3-amino-3-(4-chlorophenyl)propionate)

To 0.75 mL of 10 mmol/L aqueous potassium phosphate solution with a pH of 8.2 was added 15 mg of lipase (Amano Lipase PS (trade name); available from Aldrich Corporation) originated from *Burkholderia cepacia (Pseudomonas cepacia)* at the same temperature and the mixture was stirred at room temperature for 30 minutes. The mixture was filtered, to the obtained filtrate were added 0.75 mL of t-butyl methyl ether and 300 mg (1.2 mmol) of n-propyl 3-amino-3-(4-chlorophenyl)propionate (racemic mixtures) synthesized in Example 13, and the mixture was reacted while stirring at 30°C.

After 5 hours, 1.0 mL of acetone was added to the reaction mixture and the resulting mixture was filtered to give 112 mg of (S or R)-3-amino-3-(4-chlorophenyl)propionic acid (yield based on n-propyl 3-amino-3-(4-chlorophenyl)-propionate (racemic mixtures)=45.0%). Then, the filtrate was separated, the organic phase was extracted and dried over anhydrous magnesium sulfate, and after filtration, the filtrate was concentrated to give 135 mg of (R or S)-n-propyl 3-amino-3-(4-chlorophenyl)propionate (isolation yield based on n-propyl 3-amino-3-(4-chlorophenyl)propionate (racemic mixtures)=45.0%).

The (S or R)-3-amino-3-(4-chlorophenyl)propionic acid was led to an n-propyl ester according to the conventional manner, and when an optical purity thereof was measured by using high performance liquid chromatography which uses an optically active column, it was 99.8%ee.

When an optical purity of the (R or S)-n-propyl 3-amino-3-(4-chlorophenyl)propionate was measured by using high performance liquid chromatography which uses an optically active column, it was 94.6%ee.

Incidentally, the E value in the present reaction was 4664.
Analytical conditions of high performance liquid chromatography;
Optically active n-propyl 3-amino-3-(4-chlorophenyl)-propionate,
Column: Chiral CD-Ph (0.46cmΦx25cm, available from Shiseido Co., Ltd.)
Solvent: acetonitrile/water (=1/9 (volume ratio))
Potassium dihydrogen phosphate 40 mmol/L
Adjusted to pH 3.5 with phosphoric acid
Flow rate: 1.0 mL/min
Temperatrure: 30°C
Wavelength: 220 nm

Also, physical properties of the (S or R)-3-amino-3-(4-chlorophenyl)propionic acid were as follows.
¹H-NMR (δ (ppm), D₂O) : 2.93 (dd, 1H, J=17.1, 6.8Hz), 3.04 (dd, 1H, J=17.1, 7.8Hz), 4.63 (dd, 1H, J=7.8, 6.8Hz), 7.22 (s, 1H), 7.24 (s, 1H), 7.47 (s, 1H), 7.49 (s, 1H)
¹³C-NMR (δ (ppm), D₂O): 40.4, 53.9, 126.0, 131.9, 135.3, 137.1, 175.9
MS (EI) m/z: 199 (M⁺)
MS (CI, i-C₄H₁₀) m/z: 200 (MH⁺)

Physical properties of the (R or S)-n-propyl 3-amino-3-(4-chlorophenyl)propionate were as follows.
¹H-NMR (δ (ppm), CDCl₃) : 0.91 (t, 3H, J=7.8Hz), 1.62 (qt, 2H, 7.8, 6.8Hz), 1.72 (br, 2H), 2.63 (d, 2H, J=6.8Hz), 4.04 (t, 2H, 6.8Hz), 4.41 (t, 2H, 6.8Hz), 7.31 (s, 4H)
¹³C-NMR (δ (ppm), CDCl₃): 10.4, 21.9, 44.1, 52.1, 66.3, 127.7, 128.7, 133.1, 143.2, 171.8
MS (EI) m/z: 241 (M⁺)
MS (CI, i-C₄H₁₀) m/z: 242 (MH⁺)

### Comparative example 7 (Syntheses of (S or R)-3-amino-3-(4-chlorophenyl)propionic acid and (R or S)-ethyl 3-amino-3-(4-chlorophenyl)propionate)

To a mixed solution comprising 0.75 mL of 50 mmol/L aqueous potassium phosphate solution with a pH of 8.2 and 0.75 mL of t-butyl methyl ether was added 300 mg (1.3 mmol) of ethyl 3-amino-3-(4-chlorophenyl)propionate (racemic mixtures), and the mixture was maintained to 30°C, 15 mg of lipase (Amano Lipase PS (trade name); available from Aldrich Corporation) originated from *Burkholderia cepacia* (*Pseudomonas cepacia*) was added to the mixture at the same temperature, and the mixture was reacted while stirring at 30°C. After 5 hours, 1.0 mL of acetone was added to the reaction mixture and the resulting mixture was filtered to give 113 mg of (S or R)-3-amino-3-(4-chlorophenyl)propionic acid (isolation yield based on ethyl 3-amino-3-(4-chlorophenyl)propionate (racemic mixtures)=43.0%) and 12 mg of an enzyme fixing agent as a mixture. Then, the filtrate was separated, and the organic phase was extracted and dried over anhydrous magnesium sulfate, and after filtration, the filtrate was concentrated to give 135 mg of (R or S)-ethyl 3-amino-3-(4-chlorophenyl)propionate (isolation yield based on ethyl 3-amino-3-(4-chlorophenyl)propionate (racemic mixtures)=45.0%).

The (S or R)-3-amino-3-(4-chlorophenyl)propionic acid was led to an n-propyl ester according to the conventional manner, and when an optical purity thereof was measured by using high performance liquid chromatography which uses an optically active column, it was 99.3%ee.

When an optical purity of the (R or S)-ethyl 3-amino-3-(4-chlorophenyl)propionate was measured by using high performance liquid chromatography which uses an optically active column, it was 99.0%ee.

Incidentally, the E value in the present reaction was 1757.
Analytical conditions of high performance liquid chromatography;
Optically active ethyl 3-amino-3-(4-chlorophenyl)propionate,
Optically active n-propyl 3-amino-3-(4-chlorophenyl)propionate
Column: Chiral CD-Ph (0.46cmΦx25cm, available from Shiseido Co., Ltd.)
Solvent: acetonitrile/water (=1/9 (volume ratio))
Potassium dihydrogen phosphate 40 mmol/L
Adjusted to pH 3.5 with phosphoric acid
Flow rate: 0.5 mL/min
Temperatrure: 30°C
Wavelength: 220 nm

Also, physical properties of the (S or R)-3-amino-3-(4-chlorophenyl)propionic acid were the same as those shown in Example 14.

Physical properties of the (R or S)-ethyl 3-amino-3-(4-chlorophenyl)propionate were as follows.
¹H-NMR (δ (ppm), CDCl₃) : 1.23 (t, 3H, J=7.1Hz), 1.71 (br, 2H), 2.62 (d, 2H, J=6.8Hz), 4.14 (q, 2H, 7.1Hz), 4.40 (t, 1H, d=6.8Hz), 7.30 (s, 4H)
¹³C-NMR (δ (ppm), CDCl₃) : 14.1, 44.1, 52. 0, 60. 6, 127. 6, 128.7, 133.0, 143.1, 171.7
MS (EI) m/z: 227 (M⁺)
MS (CI, i-C₄H₁₀) m/z: 228 (MH⁺)

### Example 15 (Synthesis of 3-amino-3-(4-bromophenyl)propionic acid (racemic mixtures))

To 30 mL of ethanol were added 5.00 g (27.0 mmol) of 4-bromobenzaldehyde, 2.80 g (27.0 mmol) of malonic acid and 3.10 g (40.2 mmol) of ammonium acetate, and the mixture was reacted while stirring under reflux (75 to 80°C) for 10 hours. After completion of the reaction, the obtained reaction mixture was stirred at room temperature for 1 hour and then filtered to give 4.8 g of 3-amino-3-(4-bromophenyl)propionic acid (racemic mixtures) (isolation yield based on 4-bromobenzaldehyde: 72.2%) as white powder.

Incidentally, physical properties of the 3-amino-3-(4-bromophenyl)propionic acid (racemic mixtures) were as follows.
¹H-NMR (δ (ppm), D₂O) : 2.93 (dd, 1H, J=17.1, 6.8Hz), 3.04 (dd, 1H, J=17.1, 7.8Hz), 4.63 (dd, 1H, J=7.8, 6.8Hz), 7.22 (s, 1H), 7.24 (s, 1H), 7.47 (s, 1H), 7.49 (s, 1H)
¹³C-NMR (δ (ppm), D₂O) : 40.4, 53. 9, 126.0, 131.9, 135.3, 137.1, 175.9

### Example 16 (Synthesis of n-propyl 3-amino-3-(4-bromophenyl)propionate (racemic mixtures))

To 18.0 mL (241 mmol) of n-propyl alcohol were added 4.50 g (17.2 mmol) of 3-amino-3-(4-bromophenyl)propionic acid (racemic mixtures) synthesized in Example 15 and 2.70 g (27.5 mmol) of conc. sulfuric acid, and the mixture was reacted while stirring at 55 to 60°C for 2.5 hours. At the same temperature, the solvent was removed by distillation from the reaction system with a predetermined amount, and the same amount of n-propyl alcohol to that of the removed solvent was added to the mixture and the reaction was continued. After completion of the reaction, 9 mL of water was added to the reaction mixture, and a pH of the mixture was adjusted to 7.4 with 6 mol/L aqueous sodium hydroxide solution and the mixture was concentrated under reduced pressure. To the obtained concentrated residue was added 23 mL of t-butyl methyl ether, and a pH of the mixture was adjusted to 10.2 by using 6 mol/L aqueous sodium hydroxide solution. The obtained mixture was allowed to stand, liquids were separated and the organic phase was extracted and dried over anhydrous magnesium sulfate. After filtration, the filtrate was concentrated under reduced pressure to give 4.93 g of n-propyl 3-amino-3-(4-bromophenyl)-propionate (racemic mixtures) (isolation yield based on 3-amino-3-(4-bromophenyl)propionic acid (racemic mixtures): 84.1%) as colorless liquid.

Incidentally, physical properties of the n-propyl 3-amino-3-(4-bromophenyl)propionate (racemic mixtures) were as follows.
¹H-NMR (δ (ppm), CDCl₃): 0.91 (t, 3H, J=7.3Hz), 1.64 (qt, 2H, 7.3, 6.8Hz), 1.71 (br, 2H), 2.63 (d, 2H, J=6.8Hz), 4.04 (t, 2H, 6.8Hz), 4.39 (t, 2H, 6.8Hz), 7.31 (s, 4H)
¹³C-NMR (δ (ppm), CDCl₃): 10.4, 21.9, 44.1, 52.1, 66.3, 127.7, 128.1, 129.4, 131.7, 132.1171.8
MS (EI) m/z: 285, 287 (M⁺)
MS (CI, i-C₄H₁₀) m/z: 286, 288 (MH⁺)

### Example 17 (Syntheses of (S or R)-3-amino-3-(4-bromophenyl)propionic acid and (R or S)-n-propyl 3-amino-3-(4-bromophenyl)propionate)

To a mixture comprising 0.75 mL of 50 mmol/L aqueous potassium phosphate solution with a pH of 8.2 and 0.75 mL of t-butyl methyl ether was added 300 mg (1.1 mmol) of n-propyl 3-amino-3-(4-bromophenyl)propionate acid ester (racemic mixtures) synthesized in Example 16, and the mixture was maintained to 30°C, 15 mg of lipase (Amano Lipase PS (trade name); available from Aldrich Corporation) originated from *Burkholderia cepacia* (*Pseudomonas cepacia*) was added to the mixture at the same temperature, and the mixture was reacted while stirring at 30°C. After 5 hours, 1.0 mL of acetone was added to the reaction mixture and the resulting mixture was filtered to give 113 mg of (S or R)-3-amino-3-(4-bromophenyl)propionic acid (isolation yield based on n-propyl 3-amino-3-(4-bromophenyl)propionate (racemic mixtures)=44.0%) and 12 mg of an enzyme fixing agent as a mixture. Then, liquids of the filterate were separated, and the organic phase was extracted and dried over anhydrous magnesium sulfate, and after filtration, the filtrate was concentrated to give 135 mg of (R or S)-n-propyl 3-amino-3-(4-bromophenyl)propionate (isolation yield based on n-propyl 3-amino-3-(4-bromophenyl)propionate (racemic mixtures)=45.0%).

The (S or R)-3-amino-3-(4-bromophenyl)propionic acid was led to an n-propyl ester according to the conventional manner, and when an optical purity thereof was measured by using high performance liquid chromatography which uses an optically active column, it was 99.7%ee.

When an optical purity of the (R or S)-n-propyl 3-amino-3-(4-bromophenyl)propionate was measured by using high performance liquid chromatography which uses an optically active column, it was 94.0%ee.

Incidentally, the E value in the present reaction was 2779.
Analytical conditions of high performance liquid chromatography;
optically active n-propyl 3-amino-3-(4-bromophenyl)propionate
Column: Chiral CD-Ph (0.46cmΦx25cm, available from Shiseido Co., Ltd.)
Solvent: acetonitrile/water (=1/9 (volume ratio))
Potassium dihydrogen phosphate 40 mmol/L
Adjusted to pH 3.5 with phosphoric acid
Flow rate: 0.5 mL/min
Temperatrure: 30°C
Wavelength: 220 nm

Also, physical properties of the (S or R)-3-amino-3-(4-bromophenyl)propionic acid were as follows.
¹H-NMR (δ (ppm), D₂O): 2.93 (dd, 1H, J=17.1, 6.8Hz), 3.04 (dd, 1H, J=17.1, 7.8Hz), 4.63 (dd, 1H, J=7.8, 6.8Hz), 7.22 (s, 1H), 7.24 (s, 1H), 7.47 (s, 1H), 7.49 (s, 1H)
¹³C-NMR (δ (ppm), D₂O): 40.4, 53.9, 126.0, 131.9, 135.3, 137.1, 175.9

Physical properties of the (R or S)-n-propyl 3-amino-3-(4-bromophenyl)propionate were as follows.
¹H-NMR (δ (ppm), CDCl₃): 0.91 (t, 3H, J=7.3Hz), 1.64 (qt, 2H, 7.3, 6.8Hz), 1.71 (br, 2H), 2.63 (d, 2H, J=6.8Hz), 4.04 (t, 2H, 6.8Hz), 4.39 (t, 2H, 6.8Hz), 7.31 (s, 4H)
¹³C-NMR (δ (ppm), CDCl₃): 10.4, 21.9, 44.1, 52.1, 66.3, 127.7, 128.1, 129.4, 131.7, 132.1171.8
MS (EI) m/z: 285, 287 (M⁺)
MS (CI, i-C₄H₁₀) m/z: 286, 288 (MH⁺)

### Comparative example 8 (Syntheses of (S or R)-3-amino-3-(4-bromophenyl)propionic acid and (R or S)-ethyl 3-amino-3-(4-bromophenyl)propionate)

To a mixture comprising 0.75 mL of 50 mmol/L aqueous potassium phosphate solution with a pH of 8.2 and 0.75 mL of t-butyl methyl ether was added 300 mg (1.3 mmol) of ethyl 3-amino-3-(4-bromophenyl)propionate (racemic mixtures) and the mixture was maintained to 30°C, 15 mg of lipase (Amano Lipase PS (trade name); available from Aldrich Corporation) originated from *Burkholderia cepacia* (*Pseudomonas cepacia*) was added to the mixture at the same temperature, and the mixture was reacted while stirring at 30°C. After 6 hours, 1.0 mL of acetone was added to the reaction mixture and the resulting mixture was filtered to give 116 mg of (S or R)-3-amino-3-(4-bromophenyl)propionic acid (isolation yield based on ethyl 3-amino-3-(4-bromophenyl)propionate (racemic mixtures)=43.0%) and 13 mg of an enzyme fixing agent as a mixture. Then, liquids of the filterate were separated, and the organic phase was extracted and dried over anhydrous magnesium sulfate, and after filtration, the filtrate was concentrated to give 132 mg of (R or S)-ethyl 3-amino-3-(4-bromophenyl)propionate (isolation yield based on ethyl 3-amino-3-(4-bromophenyl)-propionate (racemic mixtures)=44.0%).

The (S or R)-3-amino-3-(4-bromophenyl)propionic acid was led to an n-propyl ester according to the conventional manner, and when an optical purity thereof was measured by using high performance liquid chromatography which uses an optically active column, it was 99.4%ee.

When an optical purity of the (R or S)-ethyl 3-amino-3-(4-bromophenyl)propionate was measured by using high performance liquid chromatography which uses an optically active column, it was 95.5%ee.

Incidentally, the E value in the present reaction was 1219.
Analytical conditions of high performance liquid chromatography;
Optically active ethyl 3-amino-3-(4-bromophenyl)propionate Optically active n-propyl 3-amino-3-(4-bromophenyl)propionate
Column: Chiral CD-Ph (0.46cmΦx25cm, available from Shiseido Co., Ltd.)
Solvent: acetonitrile/water (=1/9 (volume ratio))
Potassium dihydrogen phosphate 40 mmol/L
Adjusted to pH 3.5 with phosphoric acid
Flow rate: 0.5 mL/min
Temperatrure: 30°C
Wavelength: 220 nm

Also, physical properties of the (S or R)-3-amino-3-(4-bromophenyl)propionic acid were the same as those shown in Example 17.

Physical properties of the (R or S)-ethyl 3-amino-3-(4-bromophenyl)propionate were as follows.
¹H-NMR (δ (ppm), CDCl₃): 1.23 (t, 3H, J=7.3Hz), 1.73 (br, 2H), 2.61 (d, 2H, J=6.8Hz), 4.14 (q, 2H, 7.3Hz), 4.39 (t, 1H, d=6.8Hz), 7.23-7.27 (m, 2H), 7.44-7.47 (m, 2H)
¹³C-NMR (δ (ppm), CDCl₃) : 14.2, 44.1, 52.1, 60.6, 128.1, 131.7, 132.1, 143.7, 171.7
MS (EI) m/z: 271, 273 (M⁺)
MS (CI, i-C₄H₁₀) m/z: 272, 274 (MH⁺)

### Example 18 (Synthesis of 3-amino-3-(4-fluorophenyl)-propionic acid (racemic mixtures))

To 150 mL of ethanol were added 30.5 g (0.25 mol) of 4-fluorobenzaldehyde, 25.6 g (0.25 mol) of malonic acid and 28.4 g (0.37 mol) of ammonium acetate, and the mixture was reacted while stirring under reflux (75 to 80°C) for 8 hours. After completion of the reaction, the obtained reaction mixture was stirred at room temperature for 1 hour, filtered and dried under reduced pressure at 45°C to give 28.3 g of 3-amino-3-(4-fluorophenyl)propionic acid (racemic mixtures) (isolation yield based on 4-fluorobenzaldehyde: 62.8%) as white powder.

Incidentally, physical properties of the 3-amino-3-(4-fluorophenyl)propionic acid (racemic mixtures) were as follows.
¹H-NMR (δ (ppm), D₂O): 2.49 (dd, 1H, J=17.1, 6.8Hz), 2.62 (dd, 1H, J=17.1, 7.8Hz), 4.20 (dd, 1H, J=7.8, 6.8Hz), 6.58-6.62 (m, 2H), 6.88-6.91 (s, 1H)
¹³C-NMR (δ (ppm), D₂O): 37.1, 50.5.1, 115.5, 115.8, 128.9, 130.4, 172.6
MS (EI) m/z: 183 (M⁺)
MS (CI, i-C₄H₁₀) m/z: 184 (MH⁺)

### Example 19 (Synthesis of 3-amino-3-(4-fluorophenyl)-propionic acid n-propyl ester (racemic mixtures))

To 65.0 mL (870 mmol) of n-propyl alcohol were added 13.0 g (71.0 mmol) of 3-amino-3-(4-fluorophenyl)propionic acid (racemic mixtures) synthesized in Example 18 and 10.4 g (106 mmol) of conc. sulfuric acid, and the mixture was reacted while stirring at 55 to 60°C for 2 hours. At the same temperature, the solvent was removed by distillation from the reaction system with a predetermined amount, and the same amount of n-propyl alcohol to that of the removed solvent was added to the mixture and the reaction was continued. After completion of the reaction, 20 mL of water was added to the mixture, a pH of the mixture was adjusted to 7.5 with 6 mol/L aqueous sodium hydroxide solution and the mixture was concentrated under reduced pressure. To the obtained concentrated residue was added 65 mL of t-butyl methyl ether, and a pH of the mixture was adjusted to 10.2 by using 6 mol/L aqueous sodium hydroxide solution. The obtained mixture was allowed to stand, liquids were separated, the organic phase was extracted, washed with 50 mL of saturated sodium chloride and dried over anhydrous magnesium sulfate. After filtration, the filtrate was concentrated under reduced pressure to give 13.5 g of n-propyl 3-amino-3-(4-fluorophenyl)propionate (racemic mixtures) (isolation yield based on 3-amino-3-(4-fluorophenyl)propionic acid (racemic mixtures): 84.6%) as colorless liquid.

Incidentally, physical properties of the n-propyl 3-amino-3-(4-fluorophenyl)propionate (racemic mixtures) were as follows.
¹H-NMR (δ (ppm), CDCl₃) : 0.91 (t, 3H, J=7.3Hz), 1.62 (qt, 2H, 7.3, 6.8Hz), 1.72 (br, 2H), 2.64 (d, 2H, J=6.8Hz), 4.04 (t, 2H, 6.8Hz), 4.42 (t, 2H, 6.8Hz), 6.99-7.04 (m, 2H), 7.32-7.35 (m, 2H)
¹³C-NMR (δ (ppm), CDCl₃): 10.3, 21.9, 44.3, 52.0, 66.2, 115.2, 115.4, 127.8, 127.9, 171.9
MS (EI) m/z: 225 (M⁺)
MS (CI, i-C₄H₁₀) m/z: 226 (MH⁺)

### Example 20 (Syntheses of (S or R)-3-amino-3-(4-fluorophenyl)propionic acid and (R or S)-n-propyl 3-amino-3-(4-fluorophenyl)propionate)

To a mixture comprising 0.75 mL of 50 mmol/L aqueous potassium phosphate solution with a pH of 8.2 and 0.75 mL of t-butyl methyl ether was added 300 mg (1.3 mmol) of n-propyl 3-amino-3-(4-fluorophenyl)propionate (racemic mixtures) synthesized in Example 19, and the mixture was maintained to 30°C, 15 mg of lipase (Amano Lipase PS (trade name); available from Aldrich Corporation) originated from *Burkholderia cepacia* (*Pseudomonas cepacia*) was added to the mixture at the same temperature, and the mixture was reacted while stirring at 30°C. After 5 hours, 1.0 mL of acetone was added to the reaction mixture and the resulting mixture was filtered to give 98 mg of (S or R)-3-amino-3-(4-fluorophenyl)propionic acid (isolation yield based on n-propyl 3-amino-3-(4-fluorophenyl)propionate (racemic mixtures)=40.0%) and 12 mg of an enzyme fixing agent as a mixture. Then, liquids of the filterate were separated, and the organic phase was extracted, washed with a saturated aqueous sodium chloride solution, dried over anhydrous magnesium sulfate, and after filtration, the filtrate was concentrated to give 135 mg of (R or S)-n-propyl 3-amino-3-(4-fluorophenyl)propionate (isolation yield based on n-propyl 3-amino-3-(4-fluorophenyl)propionate (racemic mixtures)=45.0%).

The (S or R)-3-amino-3-(4-fluorophenyl)propionic acid was led to an n-propyl ester according to the conventional manner, and further led to n-propyl (S or R)-3-(2,3-dimethoxybenzoylamino)-3-(4-fluorophenyl)propionate, and when an optical purity thereof was measured by using high performance liquid chromatography which uses an optically active column, it was 98.1%ee.

The (R or S)-n-propyl 3-amino-3-(4-fluorophenyl)propionate was led to (R or S)-n-propyl 3-(2,3-dimethoxybenzoylamino)-3-(4-fluorophenyl)propionate and when an optical purity thereof was measured by using high performance liquid chromatography which uses an optically active column, it was 99.4%ee.

Incidentally, the E value in the present reaction was 607.
Analytical conditions of high performance liquid chromatography;
Optically active n-propyl 3-(2,3-dimethoxybenzoylamino)-3-(4-fluorophenyl)propionate,
Column: Chiralcel OJ-H (0.46cmΦx25cm, available from
DAICEL CHEMICAL INDUSTRIES, LTD.)
Solvent: hexane/isopropyl alcohol (=5/95 (volume ratio)) Flow rate: 0.5 mL/min
Temperatrure: 30°C
Wavelength: 220 nm

Also, physical properties of the (S or R)-3-amino-3-(4-fluorophenyl)propionic acid were as follows.
¹H-NMR (δ (ppm), D₂O) : 2.49 (dd, 1H, J=17.1, 6.8Hz), 2.62 (dd, 1H, J=17.1, 7.8Hz), 4.20 (dd, 1H, J=7.8, 6.8Hz), 6.58-6.62 (m, 2H), 6.88-6.91 (s, 1H)
¹³C-NMR (δ (ppm), D₂O) : 37.1, 50.5.1, 115.5, 115.8, 128.9, 130.4, 172.6
MS (EI) m/z: 183 (M⁺)
MS (CI, i-C₄H₁₀) m/z: 184 (MH⁺)

Physical properties of the (R or S)-n-propyl 3-amino-3-(4-fluorophenyl)propionate were as follows.
¹H-NMR (δ (ppm), CDCl₃) : 0.91 (t, 3H, J=7.3Hz), 1.62 (qt, 2H, 7.3, 6.8Hz), 1.72 (br, 2H), 2.64 (d, 2H, J=6.8Hz), 4.04 (t, 2H, 6.8Hz), 4.42 (t, 2H, 6.8Hz), 6.99-7.04 (m, 2H),
7.32-7.35 (m, 2H)
¹³C-NMR (δ (ppm), CDCl₃) : 10.3, 21.9, 44.3, 52.0, 66.2, 115.2, 115.4, 127.8, 127.9, 171.9
MS (EI) m/z: 225 (M⁺)
MS (CI, i-C₄H₁₀) m/z: 226 (MH⁺)

### Comparative example 9 (Syntheses of (S or R)-3-amino-3-(4-fluorophenyl)propionic acid and (R or S)-ethyl 3-amino-3-(4-fluorophenyl)propionate)

To a mixture comprising 0.75 mL of 50 mmol/L aqueous potassium phosphate solution with a pH of 8.2 and 0.75 mL of t-butyl methyl ether was added 300 mg (1.3 mmol) of ethyl 3-amino-3-(4-fluorophenyl)propionate (racemic mixtures), and the mixture was maintained to 30°C, 15 mg of lipase (Amano Lipase PS (trade name); available from Aldrich Corporation) originated from *Burkholderia cepacia* (*Pseudomonas cepacia*) was added to the mixture at the same temperature, and the mixture was reacted while stirring at 30°C. After 6 hours, 1.0 mL of acetone was added to the reaction mixture and the resulting mixture was filtered to give 116 mg of (S or R)-3-amino-3-(4-fluorophenyl)propionic acid (isolation yield based on ethyl 3-amino-3-(4-fluorophenyl)propionate (racemic mixtures)=43.0%) and 13 mg of an enzyme fixing agent as a mixture. Then, liquids of the filterate were separated, and the organic phase was extracted and dried over anhydrous magnesium sulfate, and after filtration, the filtrate was concentrated to give 132 mg of (R or S)-ethyl 3-amino-3-(4-fluorophenyl)propionate (isolation yield based on ethyl 3-amino-3-(4-fluorophenyl)-propionate (racemic mixtures)=44.0%).

The (S or R)-3-amino-3-(4-fluorophenyl)propionic acid was led to an n-propyl ester according to the conventional manner, and further led to n-propyl (S or R)-3-(2,3-dimethoxybenzoylamino)-3-(4-fluorophenyl)propionate, and when an optical purity thereof was measured by using high performance liquid chromatography which uses an optically active column, it was 94.3%ee.

The (R or S)-ethyl 3-amino-3-(4-fluorophenyl)propionate was led to (R or S)-ethyl 3-(2,3-dimethoxybenzoylamino)-3-(4-fluorophenyl)propionate, and when an optical purity thereof was measured by using high performance liquid chromatography which uses an optically active column, it was 88.6%ee.

Incidentally, the E value in the present reaction was 101.
Analytical conditions of high performance liquid chromatography;
Optically active n-propyl 3-(2,3-dimethoxybenzoylamino)-3-(4-fluorophenyl)propionate,
Optically active ethyl 3-(2,3-dimethoxybenzoylamino)-3-(4-fluorophenyl)propionate
Column: Chiralcel OJ-H (0.46cmΦ×25cm, available from
DAICEL CHEMICAL INDUSTRIES, LTD.)
Solvent: hexane/isopropyl alcohol (=5/95 (volume ratio)) Flow rate: 0.5 mL/min
Temperatrure: 30°C
Wavelength: 220 nm

Also, physical properties of the (S or R)-3-amino-3-(4-fluorophenyl)propionic acid were the same as those shown in Example 20.

Physical properties of the (R or S)-ethyl 3-amino-3-(4-fluorophenyl)propionate acid ester were as follows.
¹H-NMR (δ (ppm), CDCl₃) : 1.23 (t, 3H, J=7.1Hz), 1.73 (br, 2H), 2.62 (d, 2H, J=6.8Hz), 4.13 (q, 2H, 7.1Hz), 4.41 (t, 1H, d=6.8Hz), 6.99-7.04 (m, 2H), 7.32-7.35 (m, 2H)
¹³C-NMR (δ (ppm), CDCl₃) : 14.2, 44.3, 52.0, 60.6, 115. 3, 115.5, 127.8, 127.9, 171.8
MS (EI) m/z: 211 (M⁺)
MS (CI, i-C₄H₁₀) m/z: 212 (MH⁺)

### Example 21 (Synthesis of 3-amino-3-(3-fluorophenyl)-propionic acid (racemic mixtures))

To 5.00 g (40.3 mmol) of 3-fluorobenzaldehyde were added 20 mL of ethanol, 4.19 g (40.3 mmol) of malonic acid and 4.66 g (60.4 mmol) of ammonium acetate, and the mixture was reacted while stirring under reflux (80 to 90)°C for 7 hours. After completion of the reaction, the obtained reaction mixture was stirred at 0 to 5°C for 1 hour and filtered to give 4.27 g of 3-amino-3-(3-fluorophenyl)-propionic acid (racemic mixtures) (isolation yield based on 3-fluorobenzaldehyde: 57.9%) as white powder.

Incidentally, physical properties of the 3-amino-3-(3-fluorophenyl)propionic acid (racemic mixtures) were as follows.
¹H-NMR (δ (ppm), D₂O) : 2.83 (dd, 1H, J=16.1, 6.8Hz), 2.90 (dd, 1H, J=16.1, 7.8Hz), 4.67 (dd, 1H, J=6.8, 7.8Hz), 7.2-7.5 (m, 5H)
¹³C-NMR (δ (ppm), D₂O) : 43.4, 55.1, 116.9, 119.0, 125.7, 134.0, 141.5, 164.3, 179.9
MS (EI) m/z: 183 (M⁺)
MS (CI, i-C₄H₁₀) m/z: 184 (MH⁺)

### Example 22 (Synthesis of n-propyl 3-amino-3-(3-fluorophenyl)propionate (racemic mixtures))

To 4.00 g (21.8 mmol) of 3-amino-3-(3-fluorophenyl)-propionic acid (racemic mixtures) synthesized in Example 21 were added 12.0 mL of n-propyl alcohol and 3.12 g (32.8 mmol) of conc. sulfuric acid, and the mixture was stirred at 55°C for 2 hours. After 2 hours, 6 mL of a volatile material was removed under reduced pressure, 6 mL of n-propyl alcohol was added to the residue, and the mixture was stirred at the same temperature. After 1 hour, 6 mL of a volatile material was removed under reduced pressure, 6 mL of n-propyl alcohol was added to the residue, and the mixture was stirred at the same temperature. After 1 hour, the mixture was cooled to room temperature and 8 mL of water was added thereto. Then, 6 mol/L aqueous sodium hydroxide solution was added to the mixture whereby a pH of the reaction mixture was adjusted to 7.0. Under reduced pressure, a volatile material was removed, 20 mL of ethyl acetate was added, and 6 mol/L aqueous sodium hydroxide solution was added to the mixture whereby a pH of the reaction mixture was adjusted to 8.5. The organic phase was extracted and washed with 4 mL of saturated brine. The obtained organic phase was dried over anhydrous magnesium sulfate. After filtration, the filtrate was concentrated under reduced pressure to give 4.50 g of n-propyl 3-amino-3-(3-fluorophenyl)propionate (racemic mixtures) (isolation yield based on 3-amino-3-(3-fluorophenyl)propionic acid (racemic mixtures): 91.5%) as colorless liquid.

Incidentally, physical properties of the n-propyl 3-amino-3-(3-fluorophenyl)propionate (racemic mixtures) were as follows.
¹H-NMR (δ (ppm), CDCl₃) : 0.91 (t, 3H, J=7.3Hz), 1.62 (tq, 2H, J=6.8, 7.3Hz), 2.63 (dd, 1H, J=5.8, 16.1Hz), 2.67 (d, 1H, J=7.8, 16.1Hz), 4.05 (t, 2H, J=6.8Hz), 4.42 (dd, 1H, J=5.8, 7.8Hz), 6.9-7.3 (m, 5H)
¹³C-NMR (δ (ppm), CDCl₃): 10.3, 21.9.44.1, 52.3, 66.3, 113.3, 114.4, 121.9, 130.1, 147.3, 161.8, 171.8
MS (EI) m/z: 225 (M⁺)
MS (CI, i-C₄H₁₀) m/z: 226 (MH⁺)

### Example 23 (Syntheses of (S or R)-3-amino-3-(3-fluorophenyl)propionic acid and (R or S)-3-amino-3-(3-fluorophenyl)propionic acid n-propyl ester)

To a mixture comprising 248 g (1.10 mol) of n-propyl 3-amino-3-(3-fluorophenyl)propionate (racemic mixtures) synthesized in Example 22 and 620 mL of cyclohexane was added 620 mL of 50 mmol/L aqueous potassium phosphate solution with a pH of 8.2, and the mixture was maintained to 30°C. To the obtained mixture was added 12.4 g of lipase (Amano Lipase PS (trade name); available from Aldrich Corporation) originated from *Burkholderia cepacia* (*Pseudomonas cepacia*) at the same temperature, and the mixture was reacted while stirring at 30°C. After 10 hours, 620 mL of cyclohexane was added to the reaction mixture, and the mixture was cooled to 12°C. To the obtained mixture was added 201 g of sodium chloride, and the mixture was stirred at the same temperature. After 1 hour, the mixture was filtered under reduced pressure, washed with 250 mL of cyclohexane, and dried at 45°C under reduced pressure to give 89.9 g of (S or R)-3-amino-3-(3-fluorophenyl)propionic acid (isolation yield based on n-propyl 3-amino-3-(3-fluorophenyl)propionate (racemic mixtures)=45.0%) as white crystals, sodium chloride and an enzyme fixing agent as a mixture. Also, an organic phase was extracted from the filtrate, and washed with saturated brine, and dried over magnesium sulfate. After filtration, the filtrate was concentrated under reduced pressure to give 119 g of (R or S)-n-propyl 3-amino-3-(3-fluorophenyl)-propionate (isolation yield based on n-propyl 3-amino-3-(3-fluorophenyl)propionate (racemic mixtures): 48.0%) as colorless liquid.

When an optical purity of the (S or R)-3-amino-3-(3-fluorophenyl)propionic acid was measured by using high performance liquid chromatography which uses an optically active column, it was 99.9%ee.

The (R or S)-n-propyl 3-amino-3-(3-fluorophenyl)-propionate was led to (R or S)-n-propyl 3-(4-toluoylamino)-3-(3-fluorophenyl)propionate, and when an optical purity thereof was measured by using high performance liquid chromatography which uses an optically active column, it was 99.9%ee.

Incidentally, the E value in the present reaction was larger than 10000.
Analytical conditions of high performance liquid chromatography;
Optically active 3-amino-3-(3-fluorophenyl)propionic acid Column: Chiral CD-Ph (0.46cmΦ×25cmx2 columns connected, available from Shiseido Co., Ltd.)
Solvent: acetonitrile/water (=5/95 (volume ratio))
Potassium dihydrogen phosphate 40 mmol/L
Adjusted to pH 3.5 with phosphoric acid
Flow rate: 0.5 mL/min
Temperatrure: 30°C
Wavelength: 220 nm
Optically active n-propyl 3-(4-toluoylamino)-3-(3-fluorophenyl)propionate
Column: Chiralcel OJ-H (0.46cmΦ×25cm, available from
DAICEL CHEMICAL INDUSTRIES, LTD.)
Solvent: hexane/isopropyl alcohol (=5/95 (volume ratio)) Flow rate: 0.5 mL/min
Temperatrure: 30°C
Wavelength: 220 nm

Also, physical properties of the (S or R)-3-amino-3-(3-fluorophenyl)propionic acid were as follows.
¹H-NMR (δ (ppm), D₂O) : 2.83 (dd, 1H, J=16.1, 6.8Hz), 2.90 (dd, 1H, J=16.1, 7.8Hz), 4.67 (dd, 1H, J=6.8, 7.8Hz), 7.2-7.5 (m, 5H)
¹³C-NMR (δ (ppm), D₂O) : 43.4, 55.1, 116.9, 119.0, 125.7, 134.0, 141.5, 164.3, 179.9
MS (EI) m/z: 183 (M⁺)
MS (CI, i-C₄H₁₀) m/z: 184 (MH⁺)

The (S or R)-3-amino-3-(3-fluorophenyl)propionic acid was led to an ethyl ester hydrochloride according to the conventional manner and a specific rotation was measured. (S or R)-ethyl 3-amino-3-(3-fluorophenyl)propionate hydrochloride: [α]²⁵_{D} +10.7° (c 1.00, H₂O)

Physical properties of the (R or S)-n-propyl 3-amino-3-(3-fluorophenyl)propionate were as follows.
¹H-NMR (δ (ppm), CDCl₃) : 0.91 (t, 3H, J=7.3Hz), 1.62 (tq, 2H, J=6.8, 7.3Hz), 2.63 (dd, 1H, J=5.8, 16.1Hz), 2.67 (d, 1H, J=7.8, 16.1Hz), 4.05 (t, 2H, J=6.8Hz), 4.42 (dd, 1H, J=5.8, 7.8Hz), 6.9-7.3 (m, 5H)
¹³C-NMR (δ (ppm), CDCl₃) : 10. 3, 21. 9. 44. 1, 52. 3, 66. 3, 113.3, 114.4, 121.9, 130.1, 147.3, 161.8, 171.8
MS (EI) m/z: 225 (M⁺)
MS (CI, i-C₄H₁₀) m/z: 226 (MH⁺)

### Comparative example 10 (Syntheses of (S or R)-3-amino-3-(3-fluorophenyl)propionic acid and (R or S)-ethyl 3-amino-3-(3-fluorophenyl)propionate)

To a mixture comprising 100 mg (0.473 mmol) of ethyl 3-amino-3-(3-fluorophenyl)propionate (racemic mixtures) and 250 µL of cyclohexane was added 250 µL of 50 mmol/L aqueous potassium phosphate solution with a pH of 8.2 and the mixture was maintained to 30°C. To the obtained mixture was added 5.0 mg of lipase (Amano Lipase PS (trade name); available from Aldrich Corporation) originated from *Burkholderia cepacia* (*Pseudomonas cepacia*) at the same temperature, and the mixture was reacted while stirring at 30°C. After 5 hours, 250 µL of cyclohexane was added to the reaction mixture, and the mixture was cooled to 12°C. To the obtained mixture was added 90 mg of sodium chloride, and the mixture was stirred at the same temperature. After 1 hour, the mixture was filtered under reduced pressure, washed with 1 mL of cyclohexane, and dried at 45°C under reduced pressure to give 39.0 mg of (S or R)-3-amino-3-(3-fluorophenyl)propionic acid (isolation yield based on ethyl 3-amino-3-(3-fluorophenyl)propionate (racemic mixtures)= 45.0%) as white crystals, sodium chloride and an enzyme fixing agent as a mixture. Also, an organic layer was extracted from the filtrate, washed with saturated brine, and dried over magnesium sulfate. After filtration, the filtrate was concentrated under reduced pressure to give 48.0 mg of (R or S)-ethyl 3-amino-3-(3-fluorophenyl)propionate (isolation yield based on ethyl 3-amino-3-(3-fluorophenyl)propionate (racemic mixtures): 48.0%) as colorless liquid.

When an optical purity of the (S or R)-3-amino-3-(3-fluorophenyl)propionic acid was measured by using high performance liquid chromatography which uses an optically active column, it was 95.6%ee.

The (R or S)-ethyl 3-amino-3-(3-fluorophenyl)propionate was led to (R or S)-ethyl 3-(4-toluoylamino)-3-(3-fluorophenyl)propionate, and when an optical purity thereof was measured by using high performance liquid chromatography which uses an optically active column, it was 95.0%ee.

Incidentally, the E value in the present reaction was 167.
Analytical conditions of high performance liquid chromatography;
Optically active 3-amino-3-(3-fluorophenyl)propionic acid Column: Chiral CD-Ph (0.46cmΦ×25cmx2 columns connected, available from Shiseido Co., Ltd.)
Solvent: acetonitrile/water (=5/95 (volume ratio))
Potassium dihydrogen phosphate 40 mmol/L
Adjusted to pH 3.5 with phosphoric acid
Flow rate: 0.5 mL/min
Temperatrure: 30°C
Wavelength: 220 nm
Optically active ethyl 3-(4-toluoylamino)-3-(3-fluorophenyl)propionate
Column: Chiralcel OJ-H (0.46cmΦx25cm, available from
DAICEL CHEMICAL INDUSTRIES, LTD.)
Solvent: hexane/isopropyl alcohol (=5/95 (volume ratio)) Flow rate: 0.5 mL/min
Temperatrure: 30°C
Wavelength: 220 nm

Also, physical properties of the (S or R)-3-amino-3-(3-fluorophenyl)propionic acid were the same as those shown in Example 23.

Physical properties of the (R or S)-ethyl 3-amino-3-(3-fluorophenyl)propionate were as follows.
¹H-NMR (δ (ppm), CDCl₃): 1.24 (t, 3H, J=7.3Hz), 2.61 (dd, 1H, J=7.8, 16.1Hz), 2.67 (dd, 1H, J=5.8, 16.1Hz), 4.14 (t, 2H, J=7.3Hz), 4.42 (dd, 1H, J=5.8, 7.8Hz), 6.9-7.3 (m, 5H) ¹³C-NMR (δ (ppm), CDCl₃): 14.2, 44.1, 52.3, 60.6, 113.3, 114.4, 121.9, 130.1, 147.3, 161.8, 171.7
MS (EI) m/z: 211 (M⁺)
MS (CI, i-C₄H₁₀) m/z: 212 (MH⁺)

### Example 24 (Synthesis of 3-amino-3-(4-methoxyphenyl)-propionic acid (racemic mixtures))

To 150 mL of ethanol were added 28.5 g (0.21 mol) of 4-methoxybenzaldehyde, 21.8 g (0.21 mol) of malonic acid and 32.3 g (0.42 mol) of ammonium acetate, and the mixture was reacted while stirring under reflux (75 to 80°C) for 8 hours. After completion of the reaction, the obtained reaction mixture was stirred at room temperature for 20 hours, and filtered at room temperature to give 23.6 g of 3-amino-3-(4-methoxyphenyl)propionic acid (racemic mixtures) (isolation yield based on 4-methoxybenzaldehyde: 82.3%) as white powder.

Incidentally, physical properties of the 3-amino-3-(4-methoxyphenyl)propionic acid (racemic mixtures) were as follows.
¹H-NMR (δ (ppm), D₂O) : 2.50 (dd, 1H, J=17.1, 6.8Hz), 2.65 (dd, 1H, J=17.1, 7.8Hz), 3.24 (s, 3H), 6.46 (s, 1H), 6.48 (s, 1H), 6.86 (s, 1H), 6.88 (s, 1H)
¹³C-NMR (δ (ppm), D₂O) : 37.1, 50.7, 55.1, 114.3, 126.9, 128.2, 159.1, 172.7
MS (EI) m/z: 195 (M⁺)
MS (CI, i-C₄H₁₀) m/z: 196 (MH⁺)

### Example 25 (Synthesis of n-propyl 3-amino-3-(4-methoxyphenyl)propionate (racemic mixtures))

To 50.0 mL (669 mmol) of n-propyl alcohol were added 10.0 g (51.2 mmol) of 3-amino-3-(4-methoxyphenyl)propionic acid (racemic mixtures) synthesized in Example 24 and 7.50 g (76.5 mmol) of conc. sulfuric acid, and the mixture was reacted while stirring at 55 to 60°C for 2.5 hours. At the same temperature, the solvent was removed by distillation from the reaction system with a predetermined amount, and the same amount of n-propyl alcohol to that of the removed solvent was added to the mixture and the reaction was continued. After completion of the reaction, 20 mL of water was added to the reaction mixture, a pH of the mixture was adjusted to 7.7 with 6 mol/L sodium hydroxide and the mixture was concentrated under reduced pressure. To the obtained concentrated residue was added 50 mL of t-butyl methyl ether, and a pH of the mixture was adjusted to 10.0 by using 6 mol/L aqueous sodium hydroxide solution. The obtained mixture was allowed to stand and the extracted organic phase was dried over anhydrous magnesium sulfate. After filtration, the filtrate was concentrated under reduced pressure to give 11.0 g of n-propyl 3-amino-3-(4-methoxyphenyl)propionate (racemic mixtures) (isolation yield based on 3-amino-3-(4-methoxyphenyl)propionic acid (racemic mixtures): 90.5%) as colorless liquid as colorless liquid.

Incidentally, physical properties of the 3-amino-3-(4-methoxyphenyl)propionic acid n-propyl ester (racemic mixtures) were as follows.
¹H-NMR (δ (ppm), CDCl₃) : 0.91 (t, 3H, J=7.3Hz), 1.62 (qt, 2H, 7.3, 6.8Hz), 1.84 (br, 2H), 2.64 (d, 2H, J=7.3Hz), 3.79 (s, 3H), 4.03 (t, 2H, 6.8Hz), 4.38 (t, 1H, d=7.3Hz), 6.85 (s, 1H), 6.88 (s, 1H), 7.27 (s, 1H), 7.29 (s, 1H)
¹³C-NMR (δ (ppm), CDCl₃) : 10.4, 22.0, 44.3, 52.1, 55.3, 66.1, 114.0, 127.3, 136.8, 158.9, 172.2
MS (EI) m/z: 237 (M⁺)
MS (CI, i-C₄H₁₀) m/z: 238 (MH⁺)

### Example 26 (Syntheses of (S or R)-3-amino-3-(4-methoxyphenyl)propionic acid and (R or S)-n-propyl 3-amino-3-(4-methoxyphenyl)propionate)

To a mixture comprising 0.75 mL of 50 mmol/L aqueous potassium phosphate solution with a pH of 8.2 and 0.75 mL of t-butyl methyl ether was added 300 mg (1.3 mmol) of n-propyl 3-amino-3-(4-methoxyphenyl)propionate (racemic mixtures) synthesized in Example 25, and the mixture was maintained to 30°C, 15 mg of lipase (Amano Lipase PS (trade name); available from Aldrich Corporation) originated from *Burkholderia cepacia* (*Pseudomonas cepacia*) was added to the mixture at the same temperature, and the mixture was reacted while stirring at 30°C. After 5 hours, 1.0 mL of acetone was added to the reaction mixture and the resulting mixture was filtered to give 109 mg of (S or R)-3-amino-3-(4-methoxyphenyl)propionic acid (isolation yield based on n-propyl 3-amino-3-(4-methoxyphenyl)propionate (racemic mixtures)=44.0%) and 12 mg of an enzyme fixing agent as a mixture. Then, liquids of the filterate were separated, and the organic phase was extracted and dried over anhydrous magnesium sulfate, and after filtration, the filtrate was concentrated to give 135 mg of (R or S)-n-propyl 3-amino-3-(4-methoxyphenyl)propionate (isolation yield based on n-propyl 3-amino-3-(4-methoxyphenyl)propionate (racemic mixtures)=45.0%).

The (S or R)-3-amino-3-(4-methoxyphenyl)propionic acid was led to an n-propyl ester according to the conventional manner, and when an optical purity thereof was measured by using high performance liquid chromatography which uses an optically active column, it was 99.7%ee.

When an optical purity of the (R or S)-n-propyl 3-amino-3-(4-methoxyphenyl)propionate was measured by using high performance liquid chromatography which uses an optically active column, it was 98.2%ee.

Incidentally, the E value in the present reaction was 3549.
Analytical conditions of high performance liquid chromatography;
Optically active n-propyl 3-amino-3-(4-methoxyphenyl)-propionate
Column: Chiral CD-Ph (0.46cmΦx25cm, available from Shiseido Co., Ltd.)
Solvent: acetonitrile/water (=1/9 (volume ratio))
Potassium dihydrogen phosphate 40 mmol/L
Adjusted to pH 3.5 with phosphoric acid
Flow rate: 0.5 mL/min
Temperatrure: 30°C
Wavelength: 220 nm

Also, physical properties of the (S or R)-3-amino-3-(4-methoxyphenyl)propionic acid were as follows.
¹H-NMR (δ (ppm), D₂O) : 2.50 (dd, 1H, J=17.1, 6.8Hz), 2.65 (dd, 1H, J=17.1, 7.8Hz), 3.24 (s, 3H), 6.46 (s, 1H), 6.48 (s, 1H), 6.86 (s, 1H), 6.88 (s, 1H)
¹³C-NMR (δ (ppm), D₂O) : 37.1, 50.7, 55.1, 114.3, 126.9, 128.2, 159.1, 172.7
MS (EI) m/z: 195 (M⁺)
MS (CI, i-C₄H₁₀) m/z: 196 (MH⁺)

Physical properties of the (R or S)-n-propyl 3-amino-3-(4-methoxyphenyl)propionate were as follows.
¹H-NMR (δ (ppm), CDCl₃) : 0.91 (t, 3H, J=7.3Hz), 1.62 (qt, 2H, 7.3, 6.8Hz), 1.84 (br, 2H), 2.64 (d, 2H, J=7.3Hz), 3.79 (s, 3H), 4.03 (t, 2H, 6.8Hz), 4.38 (t, 1H, d=7.3Hz), 6.85 (s, 1H), 6.88 (s, 1H), 7.27 (s, 1H), 7.29 (s, 1H)
¹³C-NMR (δ (ppm), CDCl₃) : 10.4, 22.0, 44.3, 52.1, 55.3, 66.1, 114.0, 127.3, 136.8, 158.9, 172.2
MS (EI) m/z: 237 (M⁺)
MS (CI, i-C₄H₁₀) m/z: 238 (MH⁺)

### Comparative example 11 (Syntheses of (S or R)-3-amino-3-(4-methoxyphenyl)propionic acid and (R or S)-ethyl 3-amino-3-(4-methoxyphenyl)propionate)

To a mixture comprising 0.75 mL of 50 mmol/L aqueous potassium phosphate solution with a pH of 8.2 and 0.75 mL of t-butyl methyl ether was added 300 mg (1.3 mmol) of ethyl 3-amino-3-(4-methoxyphenyl)propionate (racemic mixtures), and the mixture was maintained to 30°C, 15 mg of lipase (Amano Lipase PS (trade name); available from Aldrich Corporation) originated from *Burkholderia cepacia* (*Pseudomonas cepacia*) was added to the mixture at the same temperature, and the mixture was reacted while stirring at 30°C. After 8 hours, 1.0 mL of acetone was added to the reaction mixture and the resulting mixture was filtered to give 106 mg of (S or R)-3-amino-3-(4-methoxyphenyl)propionic acid (isolation yield based on ethyl 3-amino-3-(4-methoxyphenyl)propionate (racemic mixtures)=43.0%) and 13 mg of an enzyme fixing agent as a mixture. Then, liquids of the filterate were separated, and the organic phase was extracted and dried over anhydrous magnesium sulfate, and after filtration, the filtrate was concentrated to give 132 mg of (R or S)-ethyl 3-amino-3-(4-methoxyphenyl)propionate (isolation yield based on ethyl 3-amino-3-(4-methoxyphenyl)propionate (racemic mixtures)=44.0%).

The (S or R)-3-amino-3-(4-methoxyphenyl)propionic acid was led to an n-propyl ester according to the conventional manner, and when an optical purity thereof was measured by using high performance liquid chromatography which uses an optically active column, it was 98.2%ee.

When an optical purity of the (R or S)-ethyl 3-amino-3-(4-methoxyphenyl)propionate was measured by using high performance liquid chromatography which uses an optically active column, it was 97.4%ee.

Incidentally, the E value in the present reaction was 488.
Analytical conditions of high performance liquid chromatography;
Optically active n-propyl 3-amino-3-(4-methoxyphenyl)-propionate
Optically active ethyl 3-amino-3-(4-methoxyphenyl)propionate
Column: Chiral CD-Ph (0.46cmΦ×25cm, available from Shiseido Co., Ltd.)
Solvent: acetonitrile/water (=1/9 (volume ratio))
Potassium dihydrogen phosphate 40 mmol/L
Adjusted to pH 3.5 with phosphoric acid
Flow rate: 0.5 mL/min
Temperatrure: 30°C
Wavelength: 220 nm

Also, physical properties of the (S or R)-3-amino-3-(4-methoxyphenyl)propionic acid were the same as those shown in Example 26.

Physical properties of the (R or S)-ethyl 3-amino-3-(4-methoxyphenyl)propionate were as shown below.
¹H-NMR (δ (ppm), CDCl₃) : 1.24 (t, 3H, J=7.3Hz), 1.71 (br, 2H), 2.63 (d, 2H, J=6.8Hz), 3.80 (s, 3H), 4.14 (q, 2H, 7.3Hz), 4.38 (t, 1H, d=6.8Hz), 6.86 (s, 1H), 6.88 (s, 1H), 7.27 (s, 1H), 7.29 (s, 1H)
¹³C-NMR (δ (ppm), CDCl₃): 41.3, 54.6, 104.6, 110.3, 111.9, 124.3, 132.0, 150.8, 150.9, 177.3
MS (EI) m/z: 223 (M⁺)
MS (CI, i-C₄H₁₀) m/z: 224 (MH⁺)

### Example 27 (Synthesis of 3-amino-3-(3,4-methylenedioxyphenyl)propionic acid (racemic mixtures))

To 100 mL of ethanol were added 20.0 g (0.13 mol) of piperonal, 13.9 g (0.13 mol) of malonic acid and 32.3 g (0.26 mol) of ammonium acetate, and the mixture was reacted while stirring under reflux (75 to 80°C) for 15 hours. After completion of the reaction, the obtained reaction mixture was stirred at room temperature for 20 hours, and then, filtered at room temperature to give 19.5 g of 3-amino-3-(3,4-methylenedioxyphenyl)propionic acid (racemic mixtures) (isolation yield based on piperonal: 70.0%) as white powder.

Incidentally, physical properties of the 3-amino-3-(3,4-methylenedioxyphenyl)propionic acid (racemic mixtures) were as mentioned below.
¹H-NMR (δ (ppm), D₂O+DCl) : 2.99 (dd, 2H, J=16.7, 6.8Hz), 3.11 (dd, 1H, J=16.7, 8.1Hz), 4.69 (dd, 1H, J=8.1, 6.8Hz), 6.92-7.00 (m, 3H)
¹³C-NMR (δ (ppm), D₂O+DCl) : 41.3, 54.6, 104.6, 110. 3, 111. 9, 124.3, 132.0, 150.8, 150.9, 177.3
MS (EI) m/z: 209 (M⁺)
MS (CI, i-C₄H₁₀) m/z: 210 (MH⁺)

### Example 28 (Synthesis of n-propyl 3-amino-3-(3,4-methylenedioxyphenyl)propionate (racemic mixtures))

To 35.0 mL (468 mmol) of n-propyl alcohol were added 7.00 g (33.5 mmol) of 3-amino-3-(3,4-methylenedioxyphenyl)-propionic acid (racemic mixtures) synthesized in Example 27 and 6.60 g (67.0 mmol) of conc. sulfuric acid, and the mixture was reacted while stirring at 55 to 60°C for 8 hours. After completion of the reaction, 14 mL of water was added to the reaction mixture, a pH of the mixture was adjusted to 7.5 with 6 mol/L sodium hydroxide and the mixture was concentrated under reduced pressure. To the obtained concentrated residue was added 21 mL of cyclohexane, and a pH of the mixture was adjusted to 10.1 by using 6 mol/L aqueous sodium hydroxide solution. The obtained mixture was allowed to stand, and the extracted organic phase was dried over anhydrous magnesium sulfate. After filtration, the filtrate was concentrated under reduced pressure to give 7.6 g of n-propyl 3-amino-3-(3,4-methylenedioxyphenyl)propionate (racemic mixtures) (isolation yield based on 3-amino-3-(3,4-methylenedioxyphenyl)-propionic acid (racemic mixtures): 90.0%) as colorless liquid.

Incidentally, physical properties of the n-propyl 3-amino-3-(3,4-methylenedioxyphenyl)propionate (racemic mixtures) were as follows.
¹H-NMR (δ (ppm), CDCl₃): 0.91 (t, 3H, J=7.3Hz), 1.61 (qt, 2H, 7.3, 6.8Hz), 1.71 (br, 2H), 2.60 (d, 2H, J=6.8Hz), 4.03 (t, 2H, 6.8Hz), 4.33 (t, 2H, 6.8Hz), 6.72-6.87 (m, 3H)
MS (EI) m/z: 251 (M⁺)
MS (CI, i-C₄H₁₀) m/z: 252 (MH⁺)

### Example 29 (Syntheses of (S or R)-3-amino-3-(3,4-methylenedioxyphenyl)propionic acid and (R or S)-n-propyl 3-amino-3-(3,4-methylenedioxyphenyl)propionate)

To a mixture comprising 2.5 mL of 50 mmol/L aqueous potassium phosphate solution with a pH of 8.2 and 2.5 mL of cyclohexane was added 1.00 g (3.98 mmol) of 3-amino-3-(3,4-methylenedioxyphenyl)propionic acid n-propyl ester (racemic mixtures) synthesized in Example 28, and the mixture was maintained to 30°C, 50 mg of lipase (Amano Lipase PS (trade name); available from Aldrich Corporation) originated from *Burkholderia cepacia* (*Pseudomonas cepacia*) was added to the mixture at the same temperature, and the mixture was reacted while stirring at 30°C. After 5 hours, 3 mL of acetone was added to the reaction mixture and the resulting mixture was filtered to give 350 mg of (S or R)-3-amino-3-(3,4-methylenedioxyphenyl)propionic acid (isolation yield based on n-propyl 3-amino-3-(3,4-methylenedioxyphenyl)-propionate (racemic mixtures)=42.0%) and 5 mg of an enzyme fixing agent as a mixture. Then, liquids of the filtrate were separated, the organic phase was extracted and dried over anhydrous magnesium sulfate, and after filtration, the mixture was concentrated under reduced pressure to give 450 mg of (R or S)-n-propyl 3-amino-3-(3,4-methylenedioxyphenyl)propionate (isolation yield based on n-propyl 3-amino-3-(3,4-methylenedioxyphenyl)propionate (racemic mixtures)=45.0%).

The (S or R)-3-amino-3-(3,4-methylenedioxyphenyl)-propionic acid was led to an n-propyl ester according to the conventional manner, and when an optical purity thereof was measured by using high performance liquid chromatography which uses an optically active column, it was 99.3%ee.

When an optical purity of the (R or S)-n-propyl 3-amino-3-(3,4-methylenedioxyphenyl)propionate was measured by using high performance liquid chromatography which uses an optically active column, it was 79.2%ee.

Incidentally, the E value in the present reaction was 752.
Analytical conditions of high performance liquid chromatography;
Optically active n-propyl 3-amino-3-(3,4-methylenedioxyphenyl)propionate
Column: Chiral CD-Ph (0.46cmΦx25cm, available from Shiseido Co., Ltd.)
Solvent: acetonitrile/water (=1/9 (volume ratio))
Potassium dihydrogen phosphate 40 mmol/L
Adjusted to pH 3.5 with phosphoric acid
Flow rate: 1.0 mL/min
Temperatrure: 30°C
Wavelength: 220 nm

Also, physical properties of the (S or R)-3-amino-3-(3,4-methylenedioxyphenyl)propionic acid were as follows. ¹H-NMR (δ (ppm), D₂O+DCl): 2.99 (dd, 2H, J=16.7, 6.8Hz), 3.11 (dd, 1H, J=16.7, 8.1Hz), 4.69 (dd, 1H, J=8.1, 6.8Hz), 6.92-7.00 (m, 3H)
¹³C-NMR (δ (ppm), D₂O+DCl) : 41.3, 54.6, 104.6, 110.3, 111.9, 124.3, 132.0, 150.8, 150.9, 177.3
MS (EI) m/z: 209 (M⁺)
MS (CI, i-C₄H₁₀) m/z: 210 (MH⁺)

Physical properties of the (R or S)-n-propyl 3-amino-3-(3,4-methylenedioxyphenyl)propionate were as follows.
¹H-NMR (δ (ppm), CDCl₃) : 0.91 (t, 3H, J=7.3Hz), 1.61 (qt, 2H, 7.3, 6.8Hz), 1.71 (br, 2H), 2.60 (d, 2H, J=6.8Hz), 4.03 (t, 2H, 6.8Hz), 4.33 (t, 2H, 6.8Hz), 6.72-6.87 (m, 3H)
MS (EI) m/z: 251 (M⁺)
MS (CI, i-C₄H₁₀) m/z: 252 (MH⁺)

### Comparative example 12 (Syntheses of (S or R)-3-amino-3-(3,4-methylenedioxyphenyl)propionic acid and (R or S)-ethyl 3-amino-3-(3,4-methylenedioxyphenyl)propionate)

To a mixture comprising 0.75 mL of 50 mmol/L aqueous potassium phosphate solution with a pH of 8.2 and 0.75 mL of t-butyl methyl ether was added 300 mg (1.3 mmol) of ethyl 3-amino-3-(3,4-methylenedioxyphenyl)propionate (racemic mixtures), and the mixture was maintained to 30°C, 15 mg of lipase (Amano Lipase PS (trade name); available from Aldrich Corporation) originated from *Burkholderia cepacia* (*Pseudomonas cepacia*) was added to the mixture at the same temperature, and the mixture was reacted while stirring at 30°C. After 15 hours, 1.0 mL of acetone was added to the reaction mixture and the resulting mixture was filtered to give 111 mg of (S or R)-3-amino-3-(3,4-methylenedioxyphenyl)propionic acid (isolation yield based on ethyl 3-amino-3-(3,4-methylenedioxyphenyl)propionate (racemic mixtures)=42.0%) and 8 mg of an enzyme fixing agent as a mixture. Then, liquids of the filterate were separated, and the organic phase was extracted and dried over anhydrous magnesium sulfate, and after filtration, the filtrate was concentrated to give 132 mg of (R or S)-ethyl 3-amino-3-(3,4-methylenedioxyphenyl)propionate (isolation yield based on ethyl 3-amino-3-(3,4-methylenedioxyphenyl)-propionate (racemic mixtures)=44.0%).

The (S or R)-3-amino-3-(3,4-methylenedioxyphenyl)-propionic acid was led to an n-propyl ester according to the conventional manner, and when an optical purity thereof was measured by using high performance liquid chromatography which uses an optically active column, it was 82.6%ee.

When an optical purity of the (R or S)-ethyl 3-amino-3-(3,4-methylenedioxyphenyl)propionate was measured by using high performance liquid chromatography which uses an optically active column, it was 93.8%ee.

Incidentally, the E value in the present reaction was 36.
Analytical conditions of high performance liquid chromatography;
Optically active n-propyl 3-amino-3-(3,4-methylenedioxyphenyl)propionate
Optically active ethyl 3-amino-3-(3,4-methylenedioxyphenyl)propionate
Column: Chiral CD-Ph (0.46cmΦ×25cm, available from Shiseido Co., Ltd.)
Solvent: acetonitrile/water (=1/9 (volume ratio))
Potassium dihydrogen phosphate 40 mmol/L
Adjusted to pH 3.5 with phosphoric acid
Flow rate: 1.0 mL/min
Temperatrure: 30°C
Wavelength: 220 nm

Also, physical properties of the (S or R)-3-amino-3-(3,4-methylenedioxyphenyl)propionic acid were the same as those shown in Example 29.

Physical properties of the (R or S)-ethyl 3-amino-3-(3,4-methylenedioxyphenyl)propionate were as follows.
¹H-NMR (δ (ppm), CDCl₃) : 1.22 (t, 3H, J=7.1Hz), 1.73 (br, 2H), 2.58 (d, 2H, J=6.8Hz), 4.11 (q, 2H, 7.1Hz), 4.31 (t, 2H, 6.8Hz), 6.7-6.87 (m, 3H)
MS (EI) m/z: 237 (M⁺)
MS (CI, i-C₄H₁₀) m/z: 238 (MH⁺)

### Comparative example 13 (Syntheses of (S or R)-3-amino-3-(4-tolyl)propionic acid and (R or S)-n-propyl 3-amino-3-(4-tolyl)propionate)

To a mixture comprising 500 mg (2.22 mmol) of n-propyl 3-amino-3-(4-tolyl)propionate (racemic mixtures) synthesized in Example 2 and 1.25 mL of cyclohexane was added 1.25 mL of water adjusted to pH 8.2 with 1 mol/L aqueous potassium hydroxide solution, and the mixture was maintained to 30°C. To the obtained mixture was added 25 mg of lipase (Amano Lipase PS (trade name); available from Aldrich Corporation) originated from *Burkholderia cepacia* (*Pseudomonas cepacia*) at the same temperature, and the mixture was reacted while stirring at 30°C. After 14 hours, a reaction conversion reached 49.9%.

When an optical purity of the (S or R)-3-amino-3-(4-tolyl)propionic acid was measured by using high performance liquid chromatography which uses an optically active column, it was 98.8%ee.

The (R or S)-n-propyl 3-amino-3-(4-tolyl)propionate was led to (R or S)-n-propyl 3-(2-furoylamino)-3-(4-tolyl)-propionate, and when an optical purity thereof was measured by using high performance liquid chromatography which uses an optically active column, it was 98.5%ee.

Incidentally, the E value in the present reaction was 837.
Analytical conditions of high performance liquid chromatography;
Optically active 3-amino-3-(4-tolyl)propionic acid
Column: Chiral CD-Ph (0.46cmΦx25cmx2 columns connected, available from Shiseido Co., Ltd.)
Solvent: acetonitrile/water (=5/95 (volume ratio))
Potassium dihydrogen phosphate 40 mmol/L
Adjusted to pH 3.5 with phosphoric acid
Flow rate: 0.5 mL/min
Temperatrure: 30°C
Wavelength: 220 nm
Optically active n-propyl 3-(2-furoylamino)-3-(4-tolyl)-propionate
Column: Chiralcel OJ-H (0.46cmΦx25cm, available from
DAICEL CHEMICAL INDUSTRIES, LTD.)
Solvent: hexane/isopropyl alcohol (=1/9 (volume ratio)) Flow rate: 0.5 mL/min
Temperatrure: 30°C
Wavelength: 220 nm

Also, physical properties of the (S or R)-3-amino-3-(4-tolyl)propionic acid and (R or S)-n-propyl 3-amino-3-(4-tolyl)propionate were the same as those shown in Comparative example 5.

### Comparative example 14 (Syntheses of (S or R)-3-amino-3-(3-fluorophenyl)propionic acid and (R or S)-n-propyl 3-amino-3-(3-fluorophenyl)propionate)

To a mixture comprising 500 mg (2.22 mmol) of n-propyl 3-amino-3-(3-fluorophenyl)propionate (racemic mixtures) synthesized in Example 22 and 1.25 mL of cyclohexane was added 1.25 mL of water a pH of which had been adjusted to 8.2 with 1 mol/L aqueous potassium hydroxide solution, and the mixture was maintained to 30°C. To the obtained mixture was added 25 mg of lipase (Amano Lipase PS (trade name); available from Aldrich Corporation) originated from *Burkholderia cepacia* (*Pseudomonas cepacia*) at the same temperature, and the mixture was reacted while stirring at 30°C. After 14 hours, a reaction conversion reached 50.0%.

When an optical purity of the (S or R)-3-amino-3-(3-fluorophenyl)propionic acid was measured by using high performance liquid chromatography which uses an optically active column, it was 99.0%ee.

The (R or S)-n-propyl 3-amino-3-(3-fluorophenyl)-propionate was led to (R or S)-n-propyl 3-(4-toluoylamino)-3-(3-fluorophenyl)propionate, and when an optical purity thereof was measured by using high performance liquid chromatography which uses an optically active column, it was 98.8%ee.

Incidentally, the E value in the present reaction was 980.
Analytical conditions of high performance liquid chromatography;
Optically active 3-amino-3-(3-fluorophenyl)propionic acid Column: Chiral CD-Ph (0.46cmΦ×25cmx2 columns connected, available from Shiseido Co., Ltd.)
Solvent: acetonitrile/water (=5/95 (volume ratio))
Potassium dihydrogen phosphate 40 mmol/L
Adjusted to pH 3.5 with phosphoric acid
Flow rate: 0.5 mL/min
Temperatrure: 30°C
Wavelength: 220 nm
Optically active n-propyl 3-(4-toluoylamino)-3-(3-fluorophenyl)propionate
Column: Chiralcel OJ-H (0.46cmΦx25cm, available from DAICEL CHEMICAL INDUSTRIES, LTD.)
Solvent: hexane/isopropyl alcohol (=5/95 (volume ratio)) Flow rate: 0.5 mL/min
Temperatrure: 30°C
Wavelength: 220 nm

Also, physical properties of the (S or R)-3-amino-3-(3-fluorophenyl)propionic acid and (R or S)-n-propyl 3-amino-3-(3-fluorophenyl)propionate were the same as those shown in Example 23.

### Comparative example 15 (Syntheses of (S or R)-3-amino-3-(3-fluorophenyl)propionic acid and (R or S)-n-propyl 3-amino-3-(3-fluorophenyl)propionate)

To a mixture comprising 500 mg (2.22 mmol) of n-propyl 3-amino-3-(3-fluorophenyl)propionate (racemic mixtures) synthesized in Example 22 and 1.25 mL of cyclohexane was added 1.25 mL of water a pH of which had been adjusted to 8.2 with 1 mol/L aqueous potassium hydroxide solution, and the mixture was maintained to 30°C. To the obtained mixture was added 15 mg of lipase (Amano Lipase PS (trade name); available from Aldrich Corporation) originated from *Burkholderia cepacia (Pseudomonas cepacia)* at the same temperature, and the mixture was reacted while stirring at 30°C. After 52 hours, a reaction conversion reached 49.2%.

When an optical purity of the (S or R)-3-amino-3-(3-fluorophenyl)propionic acid was measured by using high performance liquid chromatography which uses an optically active column, it was 96.0%ee.

The (R or S)-n-propyl 3-amino-3-(3-fluorophenyl)propionate was led to (R or S)-n-propyl 3-(4-toluoylamino)-3-(3-fluorophenyl)propionate, and when an optical purity thereof was measured by using high performance liquid chromatography which uses an optically active column, it was 92.8%ee.

Incidentally, the E value in the present reaction was 167.
Analytical conditions of high performance liquid chromatography;
Optically active 3-amino-3-(3-fluorophenyl)propionic acid Column: Chiral CD-Ph (0.46cmΦx25cmx2 columns connected, available from Shiseido Co., Ltd.)
Solvent: acetonitrile/water (=5/95 (volume ratio))
Potassium dihydrogen phosphate 40 mmol/L
Adjusted to pH 3.5 with phosphoric acid
Flow rate: 0.5 mL/min
Temperatrure: 30°C
Wavelength: 220 nm
Optically active n-propyl 3-(4-toluoylamino)-3-(3-fluorophenyl)propionate
Column: Chiralcel OJ-H (0.46cmΦx25cm, available from DAICEL CHEMICAL INDUSTRIES, LTD.)
Solvent: hexane/isopropyl alcohol (=5/95 (volume ratio)) Flow rate: 0.5 mL/min
Temperatrure: 30°C
Wavelength: 220 nm

Also, physical properties of the (S or R)-3-amino-3-(3-fluorophenyl)propionic acid and (R or S)-n-propyl 3-amino-3-(3-fluorophenyl)propionate were the same as those shown in Example 23.

### Example 30 (Synthesis of n-butyl 3-amino-3-(3-fluorophenyl)propionate (racemic mixtures))

To 40.0 mL (435 mmol) of n-butyl alcohol were added 8.00 g (43.7 mmol) of 3-amino-3-(3-fluorophenyl)propionic acid (racemic mixtures) synthesized in Example 21 and 6.40 g (65.5 mmol) of conc. sulfuric acid, and the mixture was reacted while stirring at 55 to 60°C for 3 hours. After completion of the reaction, 16 mL of water was added to the reaction mixture, a pH of the mixture was adjusted to 7.0 with 6 mol/L sodium hydroxide and the mixture was concentrated under reduced pressure. To the obtained concentrated residue was added 40 mL of cyclohexane, and a pH of the mixture was adjusted to 8.5 by using 6 mol/L aqueous sodium hydroxide solution. The obtained mixture was allowed to stand, liquids were separated and the extracted organic phase was dried over anhydrous magnesium sulfate. After filtration, the filtrate was concentrated under reduced pressure to give 9.40 g of n-butyl 3-amino-3-(3-fluorophenyl)propionate (racemic mixtures) (isolation yield based on 3-amino-3-(3-fluorophenyl)propionic acid (racemic mixtures): 90%) as colorless liquid.

Incidentally, physical properties of the n-butyl 3-amino-3-(3-fluorophenyl)propionate (racemic mixtures) were as follows.
¹H-NMR (δ (ppm), CDCl₃) : 0.91 (t, 3H, J=7.3Hz), 1.29-1.38 (m, 2H), 1.54-1.61 (m, 2H), 1.72 (s, 2H), 2.57-2.66 (m, 2H), 4.08 (t, 2H, 6.6Hz), 4.41 (dd, 2H, J=5.9,7.8Hz), 6.90-6.95 (m, 2H), 7.09-7.14 (m, 2H), 7.25-7.30 (m, 2H)
¹³C-NMR (δ (ppm), CDCl₃): 13.68, 19.13, 30.66, 44.15, 52.34, 64.48, 113.71, 121.93, 130.09, 147.57, 161.81, 164.28, 171.77
MS (EI) m/z: 239 (M⁺)
MS (CI, i-C₄H₁₀) m/z: 240 (MH⁺)

### Reference example 4 (Synthesis of isopropyl 3-amino-3-(3-fluorophenyl)phenylpropionate (racemic mixtures))

To 40.0 mL (520 mmol) of isopropyl alcohol were added 8.00 g (43.7 mmol) of 3-amino-3-(3-fluorophenyl)propionic acid (racemic mixtures) synthesized in Example 21 and 6.40 g (65.5 mmol) of conc. sulfuric acid, and the mixture was reacted while stirring at 55 to 60°C for 4 hours. After completion of the reaction, 16 mL of water was added to the reaction mixture, a pH of the mixture was adjusted to 7.0 with 6 mol/L sodium hydroxide and the mixture was concentrated under reduced pressure. To the obtained concentrated residue was added 40 mL of cyclohexane, and a pH of the mixture was adjusted to 8.5 by using 6 mol/L aqueous sodium hydroxide solution. The obtained mixture was allowed to stand, liquids were separated and the extracted organic phase was dried over anhydrous magnesium sulfate. After filtration, the filtrate was concentrated under reduced pressure to give 8.90 g of isopropyl 3-amino-3-(3-fluorophenyl)propionate (racemic mixtures) (isolation yield based on 3-amino-3-(3-fluorophenyl)propionic acid (racemic mixtures): 90%) as colorless liquid.

Incidentally, physical properties of the isopropyl 3-amino-3-(3-fluorophenyl)propionate (racemic mixtures) were as follows.
¹H-NMR (δ (ppm), CDCl₃) : 1.19 (d, 3H, J=6.4Hz), 1.21 (d, 3H, J=6.4Hz), 0.91 (t, 3H, J=7.3Hz), 1.72 (s, 2H), 2.55-2.63 (m, 2H), 4.08 (t, 2H, 6.6Hz), 4.41 (dd, 2H, J=5.9, 7.8Hz), 5.01 (qq, 1H, J=6.4,6.4Hz), 6.90-6.96 (m, 2H),
7.08-7.14 (m, 2H), 7.25-7.30 (m, 2H)
¹³C-NMR (δ (ppm), CDCl₃): 21.77, 21.80, 44.43, 44.15, 52.35, 68.00, 113.71, 121.95, 130.05, 147.51, 161.77, 164.25, 171.19
MS (EI) m/z: 225 (M⁺)
MS (CI, i-C₄H₁₀) m/z: 226 (MH⁺)

### Example 31 (Syntheses of (S or R)-3-amino-3-(3-fluorophenyl)propionic acid and (R or S)-n-butyl 3-amino-3-(3-fluorophenyl)propionate)

To a mixture comprising 500 mg (2.22 mmol) of n-butyl 3-amino-3-(3-fluorophenyl)propionate (racemic mixtures) synthesized in Example 30 and 1.25 mL of cyclohexane was added 1.25 mL of 50 mmol/L aqueous potassium phosphate solution with a pH of 8.2, and the mixture was maintained to 30°C. To the obtained mixture was added 25 mg of lipase (Amano Lipase PS (trade name); available from Aldrich Corporation) originated from *Burkholderia cepacia* (*Pseudomonas cepacia*) at the same temperature, and the mixture was reacted while stirring at 30°C. After 88 hours, a reaction conversion reached 49.1%.

When an optical purity of the (S or R)-3-amino-3-(3-fluorophenyl)propionic acid was measured by using high performance liquid chromatography which uses an optically active column, it was 99.1%ee.

The (R or S)-n-butyl 3-amino-3-(3-fluorophenyl)-propionate was led to (R or S)-n-butyl 3-(4-toluoylamino)-3-(3-fluorophenyl)propionate, and when an optical purity thereof was measured by using high performance liquid chromatography which uses an optically active column, it was 95.5%ee.

Incidentally, the E value in the present reaction was 842.
Analytical conditions of high performance liquid chromatography;
Optically active 3-amino-3-(3-fluorophenyl)propionic acid Column: Chiral CD-Ph (0.46cmΦx25cmx2 columns connected, available from Shiseido Co., Ltd.)
Solvent: acetonitrile/water (=5/95 (volume ratio))
Potassium dihydrogen phosphate 40 mmol/L
Adjusted to pH 3.5 with phosphoric acid
Flow rate: 0.5 mL/min
Temperatrure: 30°C
Wavelength: 220 nm
Optically active n-butyl 3-(4-toluoylamino)-3-(3-fluorophenyl)propionate
Column: Chiralcel OJ-H (0.46cmΦx25cm, available from DAICEL CHEMICAL INDUSTRIES, LTD.)
Solvent: hexane/isopropyl alcohol (=2/98 (volume ratio)) Flow rate: 0.5 mL/min
Temperatrure: 30°C
Wavelength: 220 nm

Also, physical properties of the (S or R)-3-amino-3-(3-fluorophenyl)propionic acid were the same as those shown in Example 23.

Physical properties of the (R or S)-n-butyl 3-amino-3-(3-fluorophenyl)propionate were as follows.
¹H-NMR (δ (ppm), CDCl₃) : 0.91 (t, 3H, J=7.3Hz), 1.29-1.38 (m, 2H), 1.54-1.61 (m, 2H), 1.72 (s, 2H), 2.57-2.66 (m, 2H), 4.08 (t, 2H, 6.6Hz), 4.41 (dd, 2H, J=5.9,7.8Hz), 6.90-6.95 (m, 2H), 7.09-7.14 (m, 2H), 7.25-7.30 (m, 2H)
¹³C-NMR (δ (ppm), CDCl₃): 13.68, 19.13, 30.66, 44.15, 52.34, 64.48, 113.71, 121.93, 130.09, 147.57, 161.81, 164.28, 171.77
MS (EI) m/z: 239 (M⁺)
MS (CI, i-C₄H₁₀) m/z: 240 (MH⁺)

### Comparative example 16 (Syntheses of (S or R)-3-amino-3-(3-fluorophenyl)propionic acid and isopropyl (R or S)-3-amino-3-(3-fluorophenyl)propionate)

To a mixture comprising 500 mg (2.22 mmol) of isopropyl 3-amino-3-(3-fluorophenyl)propionate (racemic mixtures) synthesized in Reference example 4 and 1.25 mL of cyclohexane was added 1.25 mL of 50 mmol/L aqueous potassium phosphate solution with a pH of 8.2, and the mixture was maintained to 30°C. To the obtained mixture was added 25 mg of lipase (Amano Lipase PS (trade name); available from Aldrich Corporation) originated from *Burkholderia cepacia* (*Pseudomonas cepacia*) at the same temperature, and the mixture was reacted while stirring at 30°C. After 88 hours, a reaction conversion reached 18.1%.

When an optical purity of the (S or R)-3-amino-3-(3-fluorophenyl)propionic acid was measured by using high performance liquid chromatography which uses an optically active column, it was 98.1%ee.

The isopropyl (R or S)-3-amino-3-(3-fluorophenyl)-propionate was led to isopropyl (R or S)-3-(4-toluoyl-amino)-3-(3-fluorophenyl)propionate, and when an optical purity thereof was measured by using high performance liquid chromatography which uses an optically active column, it was 21.7%ee.

Incidentally, the E value in the present reaction was 128.
Analytical conditions of high performance liquid chromatography;
Optically active 3-amino-3-(3-fluorophenyl)propionic acid Column: Chiral CD-Ph (0.46cmΦx25cmx2 columns connected, available from Shiseido Co., Ltd.)
Solvent: acetonitrile/water (=5/95 (volume ratio))
Potassium dihydrogen phosphate 40 mmol/L
Adjusted to pH 3.5 with phosphoric acid
Flow rate: 0.5 mL/min
Temperatrure: 30°C
Wavelength: 220 nm
Optically active isopropyl 3-(4-toluoylamino)-3-(3-fluorophenyl)propionate
Column: Chiralcel OJ-H (0.46cmΦx25cm, available from DAICEL CHEMICAL INDUSTRIES, LTD.)
Solvent: hexane/isopropyl alcohol (=2/98 (volume ratio)) Flow rate: 0.5 mL/min
Temperatrure: 30°C
Wavelength: 220 nm

Also, physical properties of the (S or R)-3-amino-3-(3-fluorophenyl)propionic acid were the same as those shown in Example 23.

Physical properties of the isopropyl (R or S)-3-amino-3-(3-fluorophenyl)propionate were as follows.
¹H-NMR (δ (ppm), CDCl₃) : 1.19 (d, 3H, J=6.4Hz), 1.21 (d, 3H, J=6.4Hz), 0.91 (t, 3H, J=7.3Hz), 1.72 (s, 2H), 2.55-2.63 (m, 2H), 4.08 (t, 2H, 6.6Hz), 4.41 (dd, 2H, J=5.9, 7.8Hz), 5.01 (qq, 1H, J=6.4,6.4Hz), 6.90-6.96 (m, 2H),
7.08-7.14 (m, 2H), 7.25-7.30 (m, 2H)
¹³C-NMR (δ (ppm), CDCl₃): 21.77, 21.80, 44.43, 44.15, 52.35, 68.00, 113.71, 121.95, 130.05, 147.51, 161.77, 164.25, 171.19
MS (EI) m/z: 225 (M⁺)
MS (CI, i-C₄H₁₀) m/z: 226 (MH⁺)

### Reference example 5 (Synthesis of s-butyl 3-amino-3-(3-fluorophenyl)propionate)

To 60.0 mL (649 mmol) of s-butyl alcohol were added 6.00 g (32.8 mmol) of 3-amino-3-(3-fluorophenyl)propionic acid (racemic mixtures) synthesized in Example 21 and 4.80 g (49.1 mmol) of conc. sulfuric acid, and the mixture was reacted while stirring at 55 to 60°C for 3 hours. After completion of the reaction, 12 mL of water was added to the reaction mixture, a pH of the mixture was adjusted to 7.0 with 6 mol/L sodium hydroxide and the mixture was concentrated under reduced pressure. To the obtained concentrated residue was added 30 mL of cyclohexane, and a pH of the mixture was adjusted to 8.5 by using 6 mol/L aqueous sodium hydroxide solution. The obtained mixture was allowed to stand, liquids were separated and the extracted organic phase was dried over anhydrous magnesium sulfate. After filtration, the filtrate was concentrated under reduced pressure to give 5.50 g of s-butyl 3-amino-3-(3-fluorophenyl)propionate (isolation yield based on 3-amino-3-(3-fluorophenyl)propionic acid (racemic mixtures): 70%) as colorless liquid.

Incidentally, physical properties of the s-butyl 3-amino-3-(3-fluorophenyl)propionate were as follows. "Diastereomer mixture"
¹H-NMR (δ (ppm), CDCl₃): 0.83-0.89 (m, 3H), 1.16-1.65 (m, 3H), 1.46-1.65 (m,1H), 1.71 (s, 2H), 2.58-2.68 (m, 2H), 4.42 (dd, 1H, J=6.4,13.7Hz), 4.82-4.99 (m, 1H), 6.97-7.33 (m, 1H), 7.09-7.15 (m, 1H), 7.23-7.33 (m, 1H)
MS (EI) m/z: 239 (M⁺)
MS (CI, i-C₄H₁₀) m/z: 240 (MH⁺)

### Comparative example 17 (Syntheses of (S or R)-3-amino-3-(3-fluorophenyl)propionic acid and s-butyl (3R or 3S)-3-amino-3-(3-fluorophenyl)propionate)

To a mixture comprising 500 mg (2.09 mmol) of s-butyl 3-amino-3-(3-fluorophenyl)propionate synthesized in Reference example 5 and 1.25 mL of cyclohexane was added 1.25 mL of 50 mmol/L aqueous potassium phosphate solution with a pH of 8.2, and the mixture was maintained to 30°C. To the obtained mixture was added 25 mg of lipase (Amano Lipase PS (trade name); available from Aldrich Corporation) originated from *Burkholderia cepacia (Pseudomonas cepacia)* at the same temperature, and the mixture was reacted while stirring at 30°C. After 60 hours, a reaction conversion reached 21.7%.

When an optical purity of the (S or R)-3-amino-3-(3-fluorophenyl)propionic acid was measured by using high performance liquid chromatography which uses an optically active column, it was 99.0%ee.
Analytical conditions of high performance liquid chromatography;
Optically active 3-amino-3-(3-fluorophenyl)propionic acid Column: Chiral CD-Ph (0.46cmΦx25cmx2 columns connected, available from Shiseido Co., Ltd.)
Solvent: acetonitrile/water (=5/95 (volume ratio))
Potassium dihydrogen phosphate 40 mmol/L
Adjusted to pH 3.5 with phosphoric acid
Flow rate: 0.5 mL/min
Temperatrure: 30°C
Wavelength: 220 nm

Also, physical properties of the (S or R)-3-amino-3-(3-fluorophenyl)propionic acid were the same as those shown in Example 23.

Physical properties of the s-butyl (3R or 3S)-3-amino-3-(3-fluorophenyl)propionate were as follows. "Diastereomer mixture"
¹H-NMR (δ(ppm), CDCl₃): 0.83-0.89 (m, 3H), 1.16-1.65 (m, 3H), 1.46-1.65 (m,1H), 1.71 (s, 2H), 2.58-2.68 (m, 2H), 4.42 (dd, 1H, J=6.4,13.7Hz), 4.82-4.99 (m, 1H), 6.97-7.33 (m, 1H), 7.09-7.15 (m, 1H), 7.23-7.33 (m, 1H)
MS (EI) m/z: 239 (M⁺)
MS (CI, i-C₄H₁₀) m/z: 240 (MH⁺)

### Comparative example 18 (Syntheses of (S)-3-amino-3-phenylpropionic acid and (R)-n-propyl 3-amino-3-phenylpropionate)

To a mixture comprising 500 mg (2.41 mmol) of n-propyl 3-amino-3-phenylpropionate (racemic mixtures) synthesized in Reference example 2 and 1.25 mL of cyclohexane was added 1.25 mL of water a pH of which had been adjusted to 8.2 with 1 mol/L aqueous potassium hydroxide solution, and the mixture was maintained to 30°C. To the obtained mixture was added 25 mg of lipase (Amano Lipase PS (trade name); available from Aldrich Corporation) originated from *Burkholderia cepacia (Pseudomonas cepacia)* at the same temperature, and the mixture was reacted while stirring at 30°C. After 26 hours, a reaction conversion reached 42.6%.

The (R)-n-propyl 3-amino-3-phenylpropionate was led to (R)-n-propyl 3-(2-furoylamino)-3-phenylpropionate, and when an optical purity thereof was measured by using high performance liquid chromatography which uses an optically active column, it was 73.4%ee.

Incidentally, the E value in the present reaction was 441.
Analytical conditions of high performance liquid chromatography:
optically active 3-(2-furoylamino)-3-phenylpropionic acid n-propyl ester
Column: Chiralcel OJ-H (0.46cmΦx25cm, available from DAICEL CHEMICAL INDUSTRIES, LTD.)
Solvent: hexane/isopropyl alcohol (=1/9 (volume ratio)) Flow rate: 0.5 mL/min
Temperatrure: 30°C
Wavelength: 220 nm

Also, physical properties of the (S)-3-amino-3-phenylpropionic acid and (R)-n-propyl 3-amino-3-phenylpropionate were the same as those shown in Example 1.

### Utilizability in industry

According to the present invention, a novel n-alkyl 3-amino-3-arylpropionate can be provided with a high yield and a simple and easy process and a process for preparing the same can be provided.

Also, according to the present invention, an Industrially suitable process for preparing an optically active 3-amino-3-arylpropionic acid and an optically active n-alkyl 3-amino-3-arylpropionate can be provided by a simple and easy process which gives an optically active (S or R)-3-amino-3-arylpropionic acid and an optically active (R or S)-n-alkyl 3-amino-3-arylpropionate simultaneously from 3-amino-3-arylpropionic acid ester (racemic mixtures) with high E values.

## Claims

1. An n-alkyl 3-amino-3-arylpropionate represented by the formula (I): 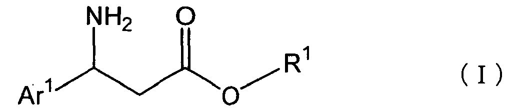
wherein Ar¹ represents an aryl group which may have a substituent(s), provided that a phenyl group and 4-methoxyphenyl group are excluded, R¹ represents an n-propyl group or an n-butyl group.

2. An (R or S)-n-alkyl 3-amino-3-arylpropionate represented by the formula (I-a): 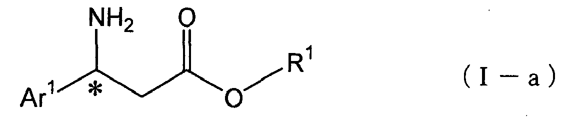
wherein Ar¹ and R¹ have the same meanings as defined in Claim 1, and * represents an asymmetric carbon.

3. A process for preparing an n-alkyl 3-amino-3-arylpropionate represented by the formula (IV): 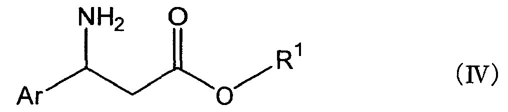
wherein R¹ represents an n-propyl group or an n-butyl group, and Ar represents an aryl group which may have a substituent(s), provided that a phenyl gruop is excluded,
which comprises
(A) a first step of reacting an arylaldehyde represented by the formula (II): 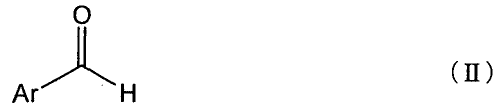
wherein Ar has the same meaning as defined above, and malonic acid and ammonium acetate in an organic solvent to make a 3-amino-3-arylpropionic acid represented by the formula (III): 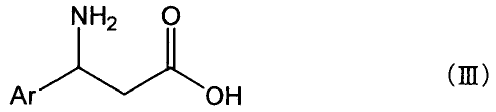
wherein Ar has the same meaning as defined above,
(B) then, a second step fo reacting the resulting compound with a 3-amino-3-arylpropionic acid and n-propyl alcohol or n-butyl alcohol in the presence of an acid catalyst.

4. The process for preparing an n-alkyl 3-amino-3-arylpropionate according to Claim 3, wherein the organic solvent is an alcohol solvent.

5. The process for preparing an n-alkyl 3-amino-3-arylpropionate according to Claim 4, wherein the alcohol solvent is ethanol or isopropyl alcohol.

6. A process for preparing an optically active (S or R)-3-amino-3-arylpropionic acid represented by the formula (III-a): 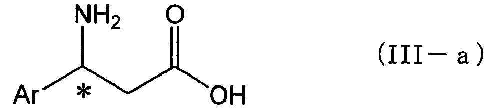
wherein Ar represents an aryl group which may have a substituent(s), and * represents an asymmetric carbon,
and an optically active (R or S)-n-alkyl 3-amino-3-arylpropionate represented by the formula (IV-a): 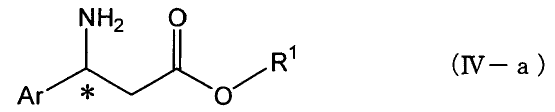
wherein Ar and * have the same meanings as defined above, and R¹ represents an n-propyl group or an n-butyl group, provided that it has a reverse absolute configuration to that of the compound of the formula (III-a),
which comprises subjecting either one of enantiomers of n-alkyl 3-amino-3-arylpropionate which is racemic mixtures and represented by the formula (IV): 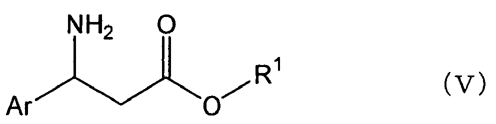
wherein Ar and R¹ have the same meanings as defined above,
to selectively hydrolysis reaction in the presence of a hydrolase in a mixed solvent of an organic solvent and a buffer.

7. The process according to Claim 6, wherein the hydrolase is protease, esterase or lipase.

8. The process according to Claim 6 or 7, wherein the hydrolase is lipase originated from *Burkholderia cepacia* (*Pseudomonas cepacia*).

9. The process according to Claim 6, wherein the organic solvent is an aliphatic hydrocarbon, an aromatic hydrocarbon or an ether, or a mixed solvent of the above.

10. The process according to Claim 6, wherein the organic solvent is cyclohexane, t-butyl methyl ether or toluene.

11. The process according to any one of Claims 6 to 10, wherein the buffer is at least one selected from the group consisting of an aqueous sodium phosphate solution, an aqueous potassium phosphate solution, an aqueous sodium acetate solution, an aqueous sodium citrate solution and an aqueous ammonium acetate solution.

12. The process according to any one of Claims 6 to 10, wherein the buffer is at least one selected from the group consisting of an aqueous sodium phosphate solution and an aqueous potassium phosphate solution.

13. The process according to Claim 11 or 12, wherein a concentration of the buffer is 0.05 to 0.5 mol/L.

14. The process according to any one of Claims 6 to 13, wherein the hydrolysis reaction is carried out at 10 to 50°C.

15. The process according to any one of Claims 6 to 13, wherein the hydrolysis reaction is carried out at 30 to 45°C.

16. The process according to Claim 6, wherein each of the optically active (S or R)-3-amino-3-arylpropionic acid represented by the formula (III-a): 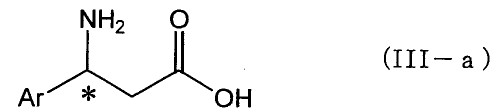
wherein Ar has the same meaning as defined above,
and * represents an asymmetric carbon,
and the optically active (R or S)-n-alkyl 3-amino-3-arylpropionate represented by the formula (IV-a): 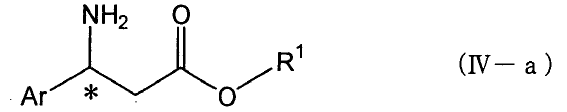
wherein Ar and R¹ have the same meanings as defined above, * represents an asymmetric carbon, provided that it has a reverse absolute configuration to the compound of the formula (III-a),
formed by the hydrolysis reaction is isolated from the mixture thereof.

17. The process according to Claim 6 or 16, wherein Ar is a phenyl group, 2-tolyl group, 3-tolyl group, 4-tolyl group, 2,3-xylyl group, 2-chlorophenyl group, 3-chlorophenyl group, 4-chlorophenyl group, 2,3-dichlorophenyl group, 2,4-dichlorophenyl group, 3,4-dichlorophenyl group, 3,5-dichlorophenyl group, 2-bromophenyl group, 3-bromophenyl group, 4-bromophenyl group, 2-fluorophenyl group, 3-fluorophenyl group, 4-fluorophenyl group, 3,4-difluorophenyl group, 2-iodophenyl group, 3-iodophenyl group, 4-iodophenyl group, 2-methoxyphenyl group, 3-methoxyphenyl group, 4-methoxyphenyl group, 2,3-dimethoxyphenyl group, 3,4-dimethoxyphenyl group, 3,5-dimethoxyphenyl group or 3,4-methylenedioxyphenyl group.
